# EUROPEAN PATENT APPLICATION

(11) **EP 1 782 859 A2**
(43) Date of publication of application: **09.05.2007**
(21) Application number: 07102700.7
(22) Date of filing: 06.04.2004
(51) Int. Cl.: A61P 3/04, A61K 31/519

(54) **Pharmaceutical use of substituted pyrazolo [1,5- a]pyrimidines**

(30) Priority: 11.04.2003 DK 200300569; 02.05.2003 US 467453 P
(62) Divisional of application: 04725889.2
(71) Applicant: NOVO NORDISK A/S, 2880 Bagsvaerd (DK)
(72) Inventor: Kampen, Gita, Camilla, Tejlgaard, 2850, Nærum (DK); Andersen, Henrik, Sune, 2800, Lyngby (DK); Christensen, Inge, Thøger, DK-2800, Kgs. Lyngby (DK); Mogensen, John, Patrick, DK-2730, Herlev (DK); Larsen, Annette, Rosendal, DK-2800, Lyngby (DK)
(74) Representative: Madsen, Inger Margrethe Schelde

(57) **Abstract**

The use of substituted pyrazolo[1,5-*a*]pyrimidines for modulating the activity of 11β-hydroxysteroid dehydrogenase type 1 (11βHSD1) and the use of these compounds as pharmaceutical compositions are described. Also a novel class of substituted pyrazolo[1,5-*a*]pyrimidines their use in therapy, pharmaceutical compositions comprising the compounds, as well as their use in the manufacture of medicaments are described The present compounds are modulators and more specifically inhibitors of the activity of 11βHSD1 and may be useful in the treatment, prevention and/or prophylaxis of a range of medical disorders where a decreased intracellular concentration of active glucocorticoid is desirable.

## Description

### FIELD OF THE INVENTION

The present invention relates to use of substituted pyrazolo[1,5-a]pyrimidines and pharmaceutical compositions comprising the compounds for treating disorders where it is desirable to modulate the activity of 11 β-hydroxysteroid dehydrogenase type 1 (11 βHSD1).

The present invention also relates to novel substituted pyrazolo[1,5-a]pyrimidines, to their use in therapy, to pharmaceutical compositions comprising the compounds, to the use of said compounds in the manufacture of medicaments, and to therapeutic methods comprising the administration of said compounds. The present compounds modulate the activity of 11 β-hydroxysteroid dehydrogenase type 1 (11βHSD1) and are accordingly useful in the treatment of diseases in which such a modulation is beneficial, such as the metabolic syndrome.

### BACKGROUND OF THE INVENTION

The metabolic syndrome is a major global health problem. In the US, the prevalence in the adult population is currently estimated to be approximately 25%, and it continues to increase both in the US and worldwide. The metabolic syndrome is characterised by a combination of insulin resistance, dyslipidemia, obesity and hypertension leading to increased morbidity and mortality of cardiovascular diseases. People with the metabolic syndrome are at increased risk of developing frank type 2 diabetes, the prevalence of which is equally escalating.

In type 2 diabetes, obesity and dyslipidemia are also highly prevalent and around 70% of people with type 2 diabetes additionally have hypertension once again leading to increased mortality of cardiovascular diseases.

In the clinical setting, it has long been known that glucocorticoids are able to induce all of the cardinal features of the metabolic syndrome and type 2 diabetes.

11 β-hydroxysteroid dehydrogenase type 1 (11βHSD1) catalyses the local generation of active glucocorticoid in several tissues and organs including predominantly the liver and adipose tissue, but also e.g. skeletal muscle, bone, pancreas, endothelium, ocular tissue and certain parts of the central nervous system. Thus, 11βHSD1 serves as a local regulator of glucocorticoid actions in the tissues and organs where it is expressed (Tannin et al., J. Biol. Chem., 266, 16653 (1991); Bujalska et al., Endocrinology, 140, 3188 (1999); Whorwood et al., J. Clin. Endocrinol. Metab., 86, 2296 (2001); Cooper et al., Bone, 27, 375 (2000); Davani et al., J. Biol. Chem., 275, 34841 (2000); Brem et al., Hypertension; 31, 459 (1998); Rauz et al., Invest. Ophthalmol. Vis. Sci., 42, 2037 (2001); Moisan et al., Endocrinology; 127, 1450 (1990)).

The role of 11βHSD1 in the metabolic syndrome and type 2 diabetes is supported by several lines of evidence. In humans, treatment with the non-specific 11βHSD1 inhibitor carbenoxolone improves insulin sensitivity in lean healthy volunteers and people with type 2 diabetes. Likewise, 11βHSD1 knock-out mice are resistant to insulin resistance induced by obesity and stress. Additionally, the knock-out mice present with an anti-atherogenic lipid profile of decreased VLDL triglycerides and increased HDL-cholesterol. Conversely, mice that overexpress 11βHSD1 in adipocytes develop insulin resistance, hyperlipidemia and visceral obesity, a phenotype that resembles the human metabolic syndrome (Andrews et al., J. Clin. Endocrinol. Metab., 88, 285 (2003); Walker et al., J. Clin. Endocrinol. Metab., 80, 3155 (1995); Morton et al., J. Biol. Chem. 276, 41293 (2001); Kotelevtsev et al., Proc. Natl. Acad. Sci. USA, 94, 14924 (1997); Masuzaki et al., Science, 294, 2166 (2001)).

The more mechanistic aspects of 11βHSD1 modulation and thereby modulation of intracellular levels of active glucocorticoid have been investigated in several rodent models and different cellular systems. 11βHSD1 promotes the features of the metabolic syndrome by increasing hepatic expression of the rate-limiting enzymes in gluconeogenesis, namely phosphoenolpyuvate carboxykinase and glucose-6-phosphatase, promoting the differentiation of preadipocytes into adipocytes thus facilitating obesity, directly and indirectly stimulating hepatic VLDL secretion, decreasing hepatic LDL uptake and increasing vessel contractility (Kotelevtsev et al., Proc. Natl. Acad. Sci. USA, 94, 14924 (1997); Morton et al., J. Biol. Chem. 276, 41293 (2001); Bujalska et al., Endocrinology, 140, 3188 (1999); Souness et al., Steroids, 67, 195 (2002); Brindley & Salter, Prog. Lipid Res., 30, 349 (1991)).

WO 01/90090, WO 01/90091, WO 01/90092, WO 01/90093 and WO 01/90094 discloses various thiazol-sulfonamides as inhibitors of the human 11 β-hydroxysteroid dehydrogenase type 1 enzyme, and further states that said compounds may be useful in treating diabetes, obesity, glaucoma, osteoporosis, cognitive disorders, immune disorders and depression.

We have now found substituted pyrazolo[1,5-a]pyrimidines that modulate the activity of 11βHSD1 leading to altered intracellular concentrations of active glucocorticoid. More specifically, the present compounds inhibit the activity of 11βHSD1 leading to decreased intracellular concentrations of active glucocorticoid. Thus, the present compounds can be used to treat disorders where a decreased level of active intracellular glucocorticoid is desirable, such as e.g. the metabolic syndrome, type 2 diabetes, impaired glucose tolerance (IGT), impaired fasting glucose (IFG), dyslipidemia, obesity, hypertension, diabetic late complications, cardiovascular diseases, arteriosclerosis, atherosclerosis, myopathy, muscle wasting, osteoporosis, neurodegenerative and psychiatric disorders, and adverse effects of treatment or therapy with glucocorticoid receptor agonists.

One object of the present invention is to provide compounds, pharmaceutical compositions and use of compounds that modulate the activity of 11 βHSD1.

### DEFINITIONS

In the following structural formulas and throughout the present specification, the following terms have the indicated meaning:

The term "halo" includes fluorine, chlorine, bromine, and iodine.

The term "trihalomethyl" includes trifluoromethyl, trichloromethyl, tribromomethyl, and triiodomethyl.

The term "trihalomethoxy" includes trifluorometoxy, trichlorometoxy, tribromometoxy, and triiodometoxy.

The term "alkyl" includes C₁-C₆ straight chain saturated and methylene aliphatic hydrocarbon groups, C₃-C₆ branched saturated hydrocarbon groups having the specified number of carbon atoms. For example, this definition shall include but is not limited to methyl (Me), ethyl (Et), propyl (Pr), butyl (Bu), pentyl, hexyl, isopropyl (i-Pr), isobutyl (i-Bu), *tert*-butyl (*t*-Bu), sec-butyl (s-Bu), isopentyl, neopentyl, and the like.

The term "alkenyl" includes C₂-C₆ straight chain unsaturated aliphatic hydrocarbon groups and branched C₃-C₆ unsaturated aliphatic hydrocarbon groups having the specified number of carbon atoms. For example, this definition shall include but is not limited to ethenyl, propenyl, butenyl, pentenyl, hexenyl, methylpropenyl, methylbutenyl and the like.

The term "alkynyl" includes C₂-C₆ straight chain unsaturated aliphatic hydrocarbon groups and C₄-C₆ branched unsaturated aliphatic hydrocarbon groups having the specified number of carbon atoms. For example, this definition shall include but is not limited to ethynyl, propynyl, butynyl, pentynyl, hexynyl, methylbutynyl, and the like.

The term "saturated or partially saturated cyclic, bicyclic or tricyclic ring system" represents but are not limit to aziridinyl, pyrrolinyl, pyrrolidinyl, 2-imidazolinyl, imidazolidinyl, 2-pyrazolinyl, morpholinyl, piperidinyl, piperazinyl, phthalimide, 1,2,3,4-tetrahydro-quinolinyl, 1,2,3,4-tetrahydro-isoquinolinyl, 1,2,3,4-tetrahydro-quinoxalinyl, 6-aza-bicyclo[3.2.1]octane, and indolinyl.

The term "cycloalkyl" (e.g. cyclopropyl, cyclobutyl, cyclopentyl, cyclohexyl, cycloheptyl, cyclooctyl, cyclononyl, cyclodecyl, bicyclo[3.2.1]octyl, spiro[4.5]decyl, norpinyl, norbonyl, norcaryl, adamantyl and the like) represents a saturated, mono-, bi-, tri- or spirocarbocyclic group having the specified number of carbon atoms.

The term "cycloalkylalkyl" (e.g. cyclopropylmethyl, cyclobutylethyl, adamantylmethyl and the like) represents a cycloalkyl group as defined above attached through an alkyl group having the indicated number of carbon atoms or substituted alkyl group as defined above.

The term "cycloalkenyl" (e.g. cyclobutenyl, cyclopentenyl, cyclohexenyl, cycloheptenyl, cyclooctenyl, cyclononenyl, cyclodecenyl and the like) represents a partially saturated, mono-, bi-, tri- or spirocarbocyclic group having the specified number of carbon atoms.

The term "hetcycloalkyl" (tetrahydrofuranyl, tetrahydropyranyl, tertahydrothiopyranyl, and the like) represents a saturated mono-, bi-, tri- or spirocarbocyclic group having the specified number of carbon atoms and one or two additional heteroatoms or groups selected from nitrogen, oxygen, sulphur, SO or SO₂.

The term "alkyloxy" (e.g. methoxy, ethoxy, propyloxy, allyloxy, cyclohexyloxy) represents an alkyl group as defined above having the indicated number of carbon atoms attached through an oxygen bridge.

The term "alkyloxyalkyl" (e.g. methyloxymethyl and the like) represents an alkyloxy group as defined above attached through an "alkyl" group.

The term "aryloxy" (e.g. phenoxy, naphthyloxy and the like) represents an aryl group as defined below attached through an oxygen bridge.

The term "hetaryloxy" (e.g. 2-pyridyloxy and the like) represents a hetaryl group as defined below attached through an oxygen bridge.

The term "arylalkyloxy" (e.g. phenethyloxy, naphthylmethyloxy and the like) represents an arylalkyl group as defined below attached through an oxygen bridge.

The term "hetarylalkyloxy" (e.g. 2-pyridylmethyloxy and the like) represents a hetarylalkyl group as defined below attached through an oxygen bridge.

The term "alkylthio" (e.g. methylthio, ethylthio and the like) represents an alkyl group as defined above attached through a sulphur bridge.

The term "arylthio" (e.g. benzenthiol, naphthylthiol and the like) represents an aryl group as defined below attached through a sulphur bridge.

The term "arylthioalkyl" (e.g. methylsulfanyl benzene, ethylsulfanyl naphthalene and the like) represents an arylthio group as defined below attached through an alkyl group having the indicated number of carbon atoms.

The term "hetarylthioalkyl" (e.g. 2-methylsulfanyl-pyridine, 1-ethylsulfanylisoquinoline and the like) represents a hetarylthio group as defined below attached through an alkyl group having the indicated number of carbon atoms.

The term "aryloxyalkyl" (e.g. phenoxymethyl, naphthyloxyethyl and the like) represents an aryloxy group as defined above attached through an "alkyl" group having the indicated number of carbon atoms.

The term "aryloxyaryl" (e.g. 1-phenoxy-naphthalene, phenyloxyphenyl and the like) represents an aryloxy group as defined above attached through an "aryl" group as defined below.

The term "arylalkyloxyalkyl" (e.g. ethoxymethyl-benzene, 2-methoxymethylnaphthalene and the like) represents an arylalkyloxy group as defined above attached through an "alkyl" group having the indicated number of carbon atoms.

The term "hetaryloxyalkyl" (e.g. 2-pyridyloxymethyl, 2-quinolyloxyethyl and the like) represents a hetaryloxy group as defined above attached through an "alkyl" group having the indicated number of carbon atoms.

The term "hetarylalkyloxyalkyl" (e.g. 4-methoxymethyl-pyrimidine, 2-methoxymethylquinoline and the like) represents a hetarylalkyloxy group as defined above attached through an "alkyl" group having the indicated number of carbon atoms.

The term "arylalkyl" (e.g. benzyl, phenylethyl, 3-phenylpropyl, 1-naphtylmethyl, 2-(1-naphtyl)ethyl and the like) represents an aryl group as defined below attached through an alkyl having the indicated number of carbon atoms or substituted alkyl group as defined above.

The term "hetarylalkyl" and "hetaralkyl" (e.g. (2-furyl)methyl, (3-furyl)methyl, (2-thienyl)methyl, (3-thienyl)methyl, (2-pyridyl)methyl, 1-methyl-1-(2-pyrimidyl)ethyl and the like) represents a hetaryl group as defined below attached through an alkyl having the indicated number of carbon atoms or substituted alkyl group as defined above.

The term "alkylcarbonyl" (e.g. octylcarbonyl, pentylcarbonyl, 3-hexenylcarbonyl) represents an alkyl group as defined above having the indicated number of carbon atoms attached through a carbonyl group.

The term "arylcarbonyl" (e.g. benzoyl) represents an aryl group as defined below attached through a carbonyl group.

The term "hetarylcarbonyl" (e.g. 2-thiophenylcarbonyl, 3-methoxy-anthrylcarbonyl, oxazolylcarbonyl and the like) represents a hetaryl group as defined below attached through a carbonyl group.

The term "carbonylalkyl" (e.g. acetyl and the like) represents a carbonyl group attached through alkyl group as defined above having the indicated number of carbon atoms.

The term "alkylcarbonylalkyl" (e.g. propan-2-one, 4,4-dimethyl-pentan-2-one and the like) represents an alkylcarbonyl group as defined above attached through an alkyl group as defined above having the indicated number of carbon atoms.

The term "arylcarbonylalkyl" (e.g. 1-phenyl-propan-1-one, 1-(3-chloro-phenyl)-2-methyl-butan-1-one and the like) represents a arylcarbonyl group as defined above attached through an alkyl group as defined above having the indicated number of carbon atoms.

The term "hetarylcarbonylalkyl" (e.g. 1-pyridin-2-yl-propan-1-one,1-(1-*H*-imidazol-2-yl)-propan-1-one and the like) represents a hetarylcarbonyl group as defined above attached through an alkyl group as defined above having the indicated number of carbon atoms.

The term "arylalkylcarbonyl" (e.g. phenylpropylcarbonyl, phenylethylcarbonyl and the like) represents an arylalkyl group as defined above having the indicated number of carbon atoms attached through a carbonyl group.

The term "hetarylalkylcarbonyl" (e.g. imidazolylpentylcarbonyl and the like) represents an hetarylalkyl group as defined above wherein the alkyl group is in turn attached through a carbonyl.

The term "alkylcarboxy" (e.g. heptylcarboxy, cyclopropylcarboxy, 3-pentenylcarboxy) represents an alkylcarbonyl group as defined above wherein the carbonyl is in turn attached through an oxygen bridge.

The term "arylcarboxy" (e.g. benzoic acid and the like) represents an arylcarbonyl group as defined above wherein the carbonyl is in turn attached through an oxygen bridge.

The term "arylalkylcarboxy" (e.g. benzylcarboxy, phenylpropylcarboxy and the like) represents an arylalkylcarbonyl group as defined above wherein the carbonyl is in turn attached through an oxygen bridge.

The term "arylalkylcarboxyalkyl" (e.g. benzylcarboxymethyl, phenylpropylcarboxypropyl and the like) represents an arylalkylcarboxy group as defined above wherein the carboxy group is in turn attached through an alkyl group as defined above having the indicated number of carbon atoms.

The term "hetarylcarboxy" (e.g. pyridine-2-carboxylic acid and the like) represents a hetarylcarbonyl group as defined above wherein the carbonyl is in turn attached through an oxygen bridge.

The term "hetarylalkylcarboxy" (e.g. (1-*H*-imidazol-2-yl)-acetic acid, 3-pyrimidin-2-yl-propionic acid and the like) represents a hetarylalkylcarbonyl group as defined above wherein the carbonyl is in turn attached through an oxygen bridge.

The term "alkylcarbonylamino" (e.g. methylcarbonylamino, cyclopentylcarbonylaminomethyl, methylcarbonylaminophenyl) represents an "alkylcarbonyl" group as defined above wherein the carbonyl is in turn attached through the nitrogen atom of an amino group. The nitrogen atom may itself be substituted with an alkyl or aryl group.

The term "alkylcarbonylaminoalkyl" (e.g.N-propyl-acetamide, N-butyl-propionamide and the like) represents an "alkylcarbonylamino" group attached through an alkyl group as defined above having the indicated number of carbon atoms.

The term "arylalkylcarbonylamino" (e.g. phenylacetamide, 3phenyl-propionamide and the like) represents an "arylalkylcarbonyl" group as defined above attached through an amino group.

The term "arylalkylcarbonylaminoalkyl" (e.g. N-ethyl-phenylacetamide, N-butyl-3-phenyl-propionamide and the like) represents an "arylalkylcarbonylamino" group attached through an alkyl group as defined above having the indicated number of carbon atoms.

The term "arylcarbonylamino" (e.g. benzamide, naphthalene-1-carboxylic acid amide and the like) represents an "arylcarbonyl" group as defined above attached through an amino group.

The term "arylcarbonylaminoalkyl" (e.g. N-propyl-benzamide, N-Butyl-naphthalene-1-carboxylic acid amide and the like) represents an "arylcarbonylamino" group attached through an alkyl group as defined above having the indicated number of carbon atoms.

The term "aryl" includes but is not limited to a carbocyclic aromatic ring system being either monocyclic, bicyclic, or polycyclic, such as phenyl, biphenyl, naphthyl, anthracenyl, phenanthrenyl, fluorenyl, indenyl, pentalenyl, azulenyl, biphenylenyl and the like. Aryl is also intended to include the partially hydrogenated derivatives of the carbocyclic aromatic systems enumerated above. Non-limiting examples of such partially hydrogenated derivatives are 1,2,3,4-tetrahydronaphthyl, 1,4-dihydronaphthyl and the like.

The term "aryl1" includes phenyl, biphenyl, naphthyl, anthracenyl, phenanthrenyl, and fluorenyl.

The term "aryl2" includes phenyl, biphenyl, naphthyl, and anthracenyl.

The term "hetaryl" includes but is not limited to pyrrolyl (2-pyrrolyl), pyrazolyl (3-pyrazolyl), imidazolyl (1-imidazolyl, 2-imidazolyl, 4-imidazolyl, 5-imidazolyl), triazolyl (1,2,3-triazol-1-yl, 1,2,3-triazol-2-yl, 1,2,3-triazol-4-yl, 1,2,4-triazol-3-yl), oxazolyl (2-oxazolyl, 4-oxazolyl, 5-oxazolyl), isoxazolyl (3-isoxazolyl, 4-isoxazolyl, 5-isoxazolyl), thiazolyl (2-thiazolyl, 4-thiazolyl, 5-thiazolyl), thiophenyl (2-thiophenyl, 3-thiophenyl, 4-thiophenyl, 5-thiophenyl), furanyl (2-furanyl, 3-furanyl, 4-furanyl, 5-furanyl), pyridyl (2-pyridyl, 3-pyridyl, 4-pyridyl, 5-pyridyl), 5-tetrazolyl, pyrimidinyl (2-pyrimidinyl, 4-pyrimidinyl, 5-pyrimidinyl, 6-pyrimidinyl), pyrazinyl, pyridazinyl (3-pyridazinyl, 4-pyridazinyl, 5-pyridazinyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4-isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-dihydro-benzo[b]furanyl), 3-(2,3-dihydro-benzo[b]furanyl), 4-(2,3-dihydro-benzo[b]furanyl), 5-(2,3-dihydrobenzo-[b]furanyl), 6-(2,3-dihydro-benzo[b]furanyl), 7-(2,3-dihydro-benzo[b]furanyl)), benzo[b]thiophenyl (2-benzo[b]thiophenyl, 3-benzo[b]thiophenyl, 4-benzo[b]thiophenyl, 5-benzo[b]thiophenyl, 6-benzo[b]thiophenyl, 7-benzo[b]thiophenyl), 2,3-dihydro-benzo[b]thiophenyl (2-(2,3-dihydro-benzo[b]thiophenyl), 3-(2,3-dihydro-benzo[b]thiophenyl), 4-(2,3-dihydro-benzo[b]thiophenyl), 5-(2,3-dihydro-benzo[b]thiophenyl), 6-(2,3-dihydro-benzo[b]thiophenyl), 7-(2,3-dihydro-benzo[b]thiophenyl)), 4,5,6,7-tetrahydro-benzo[b]thiophenyl (2-(4,5,6,7-tetrahydro-benzo[b]thiophenyl), 3-(4,5,6,7-tetrahydro-benzo[b]thiophenyl), 4-(4,5,6,7-tetrahydro-benzo[b]thiophenyl), 5-(4,5,6,7-tetrahydro-benzo[b]thiophenyl), 6-(4,5,6,7-tetrahydro-benzo[b]thiophenyl), 7-(4,5,6,7-tetrahydro-benzo[b]thiophenyl)), thieno[2,3-b]thiophenyl, 4,5,6,7-tetrahydro-thieno[2,3-c]pyridyl (4-(4,5,6,7-tetrahydro-thieno[2,3-c]pyridyl), 5-4,5,6,7-tetrahydro-thieno[2,3-c]pyridyl), 6-(4,5,6,7-tetrahydro-thieno[2,3-c]pyridyl), 7-(4,5,6,7-tetrahydro-thieno[2,3-c]pyridyl)), indolyl (1-indolyl, 2-indolyl, 3-indolyl, 4-indolyl, 5-indolyl, 6-indolyl, 7-indolyl), isoindolyl (1-isoindolyl, 2-isoindolyl, 3-isoindolyl, 4-isoindolyl, 5-isoindolyl, 6-isoindolyl, 7-isoindolyl), 1,3-dihydro-isoindolyl (1-(1,3-dihydro-isoindolyl), 2-(1,3-dihydro-isoindolyl), 3-(1,3-dihydro-isoindolyl), 4-(1,3-dihydro-isoindolyl), 5-(1,3-dihydro-isoindolyl), 6-(1,3-dihydro-isoindolyl), 7-(1,3-dihydro-isoindolyl)), indazole (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benz-oxazolyl, 2-benzoxazolyl), benzothiazolyl (1-benzothiazolyl, 2-benzothiazolyl, 4-benzothiazolyl, 5-benzothiazolyl, 6-benzothiazolyl, 7-benzothiazolyl), benzo-[1,2,5]oxadiazolyl, (4-benzo[1,2,5]oxadiazole, 5-benzo[1,2,5]oxadiazole), carbazolyl (1-carbazolyl, 2-carbazolyl, 3-carbazolyl, 4-carbazolyl), piperidinyl (2-piperidinyl, 3-piperidinyl, 4-piperidinyl), pyrrolidinyl (1-pyrrolidinyl, 2-pyrrolidinyl, 3-pyrrolidinyl).

Certain of the above defined terms may occur more than once in the structural formulae, and upon such occurrence each term shall be defined independently of the other.

The term "optionally substituted" as used herein means that the groups in question are either unsubstituted or substituted with one or more of the substituents specified. When the groups in question are substituted with more than one substituent the substituents may be the same or different.

The term "treatment" is defined as the management and care of a patient for the purpose of combating or alleviating the disease, condition or disorder, and the term includes the administration of the active compound to prevent the onset of the symptoms or complications, or alleviating the symptoms or complications, or eliminating the disease, condition, or disorder.

The term "pharmaceutically acceptable" is defined as being suitable for administration to humans without adverse events.

The term "prodrug" is defined as a chemically modified form of the active drug, said prodrug being administered to the patient and subsequently being converted to the active drug. Techniques for development of prodrugs are well known in the art.

### DETAILED DESCRIPTION OF THE INVENTION

In one aspect, the present invention provides the use of a substituted pyrazolo[1,5-a]pyrimidine, a pro-drug thereof, or a salt thereof with a pharmaceutically acceptable acid or base, or any optical isomer or mixture of optical isomers, including a racemic mixture, or any tautomeric forms for
a) modulation of the activity of 11βHSD1;or
b) inhibition of 11βHSD1,
in a patient in need thereof.

In another aspect, the present invention provides the use of a substituted pyrazolo[1,5-a]pyrimidine, a prodrug thereof, or a salt thereof with a pharmaceutically acceptable acid or base, or any optical isomer or mixture of optical isomers, including a racemic mixture, or any tautomeric forms for the preparation of a pharmaceutical composition for the treatment, prevention and/or prophylaxis of any disorder and disease where it is desirable to
a) modulate the activity of 11 βHSD1; or
b) inhibit 11βHSD1,
   in a patient in need thereof.

In another embodiment, the invention provides the present use of a substituted pyrazolo[1,5-a]pyrimidine or a prodrug thereof is of the general formula (I) 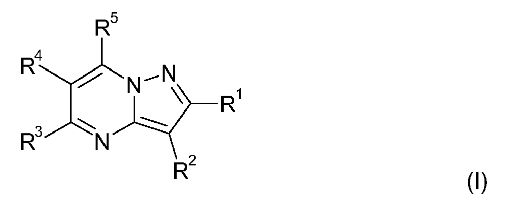 wherein
R¹ and R² independently are hydrogen, halo, C(=O)NR⁶R⁷, CO₂R¹⁵, NR⁶C(=O)R¹¹, OR¹² or SR¹²;
R³ and R⁵ independently are hydrogen, NR¹³R¹⁴, trihalomethyl, trihalomethoxy, C₁-C₆alkyl, C₂-C₆alkenyl, C₂-C₆alkynyl, C₁-C₆alkyloxy, C₁-C₆alkylthio, aryl, arylC₁-C₆alkyl, hetaryl or hetarylC₁-C₆alkyl, wherein alkyl, alkynyl, alkenyl, aryl, hetaryl, arylalkyl or hetarylalkyl groups independently are optionally substituted with one or more of R⁸;
R⁴ is hydrogen, cyano, halo, CO₂R¹⁵, C(=O)NR⁶R⁷, C₁-C₆alkyl, C₂-C₆alkenyl, C₂-C₆alkynyl, aryl, hetaryl, arylC₁-C₆alkyl, hetarylC₁-C₆alkyl, hydroxy, C₁-C₆alkyloxy, arylC₁-C₆alkyloxy, hetarylC₁-C₆alkyloxy, or C₁-C₆alkyloxyC₁-C₆alkyl, wherein the alkyl, alkynyl, alkenyl, cycloalkyl, aryl, hetaryl, arylalkyl or hetarylalkyl groups independently are optionally substituted with one or more of R⁹;
R⁶ and R⁷ independently are C₁-C₆alkyl, C₃-C₁₀cycloalkyl, hetC₃-C₁₀cycloalkyl, arylC₁-C₆alkyl or hetarylC₁-C₆alkyl, wherein the alkyl, cycloalkyl, hetcycloalkyl, arylalkyl, and hetarylalkyl groups independently are optionally substituted with one or more of R¹⁰; or
R⁶ and R⁷ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R⁸ and R⁹ independently are hydrogen, halo, hydroxy, oxo, cyano, nitro, C₃-C₁₀cycloalkyl, C₃-C₁₀hetocycloalkyl, C₁-C₆alkyl, C₁-C₆alkyloxy, trihalomethyl, trihalomethoxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆-alkylcarboxy, hetarylC₁-C₆alkylcarboxy, C₁-C₆alkylcarbonylamino or arylC₁-C₆alkylcarbonylamino;
R¹⁰ is hydrogen, halo, cyano, nitro, hydroxy, oxo, C₃-C₁₀cycloalkyl, C₃-C₁₀hetcycloalkyl, C₁-C₆alkyl, C₁-C₆alkyloxy, trihalomethyl, trihalometh dialkylamino oxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;
R¹¹ is C₁-C₆alkyl, C₂-C₆alkenyl, C₂-C₆alkynyl, arylC₁-C₆alkyl, hetarylC₁-C₆alkyl, C₁-C₆alkyl-carbonylC₁-C₆alkyl, C₁-C₆alkyloxy, aryloxy, C₁-C₆alkyloxy, arylcarbonyl, arylC₁-C₆alkylcarbonyl, hetarylcarbonylC₁-C₆alkyl, wherein the alkyl, alkenyl, alkynyl, aryl and hetaryl groups independently are optionally substituted with one or more of R⁹;
R¹² is C₁-C₆alkylcarbonylaminoC₁-C₆alkyl, arylcarbonylaminoC₁-C₆alkyl or arylC₁-C₆alkyl-carbonylaminoC₁-C₆alkyl;
R¹³and R¹⁴ independently are hydrogen, oxo, C₃-C₁₀cycloalkyl, C₁-C₆alkyl, C₁-C₆alkylcarbonyl, arylcarbonyl, hetarylcarbonyl, arylC₁-C₆alkylcarbonyl, hetarylC₁-C₆alkylcarbonyl;
R¹⁵ is hydrogen, C₃-C₁₀cycloalkyl, C₁-C₆alkyl, aryl, hetaryl, arylC₁-C₆alkyl, hetarylC₁-C₆alkyl, C₁-C₆alkyloxyalkyl or arylC₁-C₆alkyloxyalkyl; or
a salt thereof with a pharmaceutically acceptable acid or base, or any optical isomer or mixture of optical isomers, including a racemic mixture, or any tautomeric forms.

In another embodiment, the invention provides the present use of a substituted pyrazolo[1,5-a]pyrimidines, or a prodrug thereof of the general formula (I) 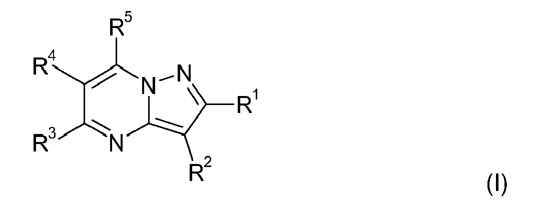 wherein
R¹ and R² independently are hydrogen, halo, C(=O)NR⁶R⁷, NC(=O)R¹¹, OR¹² or SR¹²;
R³ and R⁵ independently are hydrogen, NR¹³R¹⁴, trihalomethyl, trihalomethoxy, C₁-C₆alkyl, C₂-C₆alkenyl, C₂-C₆alkynyl, C₁-C₆alkyloxy, C₁-C₆alkylthio, aryl, arylC₁-C₆alkyl, hetaryl or hetaryl-C₁-C₆alkyl, wherein alkyl, alkynyl, alkenyl, aryl, hetaryl, arylalkyl or hetarylalkyl groups independently are optionally substituted with one or more of R⁸;
R⁴ is hydrogen, cyano, halo, CO₂R¹⁵, C(=O)NR⁶R⁷, C₁-C₆alkyl, C₂-C₆alkenyl, C₂-C₆alkynyl, aryl, hetaryl, arylC₁-C₆alkyl, hetarylC₁-C₆alkyl, hydroxy, C₁-C₆alkyloxy, arylC₁-C₆alkyloxy, hetarylC₁-C₆alkyloxy, or C₁-C₆alkyloxyC₁-C₆alkyl, wherein the alkyl, alkynyl, alkenyl, cycloalkyl, aryl, hetaryl, arylalkyl or hetarylalkyl groups independently are optionally substituted with one or more of R⁹;
R⁶ and R⁷ independently are C₁-C₆alkyl, C₃-C₁₀cycloalkyl, hetC₃-C₁₀cycloalkyl, arylC₁-C₆alkyl or hetarylC₁-C₆alkyl, wherein the alkyl, cycloalkyl, hetcycloalkyl, arylalkyl, and hetarylalkyl groups independently are optionally substituted with one or more of R¹⁰; or
R⁶ and R⁷ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆-alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R⁸ and R⁹ independently are hydrogen, halo, hydroxy, oxo, cyano, nitro, C₃-C₁₀cycloalkyl, C₃-C₁₀hetocycloalkyl, C₁-C₆alkyl, C₁-C₆alkyloxy, trihalomethyl, trihalomethoxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy, hetarylC₁-C₆alkylcarboxy, C₁-C₆alkylcarbonylamino or arylC₁-C₆alkylcarbonylamino;
R¹⁰ is hydrogen, halo, cyano, nitro, hydroxy, oxo, C₃-C₁₀cycloalkyl, C₃-C₁₀hetcycloalkyl, C₁-C₆alkyl, C₁-C₆alkyloxy, trihalomethyl, trihalometh dialkylamino oxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;
R¹¹ is C₁-C₆alkyl, C₂-C₆alkenyl, C₂-C₆alkynyl, arylC₁-C₆alkyl, hetarylC₁-C₆alkyl, C₁-C₆alkylcarbonylC₁-C₆alkyl, C₁-C₆alkyl, arylcarbonyl, arylC₁-C₆alkylcarbonyl, hetarylcarbonylC₁-C₆-alkyl, wherein the alkyl, alkenyl, alkynyl, aryl and hetaryl groups independently are optionally substituted with one or more of R⁹;
R¹² is C₁-C₆alkylcarbonylaminoC₁-C₆alkyl, arylcarbonylaminoC₁-C₆alkyl or arylC₁-C₆alkylcarbonylaminoC₁-C₆alkyl;
R¹³ and R¹⁴ independently are hydrogen, oxo, C₃-C₁₀cycloalkyl, C₁-C₆alkyl, C₁-C₆alkyloxy, trihalomethyl, trihalomethoxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy;
R¹⁵ is hydrogen, C₃-C₁₀cycloalkyl, C₁-C₆alkyl, aryl, hetaryl, arylC₁-C₆alkyl, hetarylC₁-C₆alkyl, C₁-C₆alkyloxyalkyl or arylC₁-C₆alkyloxyalkyl; or
a salt thereof with a pharmaceutically acceptable acid or base, or any optical isomer or mixture of optical isomers, including a racemic mixture, or any tautomeric forms.

In another embodiment, the invention provides the present use of a substituted pyrazolo[1,5-a]pyrimidine, or a prodrug thereof of the general formula (I) wherein R¹ and R² independently are hydrogen and/or C(=O)NR⁶R⁷.

In another embodiment, the invention provides the present use of a substituted pyrazolo[1,5-a]pyrimidine, or a prodrug thereof of the general formula (I) wherein R³ and R⁵ independently are hydrogen, trihalomethyl, C₁-C₆alkyl, C₁-C₆alkyloxy, aryl, arylC₁-C₆alkyl, hetaryl or hetarylC₁-C₆alkyl, wherein alkyl, aryl, hetaryl, arylalkyl or hetarylalkyl groups independently are optionally substituted with one or more of R⁸.

In another embodiment, the invention provides the present use of a substituted pyrazolo[1,5-*a*]pyrimidine, or a prodrug thereof of the general formula (I) wherein R⁴ is hydrogen, cyano, halo, C(=O)NR⁶R⁷, C₁-C₆alkyl, aryl, hetaryl, arylC₁-C₆alkyl, hetarylC₁-C₆alkyl, hydroxy, C₁-C₆alkyloxy, arylC₁-C₆alkyloxy, hetarylC₁-C₆alkyloxy, wherein the alkyl, aryl, hetaryl, arylalkyl or hetarylalkyl groups independently are optionally substituted with one or more of R⁹.

In another embodiment, the invention provides the present use of a substituted pyrazolo[1,5-a]pyrimidine, or a prodrug thereof of the general formula (I) wherein R⁶ and R⁷ independently are C₁C₆alkyl, C₃-C₁₀cycloalkyl, hetC₃-C₁₀cycloalkyl, arylC₁-C₆alkyl or hetarylC₁-C₆alkyl, wherein the alkyl, cycloalkyl, hetcycloalkyl, arylalkyl, and hetarylalkyl groups independently are optionally substituted with one or more of R¹⁰.

In another embodiment, the invention provides the present use of a substituted pyrazolo[1,5-a]pyrimidine, or a prodrug thereof of the general formula (I) R⁶ and R⁷ together with the nitrogen to which they are attached, are forming a saturated or partially saturated cyclic, bicyclic or tricyclic ring system containing from 4 to 10 carbon atoms and from 0 to 2 additional heteroatoms selected from nitrogen or oxygen, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy.

In another embodiment, the invention provides the present use of a substituted pyrazolo[1,5-a]pyrimidine, or a prodrug thereof of the general formula (I) wherein R⁶ and R⁷ together with the nitrogen to which they are attached, are forming a saturated or partially saturated cyclic, bicyclic or tricyclic ring system containing from 4 to 10 carbon atoms and from 0 to 2 additional nitrogen atoms, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆alkyloxyC₁-C₆alkyl.

In another embodiment, the invention provides the present use of a substituted pyrazolo[1,5-a]pyrimidine, or a prodrug thereof of the general formula (I) wherein R⁸ and R⁹ independently are hydrogen, halo, hydroxy, oxo, cyanoC₃-C₁₀cycloalkyl, C₃-C₁₀hetocycloalkyl, C₁-C₆alkyl, C₁-C₆alkyloxy, trihalomethyl, arylC₁-C₆alkyloxy, hetarylC₁-C₆alkyloxy.

In another embodiment, the invention provides the present use of a substituted pyrazolo[1,5-a]pyrimidine, or a prodrug thereof of the general formula (I) wherein R¹⁰ is hydrogen, halo, cyano, nitro, hydroxy, oxo, C₃-C₁₀cycloalkyl, C₃-C₁₀hetcycloalkyl, C₁-C₆alkyl, C₁-C₆alkyloxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy.

In another embodiment, the invention provides the present use of a substituted pyrazolo[1,5-a]pyrimidine, or a prodrug thereof of the general formula (I) wherein R¹¹ is C₁-C₆-alkyl, arylC₁-C₆alkyl, hetarylC₁-C₆alkyl, C₁-C₆alkyloxy, aryloxy, C₁-C₆alkyloxy, wherein the alkyl, aryl and hetaryl groups independently are optionally substituted with one or more of R⁹.

In another embodiment, the invention provides the present use of a substituted pyrazolo[1,5-a]pyrimidine, or a prodrug thereof of the general formula (I) wherein R¹² is C₁-C₆-alkylcarbonylaminoC₁-C₆alkyl, arylcarbonylaminoC₁-C₆alkyl or arylC₁-C₆alkylcarbonylaminoC₁-C₆alkyl.

In another embodiment, the invention provides the present use of a substituted pyrazolo[1,5-a]pyrimidine, or a prodrug thereof of the general formula (I) wherein R¹³and R¹⁴ independently are hydrogen, C₃-C₁₀cycloalkyl, C₁-C₆alkyl, C₁-C₆alkylcarbonyl, arylcarbonyl, hetarylcarbonyl, arylC₁-C₆alkylcarbonyl.

In another embodiment, the invention provides the present use of a substituted pyrazolo[1,5-a]pyrimidine, or a prodrug thereof of the general formula (I) wherein R¹⁵ is hydrogen, C₃-C₁₀cycloalkyl, C₁-C₆alkyl, aryl, hetaryl, arylC₁-C₆alkyl, hetarylC₁-C₆alkyl, C₁-C₆alkyloxyalkyl or arylC₁-C₆alkyloxyalkyl.

In another embodiment, the invention provide the present use of substituted pyrazolo[1,5-a]pyrimidine, or a prodrug thereof of general formula (I) selected from the group consisting of:
(3-Bromo-5-thiophen-2-yl-7-trifluoromethyl-pyrazolo[1,5-*a*]pyrimidin-2-yl)-(2,6-dimethylpiperidin-1-yl-)methanone;
(3-Bromo-5-thiophen-2-yl-7-trifluoromethyl-pyrazolo[1,5-*a*]pyrimidin-2-yl)-(2-ethyl-piperidin-1-yl-)methanone;
(5-Thiophen-2-yl-7-trifluoromethyl-pyrazolo[1,5-*a*]pyrimidin-2-yl)-(1,3,3-trimethyl-6-azabicyclo[3.2.1]oct-6-yl)methanone;
(3-Chloro-5-thiophen-2-yl-7-trifluoromethyl-pyrazolo[1,5-*a*]pyrimidin-2-yl)-(2-methyl-piperidin-1-yl-)methanone;
5-Methyl-7-phenyl-pyrazolo[1,5-a]pyrimidine-2-carboxylic acid cyclohexylamide;
Azepan-1-yl-(3-chloro-5-thiophen-2-yl-7-trifluoromethyl-pyrazolo[1,5-a]pyrimidin-2-yl)-methanone;
Azepan-1-yl-(3-chloro-5-thiophen-2-yl-7-trifluoromethyl-pyrazolo[1,5-a]pyrimidin-3-yl)-methanone;
(2,6-Dimethyl-piperidin-1-yl-)-(3-chloro-5-thiophen-2-yl-7-trifluoromethyl-pyrazolo[1,5-a]pyrimidin-3-yl)methanone;
3-Bromo-5-furan-2-yl-7-trifluoromethyl-pyrazolo[1,5-*a*]pyrimidin-2-carboxylic acid cyclohexylamide;
(7-Methyl-5-phenyl-pyrazolo[1,5-*a*]pyrimidin-3-yl)-(4-methyl-piperidin-1-yl)methanone;
5-(-4-Methoxy-phenyl-7-trifluoromethyl-pyrazolo[1,5-a]pyrimidine-3-carboxylic acid benzyl-methyl amide;
[5-(-4-Ethoxy-phenyl-7-methyl-pyrazolo[1,5-*a*]pyrimidine-2-yl])-piperidin-1-yl-methanone;
Azepan-1-yl-(3-bromo-5-furan-2-yl-7-trifluoromethyl-pyrazolo[1,5-*a*]pyrimidin-2-yl)methanone;
5-Benzo[1,3]dioxol-5-yl-7-trifluoromethyl-pyrazolo[1,5-*a*]pyrimidine-3-carboxylic acid cyclohexyl-methyl amide;
(3,4-Dihydro-1 H-isoquinolin-2-yl)-[5-(-4-Methoxy-phenyl-7-trifluoromethyl-pyrazolo[1,5-a]pyrimidin-3-yl]methanone;
Azepan-1-yl-[5-(4-bromo-phenyl)-7-trifluoromethyl-pyrazolo[1,5-a]pyrimidin-3-yl)methanone
Azepan-1-yl-(5,7-diphenyl)-pyrazolo[1,5-a]pyrimidin-3-yl)methanone;
[5-(4-bromo-phenyl)-7-trifluoromethyl-pyrazolo[1,5-a]pyrimidin-3-yl]-(2-methyl-piperidin-1-yl)-methanone;
(5-Furan-2-yl-7-trifluoromethyl-pyrazolo[1,5-a]pyrimidin-3-yl)-(2-methyl-piperidin-1-yl)-methanone;
(2,6-Dimethyl-piperidin-1-yl-)-[7-(4-ethoxy-phenyl)-5-methyl-pyrazolo[1,5-a]pyrimidin-2-yl] methanone;
5-(4-Ethoxy-phenyl)-7-methyl-pyrazolo[1,5-a]pyrimidine-2-carboxylic acid cyclohexyl amide;
Piperidin-1-yl-(5-thiophen-2-yl-7-trifluoromethyl-pyrazolo[1,5-a]pyrimidin-3-yl)methanone;
(5-Furan-2-yl-7-trifluoromethyl-pyrazolo[1,5-a]pyrimidin-3-yl)-(2-methyl-piperidin-1-yl)-methanone;
(2-Methyl-piperidin-1-yl)-(5-thiophen-2-yl-7-trifluoromethyl-pyrazolo[1,5-a]pyrimidin-3-yl)-methanone;
Azepan-1-yl-[5-(4-methoxy-phenyl)-7-trifluoromethyl-pyrazolo[1,5-a]pyrimidin-3-yl]-methanone;
5-Thiophen-2-yl -7-trifluoromethyl-pyrazolo[1,5-a]pyrimidine-3-carboxylic acid benzyl-methyl-amide;
5-p-Tolyl-7-trifluoromethyl-pyrazolo[1,5-a]pyrimidine-3-carboxylic acid (1,3-dimethyl-1 H-pyrazol-4-ylmethyl)-methyl amide;
5-Methyl-7-phenyl-pyrazolo[1,5-a]pyrimidine-2-carboxylic acid cyclohexyl-methyl amide;
7-Methyl-5-phenyl-pyrazolo[1,5-a]pyrimidine-2-carboxylic acid cyclohexyl-methyl amide;
(5-Phenyl-7-trifluoromethyl-pyrazolo[1,5-a]pyrimidin-2-yl)-piperidin-1-yl-methanone;
5-Naphthalen-1-yl-7-trifluoromethyl-pyrazolo[1,5-a]pyrimidine-2-carboxylic acid cyclohexyl-methyl amide;
5-Naphthalen-1-yl-7-trifluoromethyl-pyrazolo[1,5-a]pyrimidine-2-carboxylic acid cyclohexyl amide;
(5-Naphthalen-1-yl-7-trifluoromethyl-pyrazolo[1,5-a]pyrimidin-2-yl)-piperidin-1-yl-methanone
(2-Methyl-piperidin-1-yl)-(5-naphthalen-1-yl-7-trifluoromethyl-pyrazolo[1,5-a]pyrimidin-2-yl)-methanone;
5,7-Diphenyl-pyrazolo[1,5-a]pyrimidine-2-carboxylic acid cyclohexyl amide;
5,7-Diphenyl-pyrazolo[1,5-a]pyrimidine-2-carboxylic acid cyclohexyl-methyl amide;
(5,7-Diphenyl-pyrazolo[1,5-*a*]pyrimidin-2-yl)-(2-methyl-piperidin-1-yl)methanone;
(5,7-Diphenyl-pyrazolo[1,5-*a*]pyrimidin-2-yl)-piperidin-1-yl-methanone;
5-Methyl-7-trifluoromethyl-pyrazolo[1,5-a]pyrimidine-2-carboxylic acid cyclohexyl amide;
(5-Methyl-7-trifluoromethyl-pyrazolo[1,5-*a*]pyrimidin-2-yl)-piperidin-1-yl-methanone;
5-Methyl-7-trifluoromethyl-pyrazolo[1,5-*a*]pyrimidine-2-carboxylic acid cyclohexyl-methyl-amide;
(2-Methyl-piperidin-1-yl)-(5-methyl-7-trifluoromethyl-pyrazolo[1,5-a]pyrimidin-2-yl)-methanone;
5-Phenyl-7-trifluoromethyl-pyrazolo[1,5-a]pyrimidine-2-carboxylic acid cyclohexyl amide;
(5-Phenyl-7-trifluoromethyl-pyrazolo[1,5-*a*]pyrimidin-2-yl)-piperidin-1-yl-methanone;
7-Phenyl-5-trifluoromethyl-pyrazolo[1,5-a]pyrimidine-2-carboxylic acid cyclohexyl-methyl-amide;
5-Phenyl-7-trifluoromethyl-pyrazolo[1,5-a]pyrimidine-3-carboxylic acid cyclohexyl amide;
(5-Phenyl-7-trifluoromethyl-pyrazolo[1,5-a]pyrimidin-3-yl)-piperidin-1-yl-methanone; or
a salt thereof with a pharmaceutically acceptable acid or base, or any optical isomer or mixture of optical isomers, including a racemic mixture, or any tautomeric forms.

In another embodiment, the invention provides the present use of substituted pyrazolo[1,5-a]pyrimidines, or prodrugs thereof of general formula (I) selected from the group consisting of the compounds of examples 1 through 4, examples 4-2 through 4-214, example 5 and examples 5-2 through 5-126, a salt thereof with a pharmaceutically acceptable acid or base, or any optical isomer or mixture of optical isomers, including a racemic mixture, or any tautomeric forms.

In another embodiment, the present invention provides substituted pyrazolo[1,5-a]pyrimidines, or prodrugs thereof of general formula (I) selected from the group consisting of the compounds of examples 1 through 4, examples 4-2 through 4-214, example 5 and examples 5-2 through 5-126, or a salt thereof with a pharmaceutically acceptable acid or base, or any optical isomer or mixture of optical isomers, including a racemic mixture, or any tautomeric forms.

The compounds of the present invention have asymmetric centers and may occur as racemates, racemic mixtures, and as individual enantiomers or diastereoisomers, with all isomeric forms being included in the present invention as well as mixtures thereof.

The present invention also encompasses pharmaceutically acceptable salts of the present compounds. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci., 66, 2 (1977), which is incorporated herein by reference. Examples of metal salts include lithium, sodium, potassium, barium, calcium, magnesium, zinc, calcium salts and the like. Examples of amines and organic amines include ammonium, methylamine, dimethylamine, trimethylamine, ethylamine, diethylamine, propylamine, butylamine, tetramethylamine, ethanolamine, diethanolamine, triethanolamine, meglumine, ethylenediamine, choline, N,N'-dibenzylethylenediamine, N-benzylphenylethylamine, N-methyl-D-glucamine, guanidine and the like. Examples of cationic amino acids include lysine, arginine, histidine and the like.

Further, some of the compounds of the present invention may form solvates with water or common organic solvents. Such solvates are encompassed within the scope of the invention.

The pharmaceutically acceptable salts are prepared by reacting a compound of the present invention with 1 to 4 equivalents of a base such as sodium hydroxide, sodium methoxide, sodium hydride, potassium *tert*-butoxide, calcium hydroxide, magnesium hydroxide and the like, in solvents like ether, THF, methanol, *tert*-butanol, dioxane, isopropanol, ethanol etc. Mixtures of solvents may be used. Organic bases like lysine, arginine, diethanolamine, choline, guandine and their derivatives etc. may also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane etc. Mixture of solvents may also be used.

The stereoisomers of the compounds forming part of this invention may be prepared by using reactants in their single enantiomeric form in the process wherever possible or by conducting the reaction in the presence of reagents or catalysts in their single enantiomer form or by resolving the mixture of stereoisomers by conventional methods. Some of the preferred methods include use of microbial resolution, enzymatic resolution, resolving the diastereomeric salts formed with chiral acids such as mandelic acid, camphorsulfonic acid, tartaric acid, lactic acid, and the like wherever applicable or chiral bases such as brucine, (R)- or (S)-phenylethylamine, cinchona alkaloids and their derivatives and the like. Commonly used methods are compiled by Jaques et al. in "Enantiomers, Racemates and Resolution" (Wiley Interscience, 1981). More specifically the compound of the present invention may be converted to a 1:1 mixture of diastereomeric amides by treating with chiral amines, aminoacids, aminoalcohols derived from aminoacids; conventional reaction conditions may be employed to convert acid into an amide; the diastereomers may be separated either by fractional crystallization or chromatography and the stereoisomers of compound of formula I may be prepared by hydrolysing the pure diastereomeric amide.

Various polymorphs of the compounds forming part of this invention may be prepared by crystallization of said compounds under different conditions. For example, using different solvents commonly used or their mixtures for recrystallization; crystallizations at different temperatures; various modes of cooling, ranging from very fast to very slow cooling during crystallizations. Polymorphs may also be obtained by heating or melting the compound followed by gradual or fast cooling. The presence of polymorphs may be determined by solid probe nmr spectroscopy, ir spectroscopy, differential scanning calorimetry, powder X-ray diffraction or such other techniques.

The invention also encompasses prodrugs of the present compounds, which on administration undergo chemical conversion by metabolic processes before becoming active pharmacological substances. In general, such prodrugs will be functional derivatives of the present compounds, which are readily convertible *in vivo* into the required compound of the present invention. Conventional procedures for the selection and preparation of suitable prodrug derivatives are described, for example, in "Design of Prodrugs", ed. H. Bundgaard, Elsevier, 1985.

It is a well known problem in drug discovery that compounds, such as enzyme inhibitors, may be very potent and selective in biochemical assays, yet be inactive *in vivo.* This lack of so-called bioavailability may be ascribed to a number of different factors such as lack of or poor absorption in the gut, first pass metabolism in the liver and/or poor uptake in cells. Although the factors determining bioavailability are not completely understood, there are many examples in the scientific literature - well known to those skilled in the art - of how to modify compounds, which are potent and selective in biochemical assays but show low or no activity *in vivo,* into drugs that are biologically active.

It is within the scope of the invention to modify the compounds of the present invention, termed the 'original compound', by attaching chemical groups that will improve the bioavailability of said compounds in such a way that the uptake in cells or mammals is facilitated.

Examples of said modifications, which are not intended in any way to limit the scope of the invention, include changing of one or more carboxy groups to esters (for instance methyl esters, ethyl esters, *tert-*butyl, acetoxymethyl, pivaloyloxymethyl esters or other acyloxymethyl esters). Compounds of the invention, original compounds, such modified by attaching chemical groups are termed 'modified compounds'.

The invention also encompasses active metabolites of the present compounds.

The compounds according to the invention alters, and more specifically, reduces the level of active intracellular glucocorticoid and are accordingly useful for the treatment, prevention and/or prophylaxis of disorders and diseases in which such a modulation or reduction is beneficial.

Accordingly, the present compounds may be applicable for the treatment, prevention and/or prophylaxis of the metabolic syndrome, insulin resistance, dyslipidemia, hypertension, obesity, type 2 diabetes, impaired glucose tolerance (IGT), impaired fasting glucose (IFG), Latent Autoimmune Diabetes in the Adult (LADA), type 1 diabetes, diabetic late complications including cardiovascular diseases, cardiovascular disorders, disorders of lipid metabolism, neurodegenerative and psychiatric disorders, dysregulation of intraocular pressure including glaucoma, immune disorders, inappropriate immune responses, musculo-skeletal disorders, gastrointestinal disorders, polycystic ovarie syndrome (PCOS), reduced hair growth or other diseases, disorders or conditions that are influenced by intracellular glucocorticoid levels, adverse effects of increased blood levels of active endogenous or exogenous glucocorticoid, and any combination thereof, adverse effects of increased plasma levels of endogenous active glucocorticoid, Cushing's disease, Cushing's syndrome, adverse effects of glucocorticoid receptor agonist treatment of autoimmune diseases, adverse effects of glucocorticoid receptor agonist treatment of inflammatory diseases, adverse effects of glucocorticoid receptor agonist treatment of diseases with an inflammatory component, adverse effects of glucocorticoid receptor agonist treatment as a part of cancer chemotherapy, adverse effects of glucocorticoid receptor agonist treatment for surgical/post-surgical or other trauma, adverse effects of glucocorticoid receptor agonist therapy in the context of organ or tissue transplantation or adverse effects of glucocorticoid receptor agonist treatment in other diseases, disorders or conditions where glucocorticoid receptor agonists provide clinically beneficial effects.

More specifically the present compounds may be applicable for the treatment, prevention and/or prophylaxis of the metabolic syndrome, type 2 diabetes, diabetes as a consequence of obesity, insulin resistance, hyperglycemia, prandial hyperglycemia, hyperinsulinemia, inappropriately low insulin secretion, impaired glucose tolerance (IGT), impaired fasting glucose (IFG), increased hepatic glucose production, type 1 diabetes, LADA, pediatric diabetes, dyslipidemia, diabetic dyslipidemia, hyperlipidemia, hypertriglyceridemia, hyperlipoproteinemia, hypercholesterolemia, decreased HDL cholesterol, impaired LDL/HDL ratio, other disorders of lipid metabolism, obesity, visceral obesity, obesity as a consequence of diabetes, increased food intake, hypertension, diabetic late complications, micro-/macroalbuminuria, nephropathy, retinopathy, neuropathy, diabetic ulcers, cardiovascular diseases, arteriosclerosis, atherosclerosis, coronary artery disease, cardiac hypertrophy, myocardial ischemia, heart insufficiency, congestional heart failure, stroke, myocardial infarction, arrythmia, decreased blood flow, erectile dysfunction (male or female), myopathy, loss of muscle tissue, muscle wasting, muscle catabolism, osteoporosis, decreased linear growth, neurodegenerative and psychiatric disorders, Alzheimers disease, neuronal death, impaired cognitive function, depression, anxiety, eating disorders, appetite regulation, migraine, epilepsia, addiction to chemical substances, disorders of intraocular pressure, glaucoma, polycystic ovary syndrome (PCOS), inappropriate immune responses, inappropriate T helper-1/T helper-2 polarisation, bacterial infections, mycobacterial infections, fungal infections, viral infections, parasitic infestations, suboptimal responses to immunizations, immune dysfunction, partial or complete baldness, or other diseases, disorders or conditions that are influenced by intracellular glucocorticoid levels and any combination thereof, adverse effects of glucocorticoid receptor agonist treatment of allergic-inflammatory diseases such as asthma and atopic dermatitis, adverse effects of glucocorticoid receptor agonist treatment of disorders of the respiratory system e.g. asthma, cystic fibrosis, emphysema, bronchitis, hypersensitivity, pneumonitis, eosinophilic pneumonias, pulmonary fibrosis, adverse effects of glucocorticoid receptor agonist treatment of inflammatory bowel disease such as Crohn's disease and ulcerative colitis; adverse effects of glucocorticoid receptor agonist treatment of disorders of the immune system, connective tissue and joints e.g. reactive arthritis, rheumatoid arthritis, Sjögren's syndrome, systemic lupus erythematosus, lupus nephritis, Henoch-Schönlein purpura, Wegener's granulomatosis, temporal arteritis, systemic sclerosis, vasculitis, sarcoidosis, dermatomyositis-polymyositis, pemphigus vulgaris; adverse effects of glucocorticoid receptor agonist treatment of endocrinological diseases such as hyperthyroidism, hypoaldosteronism, hypopituitarism; adverse effects of glucocorticoid receptor agonist treatment of hematological diseases e.g. hemolytic anemia, thrombocytopenia, paroxysmal nocturnal hemoglobinuria; adverse effects of glucocorticoid receptor agonist treatment of cancer such as spinal cord diseases, neoplastic compression of the spinal cord, brain tumours, acute lymphoblastic leukemia, Hodgkin's disease, chemotherapy-induced nausea, adverse effects of glucocorticoid receptor agonist treatment of diseases of muscle and at the neuro-muscular joint e.g. myasthenia gravis and heriditary myopathies (e.g. Duchenne muscular dystrophy), adverse effects of glucocorticoid receptor agonist treatment in the context of surgery & transplantation e.g. trauma, post-surgical stress, surgical stress, renal transplantation, liver transplantation, lung transplantation, pancreatic islet transplantation, blood stem cell transplantation, bone marrow transplantation, heart transplantation, adrenal gland transplantation, tracheal transplantation, intestinal transplantation, corneal transplantation, skin grafting, keratoplasty, lens implantation and other procedures where immunosuppression with glucocorticoid receptor agonists is beneficial; adverse effects of glucocorticoid receptor agonist treatment of brain absess, nausea/vomiting, infections, hypercalcemia, adrenal hyperplasia, autoimmune hepatitis, spinal cord diseases, saccular aneurysms or adverse effects to glucocorticoid receptor agonist treatment in other diseases, disorders and conditions where glucocorticoid receptor agonists provide clinically beneficial effects.

Accordingly, in a further aspect the invention relates to a compound according to the invention for use as a pharmaceutical composition.

The invention also relates to pharmaceutical compositions comprising, as an active ingredient, at least one compound according to the invention together with one or more pharmaceutically acceptable carriers or diluents.

The pharmaceutical composition is preferably in unit dosage form, comprising from about 0.05 mg/day to about 2000 mg/day, preferably from about 1 mg/day to about 500 mg/day of a compound according to the invention.

In another embodiment, the patient is treated with a compound according to the invention for at least about 1 week, for at least about 2 weeks, for at least about 4 weeks, for at least about 2 months or for at least about 4 months.

In yet another embodiment, the pharmaceutical composition is for oral, nasal, transdermal, pulmonal or parenteral administration.

Furthermore, the invention relates to the use of a compound according to the invention for the preparation of a pharmaceutical composition for the treatment, prevention and/or prophylaxis of disorders and diseases wherein a modulation or an inhibition of the activity of 11βHSD1 is beneficial.

The invention also relates to a method for the treatment, prevention and/or prophylaxis of disorders and diseases wherein a modulation or an inhibition of the activity of 11βHSD1 is beneficial, the method comprising administering to a subject in need thereof an effective amount of a compound according to the invention.

In a preferred embodiment of the invention the present compounds are used for the preparation of a medicament for the treatment, prevention and/or prophylaxis of any diseases and conditions that are influenced by intracellular glucocorticoid levels as mentioned above.

Thus, in a preferred embodiment of the invention the present compounds are used for the preparation of a medicament for the treatment, prevention and/or prophylaxis of conditions and disorders where a decreased level of active intracellular glucocorticoid is desirable, such as the conditions and diseases mentioned above.

In yet a preferred embodiment of the invention the present compounds are used for the preparation of a medicament for the treatment, prevention and/or prophylaxis of the metabolic syndrome including insulin resistance, dyslipidemia, hypertension and obesity.

In yet another preferred embodiment of the invention the present compounds are used for the preparation of a medicament for the treatment, prevention and/or prophylaxis of type 2 diabetes, impaired glucose tolerance (IGT), impaired fasting glucose (IFG).

In yet another preferred embodiment of the invention the present compounds are used for the preparation of a pharmaceutical composition for the delaying or prevention of the progression from IGT to type 2 diabetes.

In yet another preferred embodiment of the invention the present compounds are used for the preparation of a pharmaceutical composition for the delaying or prevention of the progression of the metabolic syndrome into type 2 diabetes.

In still another preferred embodiment of the invention the present compounds are used for the preparation of a pharmaceutical composition for the treatment, prevention and/or prophylaxis of diabetic late complications including cardiovascular diseases; arteriosclerosis; atherosclerosis.

In a further preferred embodiment of the invention the present compounds are used for the preparation of a pharmaceutical composition for the treatment, prevention and/or prophylaxis of neurodegenerative and psychiatric disorders.

In yet a further preferred embodiment of the invention the present compounds are used for the preparation of a pharmaceutical composition for the treatment, prevention and/or prophylaxis of adverse effects of glucocorticoid receptor agonist treatment or therapy.

In another embodiment of the present invention, the route of administration may be any route which effectively transports a compound according to the invention to the appropriate or desired site of action, such as oral, nasal, buccal, transdermal, pulmonal, or parenteral.

In still a further aspect of the invention the present compounds are administered in combination with one or more further active substances in any suitable ratios. Such further active substances may e.g. be selected from antiobesity agents, antidiabetics, agents modifying the lipid metabolism, antihypertensive agents, glucocorticoid receptor agonists, agents for the treatment and/or prevention of complications resulting from or associated with diabetes and agents for the treatment and/or prevention of complications and disorders resulting from or associated with obesity.

Thus, in a further aspect of the invention the present compounds may be administered in combination with one or more antiobesity agents or appetite regulating agents.

Such agents may be selected from the group consisting of CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, PPAR (peroxisome proliferator-activated receptor) modulators, RXR (retinoid X receptor) modulators, TR β agonists, AGRP (Agouti related protein) inhibitors, H3 histamine antagonists, opioid antagonists (such as naltrexone), exendin-4, GLP-1 and ciliary neurotrophic factor.

In one embodiment of the invention the antiobesity agent is leptin; dexamphetamine or amphetamine; fenfluramine or dexfenfluramine; sibutramine; orlistat; mazindol or phentermine.

Suitable antidiabetic agents include insulin, insulin analogues and derivatives such as those disclosed in EP 792 290 (Novo Nordisk A/S), e.g. N^{εB29}-tetradecanoyl des (B30) human insulin, EP 214 826 and EP 705 275 (Novo Nordisk A/S), e.g. Asp^{B28} human insulin, US 5,504,188 (Eli Lilly), e.g. Lys^{B28} Pro^{B29} human insulin, EP 368 187 (Aventis), e.g. Lantus, which are all incorporated herein by reference, GLP-1 (glucagon like peptide-1) and GLP-1 derivatives such as those disclosed in WO 98/08871 to Novo Nordisk A/S, which is incorporated herein by reference as well as orally active hypoglycaemic agents.

The orally active hypoglycaemic agents preferably comprise sulphonylureas, biguanides, meglitinides, glucosidase inhibitors, glucagon antagonists such as those disclosed in WO 99/01423 to Novo Nordisk A/S and Agouron Pharmaceuticals, Inc., GLP-1 agonists, potassium channel openers such as those disclosed in WO 97/26265 and WO 99/03861 to Novo Nordisk A/S which are incorporated herein by reference, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents and antilipidemic agents as PPARα modulators, PPARδ modulators, cholesterol absorption inhibitors, HSL (hormone-sensitive lipase) inhibitors and HMG CoA inhibitors (statins), nicotinic acid, fibrates, anion exchangers, compounds lowering food intake, bile acid resins, RXR agonists and agents acting on the ATP-dependent potassium channel of the β-cells.

In one embodiment, the present compounds are administered in combination with insulin or an insulin analogue or derivative, such as N^{εB29}-tetradecanoyl des (B30) human insulin, Asp^{B28} human insulin, Lys^{B28} Pro^{B29} human insulin, Lantus®, or a mix-preparation comprising one or more of these.

In a further embodiment the present compounds are administered in combination with a sulphonylurea e.g. tolbutamide, glibenclamide, glipizide or glicazide.

In another embodiment the present compounds are administered in combination with a biguanide e.g. metformin.

In yet another embodiment the present compounds are administered in combination with a meglitinide e.g. repaglinide or senaglinide.

In still another embodiment the present compounds are administered in combination with a thiazolidinedione e.g. troglitazone, ciglitazone, pioglitazone, rosiglitazone or compounds disclosed in WO 97/41097 such as 5-[[4-[3-Methyl-4-oxo-3,4-dihydro-2-quinazolinyl]methoxy]phenyl-methyl]thiazolidine-2,4-dione or a pharmaceutically acceptable salt thereof, preferably the potassium salt.

In yet another embodiment the present compounds may be administered in combination with the insulin sensitizers disclosed in WO 99/19313 such as (-) 3-[4-[2-Phenoxazin-1 0-yl)ethoxy]phenyl]-2-ethoxypropanoic acid or a pharmaceutically acceptable salts thereof, preferably the arginine salt.

In a further embodiment the present compounds are administered in combination with an α-glucosidase inhibitor e.g. miglitol or acarbose.

In another embodiment the present compounds are administered in combination with an agent acting on the ATP-dependent potassium channel of the β-cells e.g. tolbutamide, glibenclamide, glipizide, glicazide or repaglinide.

Furthermore, the present compounds may be administered in combination with nateglinide.

In still another embodiment the present compounds are administered in combination with an antihyperlipidemic agent or antilipidemic agent e.g. cholestyramine, colestipol, clofibrate, gemfibrozil, fenofibrate, bezafibrate, tesaglitazar, EML-4156, LY-818, MK-767, atorvastatin, fluvastatin, lovastatin, pravastatin, simvastatin, acipimox, probucol, ezetimibe or dextrothyroxine.

In a further embodiment the present compounds are administered in combination with more than one of the above-mentioned compounds e.g. in combination with a sulphonylurea and metformin, a sulphonylurea and acarbose, repaglinide and metformin, insulin and a sulphonylurea, insulin and metformin, insulin, insulin and lovastatin, etc.

Further, the present compounds may be administered in combination with one or more antihypertensive agents. Examples of antihypertensive agents are β-blockers such as alprenolol, atenolol, timolol, pindolol, propranolol, metoprolol, bisoprololfumerate, esmolol, acebutelol, metoprolol, acebutolol, betaxolol, celiprolol, nebivolol, tertatolol, oxprenolol, amusolalul, carvedilol, labetalol, β2-receptor blockers e.g. S-atenolol, OPC-1085, ACE (angiotensin converting enzyme) inhibitors such as quinapril, lisinopril, enalapril, captopril, benazepril, perindopril, trandolapril, fosinopril, ramipril, cilazapril, delapril, imidapril, moexipril, spirapril, temocapril, zofenopril, S-5590, fasidotril, Hoechst-Marion Roussel: 100240 (EP 00481522), omapatrilat, gemopatrilat and GW-660511, calcium channel blockers such as nifedipine, felodipine, nicardipine, isradipine, nimodipine, diltiazem, amlodipine, nitrendipine, verapamil, lacidipine, lercanidipine, aranidipine, cilnidipine, clevidipine, azelnidipine, barnidipine, efonodipine, iasidipine, iemildipine, iercanidipine, manidipine, nilvadipine, pranidipine, furnidipine, α-blockers such as doxazosin, urapidil, prazosin, terazosin, bunazosin and OPC-28326, diuretics such as thiazides/sulphonamides (e.g. bendroflumetazide, chlorothalidone, hydrochlorothiazide and clopamide), loop-diuretics (e.g. bumetanide, furosemide and torasemide) and potassium sparing diuretics (e.g. amiloride, spironolactone), endothelin ET-A antagonists such as ABT-546, ambrisetan, atrasentan, SB-234551, Cl-1034, S-0139 and YM-598, endothelin antagonists e.g. bosentan and J-104133, renin inhibitors such as aliskiren, vasopressin V1 antagonists e.g. OPC-21268, vasopressin V2 antagonists such as tolvaptan, SR-121463 and OPC-31260, B-type natriuretic peptide agonists e.g. Nesiritide, angiotensin II antagonists such as irbesartan, candesartancilexetil, losartan, valsartan, telmisartan, eprosartan, candesartan, CL-329167, eprosartan, iosartan, olmesartan, pratosartan, TA-606, and YM-358, 5-HT2 agonists e.g. fenoldopam and ketanserin, adenosine A1 antagonists such as naftopidil, N-0861 and FK-352, thromboxane A2 antagonists such as KT2-962, endopeptidase inhibitors e.g. ecadotril, nitric oxide agonists such as LP-805, dopamine D1 antagonists e.g. MYD-37, dopamine D2 agonists such as nolomirole, n-3 fatty acids e.g. omacor, prostacyclin agonists such as treprostinil, beraprost, PGE1 agonists e.g. ecraprost, Na⁺/K⁺ ATPase modulators e.g. PST-2238, Potassium channel activators e.g. KR-30450, vaccines such as PMD-3117, Indapamides, CGRP-unigene, guanylate cyclase stimulators, hydralazines, methyldopa, docarpamine, moxonidine, CoAprovel, MondoBiotech-811.

Further reference can be made to Remington: The Science and Practice of Pharmacy, 19th Edition, Gennaro, Ed., Mack Publishing Co., Easton, PA, 1995.

Furthermore, the present compounds may be administered in combination with one or more glucocorticoid receptor agonists. Examples of such glucocorticoid receptor agonists are betametasone, dexamethasone, hydrocortisone, methylprednisolone, prednisolone, prednisone, beclomethasone, butixicort, clobetasol, flunisolide, flucatisone (and analogues), momethasone, triamcinolonacetonide, triamcinolonhexacetonide GW-685698, NXC-1015, NXC-1020, NXC-1021, NS-126, P-4112, P-4114, RU-24858 and T-25 series.

It should be understood that any suitable combination of the compounds according to the invention with one or more of the above-mentioned compounds and optionally one or more further pharmacologically active substances are considered to be within the scope of the present invention.

### PHARMACEUTICAL COMPOSITIONS

The compounds of the present invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19th Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as hard or soft capsules, tablets, troches, dragees, pills, lozenges, powders and granules. Where appropriate, they can be prepared with coatings such as enteric coatings or they can be formulated so as to provide controlled release of the active ingredient such as sustained or prolonged release according to methods well-known in the art.

Liquid dosage forms for oral administration include solutions, emulsions, suspensions, syrups and elixirs.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, crèmes, gels, inhalants, dermal patches, implants etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.05 to about 2000 mg, e.g. from about 0.1 to about 1000 mg, from about 0.5 mg to about 500 mg, from about 1 mg to about 200 mg, e.g. about 100 mg.

For parenteral routes, such as intravenous, intrathecal, intramuscular and similar administration, typically doses are in the order of about half the dose employed for oral administration.

The compounds of this invention are generally utilized as the free substance or as a pharmaceutically acceptable salt thereof. Examples are an acid addition salt of a compound having the utility of a free base and a base addition salt of a compound having the utility of a free acid. The term "pharmaceutically acceptable salts" refers to non-toxic salts of the compounds for use according to the present invention which are generally prepared by reaction of the free base with a suitable organic or inorganic acid or by reaction of the acid with a suitable organic or inorganic base. When a compound for use according to the present invention, contains a free base such salts are prepared in a conventional manner by treating a solution or suspension of the compound with a chemical equivalent of a pharmaceutically acceptable acid. When a compounds for use according to the present invention, contains a free acid such salts are prepared in a conventional manner by treating a solution or suspension of the compound with a chemical equivalent of a pharmaceutically acceptable base. Physiologically acceptable salts of a compound with a hydroxy group include the anion of said compound in combination with a suitable cation such as sodium or ammonium ion. Other salts which are not pharmaceutically acceptable may be useful in the preparation of compounds for use according to the present invention and these form a further aspect of the present invention.

For parenteral administration, solutions of the present compounds in sterile aqueous solution, aqueous propylene glycol or sesame or peanut oil may be employed. Such aqueous solutions should be suitable buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of suitable carriers are water, salt solutions, alcohols, polyethylene glycols, polyhydroxyethoxylated castor oil, peanut oil, olive oil, syrup, phospholipids, gelatine, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents.

The pharmaceutical compositions formed by combining the compounds of the invention and the pharmaceutically acceptable carriers are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and which may include a suitable excipient. These formulations may be in the form of powder or granules, as a solution or suspension in an aqueous or non-aqueous liquid, or as an oil-in-water or water-in-oil liquid emulsion.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations. Tablets may contain the active ingredient in admixture with non-toxic pharmaceutically-acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatine or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356,108; 4,166,452; and 4,265,874, incorporated herein by reference, to form osmotic therapeutic tablets for controlled release.

Formulations for oral use may also be presented as hard gelatine capsules where the active ingredient is mixed with an inert solid diluent, for example, calcium carbonate, calcium phosphate or kaolin, or a soft gelatine capsule wherein the active ingredient is mixed with water or an oil medium, for example peanut oil, liquid paraffin, or olive oil.

Aqueous suspensions may contain the active compounds in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present.

The pharmaceutical compositions comprising a compound for use according to the present invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, preservative and flavouring and colouring agent. The pharmaceutical compositions may be in the form of a sterile injectable aqueous or oleaginous suspension. This suspension may be formulated according to the known methods using suitable dispersing or wetting agents and suspending agents described above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution, and isotonic sodium chloride solution. In addition, sterile, fixed oils are conveniently employed as solvent or suspending medium. For this purpose, any bland fixed oil may be employed using synthetic mono- or diglycerides. In addition, fatty acids such as oleic acid find use in the preparation of injectables.

The compositions may also be in the form of suppositories for rectal administration of the compounds of the present invention. These compositions can be prepared by mixing the drug with a suitable non-irritating excipient which is solid at ordinary temperatures but liquid at the rectal temperature and will thus melt in the rectum to release the drug. Such materials include cocoa butter and polyethylene glycols, for example.

For topical use, creams, ointments, jellies, solutions of suspensions, etc., containing the compounds of the present invention are contemplated. For the purpose of this application, topical applications shall include mouth washes and gargles.

The compounds for use according to the present invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

In addition, some of the compounds for use according to the present invention may form solvates with water or common organic solvents. Such solvates are also encompassed within the scope of the present invention.

Thus, in a further embodiment, there is provided a pharmaceutical composition comprising a compound for use according to the present invention, or a pharmaceutically acceptable salt, solvate, or prodrug thereof, and one or more pharmaceutically acceptable carriers, excipients, or diluents.

If a solid carrier is used for oral administration, the preparation may be tabletted, placed in a hard gelatine capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatine capsule or sterile injectable liquid such as an aqueous or non-aqueous liquid suspension or solution.

A typical tablet which may be prepared by conventional tabletting techniques may contain:

### Core:

| | |
|---|---|
| Active compound (as free compound or salt thereof) | 5.0 mg |
| Lactosum Ph. Eur. | 67.8 mg |
| Cellulose, microcryst. (Avicel) | 31.4 mg |
| Amberlite^{®}IRP88* | 1.0 mg |
| Magnesii stearas Ph. Eur. | q.s. |

| | |
|---|---|
| * Polacrillin potassium NF, tablet disintegrant, Rohm and Haas. | |

### Coating:

| | |
|---|---|
| Hydroxypropyl methylcellulose | approx. 9 mg |
| Mywacett 9-40 T** | approx. 0.9 mg |

| | |
|---|---|
| **Acylated monoglyceride used as plasticizer for film coating. | |

The compounds of the invention may be administered to a patient which is a mammal, especially a human in need thereof. Such mammals include also animals, both domestic animals, e.g. household pets, and non-domestic animals such as wildlife.

Any novel feature or combination of features described herein is considered essential to this invention.

The present invention also relate to the below methods of preparing the compounds of the invention.

The present invention is further illustrated in the following representative examples which are, however, not intended to limit the scope of the invention in any way.

### EXAMPLES

The following examples and general procedures refer to intermediate compounds and final products identified in the specification and in the synthesis schemes. The preparation of the compounds of the present invention is described in detail using the following examples. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, that is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed by either elemental analysis or nuclear magnetic resonance (NMR), where peaks assigned to characteristic protons in the title compounds are presented where appropriate. ¹H NMR shifts (δ_{H}) are given in parts per million (ppm) down field from tetramethylsilane as internal reference standard. M.p.: is melting point and is given in °C and is not corrected. Column chromatography was carried out using the technique described by W.C. Still et al., J. Org. Chem. 43: 2923 (1978) on Merck silica gel 60 (Art. 9385). HPLC analyses are performed using 5µm C18 4 x 250 mm column eluted with various mixtures of water and acetonitrile, flow = 1 ml/min, as described in the experimental section.
**Microwave oven synthesis**: The reaction was heated by microwave irradiation in sealed microwave vessels in a single mode Emrys Optimizer EXP from PersonalChemistry®.
**Preparative HPLC: Column**: 1.9 x 15 cm Waters XTerra RP-18. Buffer: linear gradient 5 - 95 % in 15 min, MeCN, 0.1 % TFA, flow rate of 15 ml/min. The pooled fractions are either evaporated to dryness *in* vacuo, or evaporated *in* vacuo until the MeCN is removed, and then frozen and freeze dried.

The abbreviations as used in the examples have the following meaning:

| | |
|---|---|
| TLC: | Thin layer chromatography |
| CDCl₃: | Deuterio chloroform |
| CD₃OD: | Tetradeuterio methanol |
| DIPEA: | Diisopropylethylamine |
| DMSO-d₆: | Hexadeuterio dimethylsulfoxide |
| DMSO: | Dimethylsulfoxide |
| THF: | Tetrahydrofuran |
| DMF: | N,N-dimethylformamide |
| HOBT: | 1-Hydroxy-benzotriazole |
| EDAC: | 1-(3-Dimethylaminopropyl)-3-ethylcarbodiimide, hydrochloride |
| min: | minutes |
| hrs: | hours |

The compounds of the invention are prepared as illustrated in the following reaction scheme 1:

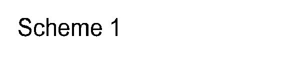

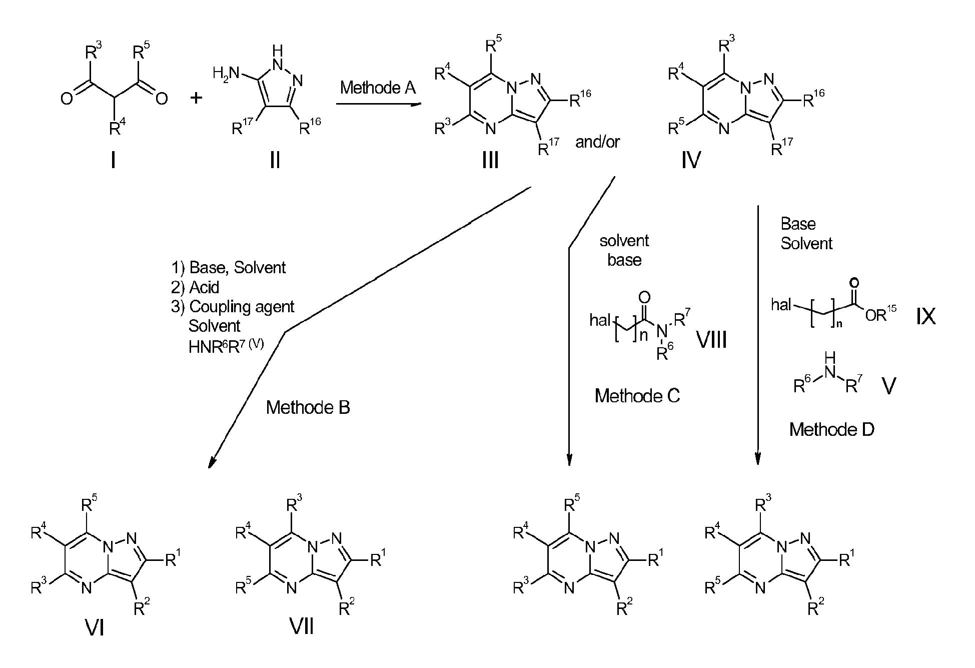

### General method A:

By allowing a 1,3 dicarbonylderivativ (I), wherein R³, R⁴ and R⁵ are as defined above, to react with a 5-amino-pyrazole (II), wherein R¹⁶ and R¹⁷ independently are selected from the group consisting of hydrogen, hal, CO₂R¹⁵, NO₂, OH or SH, wherein R¹⁵ are defined as above, in AcOH at elevated temperature or in a solvent such as EtOH, toluene and the like, catalysed by a base such as with piperidine at elevated temperature, affording the two regio isomeric compounds (III) and (IV), wherein R³, R⁴, R⁵, R¹⁶ and R¹⁷ are as defined above; the two regio-isomers (III) and (IV) can be separated by methods known those skilled in the art.

### General method B:

By allowing a pyrazolo[1,5-a]pyrimidines (III) or (IV) wherein R³, R⁴ and R⁵ are as defined above and one of R¹⁶ and R¹⁷ are CO₂R¹⁵, wherein R¹⁵ are defined as above and the other is hydrogen, is transformed into the corresponding acid by methods known to those skilled in the art and coupled with an amine (V) wherein R⁶ and R⁷ are as defined above, under standard amide forming conditions (e.g. HOBT, EDAC and DIPEA in dry THF) affording pyrazolo[1,5-a]pyrimidines (VI) and/or (VII), wherein R¹, R², R³, R⁴ and R⁵ are defined as above.

### General method C:

By allowing a pyrazolo[1,5-a]pyrimidines (III) or (IV) wherein R³, R⁴ and R⁵ are as defined above and one of R¹⁶ and R¹⁷ are OH or SH and the other are hydrogen or halo, to react with a halo-amide (VIII), wherein hal is halo, R⁶ and R⁷ are as defined above and n is 1 to 6, in a solvent such as MeOH, EtOH, *i*-PrOH, DMF, DMSO, NMP, acetone, THF, dichloromethane and the like under basic conditions (e.g. triethylamine, K₂CO₃, NaOH, NaH, n-BuLi and the like) affording pyrazolo[1,5-a]pyrimidines (VI) and/or (VII), wherein R¹, R², R³, R⁴ and R⁵ are defined as above.

### General method D:

By allowing a pyrazolo[1,5-a]pyrimidines (III) or (IV) wherein R³, R⁴ and R⁵ are as defined above and one of R¹⁶ and R¹⁷ are OH or SH and the other are hydrogen or halo to react with a halo-ester (IX); wherein hal is halo, R¹⁵ is as defined above and n is 1 to 6, in a solvent such as MeOH, EtOH, *i*-PrOH, DMF, DMSO, NMP, acetone, THF, dichloromethane and the like under basic conditions (e.g. triethylamine, K₂CO₃, NaOH, NaH, n-BuLi and the like) affording a pyrazolo[1,5-a]pyrimidines with a ester group. This is transformed into the corresponding acid by methods known to those skilled in the art and coupled with an amine (V), wherein R⁶ and R⁷ are as defined above, under standard amide forming conditions (e.g. HOBT, EDAC and DIPEA in dry THF) affording pyrazolo[1,5-a]pyrimidines (VI) and/or (VII), wherein R¹, R², R³, R⁴ and R⁵ are defined as above.

### Example 1 (General method (A))

### 5-Methyl-7-trifluoromethyl-pyrazolo[1,5-a]pyrimidine-2-carboxylic acid methyl ester

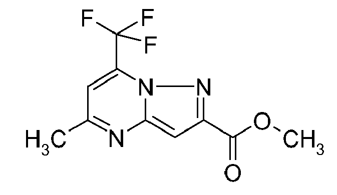

A mixture of 5-amino-1*H*-pyrazole-3-carboxylic acid methyl ester (0.99 g, 7.0 mmol) and 1,1,1-triflouro-2,4-pentanedione (1,08 g, 7.0 mmol) in acetic acid (20ml) was refluxed in a reaction flask for 16 hrs. The reaction mixture was cooled to room temperature, the solid product filtered off and dried *in* vacuo affording 0.74 g (41 %) of the title compound as a solid. ¹H NMR (400 MHz, CDCl₃) δ 2.72 (s, 3H), 4.01 (s, 3H), 7.18 (s, 1 H), 8.27 (s, 1 H).

### Example 2 (General method (A))

### 2A: 5-Methyl-7-phenyl-pyrazolo[1,5-a]pyrimidine-2-carboxylic acid methyl ester

### 2B: 7-Methyl-5-phenyl-pyrazolo[1,5-a]pyrimidine-2-carboxylic acid methyl ester

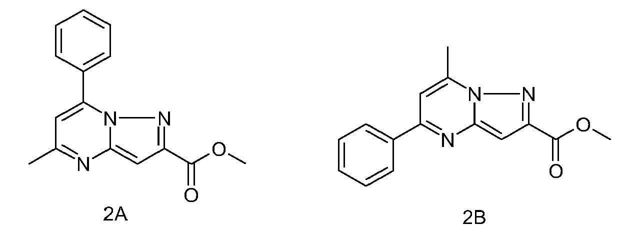

A mixture of 5-amino-1H-pyrazole-3-carboxylic acid methyl ester (0.55 g, 3.9 mmol) and 1-Benzoylacetone (0.63 g, 3.9 mmol) in acetic acid (10 ml) was heated to 140 °C in a sealed tube for 2 hrs. using a microwave oven. The reaction mixture was cooled to room temperature and the volatiles evaporated *in vacuo.* The crude product was purified by silicagel chromatography using DCM as eluent. Pure fractions were collected and the solvent evaporated *in* vacuo affording the two region-isomeric product 2A (570 mg, 55%) and 2B (150 mg, 15%).
**2A**: ¹H NMR (400 MHz, CDCl₃) δ 2.68 (s, 3H), 3.98 (s, 3H), 6.92 (s, 1 H), 7.17 (s, 1 H), 7.56-7.59 (m, 3H), 8.10 (m, 2H).
**2B**: ¹H NMR (400 MHz, CDCl₃) δ 2.93 (s, 3H), 4.04 (s, 3H), 7.24 (s, 1 H), 7.30 (s, 1 H), 7.52-7.54 (m, 3H), 8.10 (m, 2H).

### Example 3 (General method (B))

### (5-methyl-7-trifluoromethyl-pyrazolof1,5-alpyrimidin-2-yl)- (piperidin-1-yl)methanone

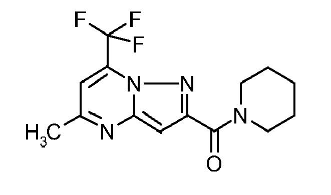

To 5-methyl-7-trifluoromethyl-pyrazolo[1,5-a]pyrimidine-2-carboxylic acid methyl ester (648 mg, 2.5 mmol) in ethanol (35 ml) was added 1 N NaOH (5 ml) and the resulting mixture stirred at room temperature for 3.5 hrs. The solvent was removed *in* vacuo and to the residue was added 1 N HCl (6ml). The solid 5-methyl-7-trifluoromethyl-pyrazolo[1,5-a]pyrimidine-2-carboxylic acid was collected by filtration and dried *in* vacuo. A mixture of 5-methyl-7-trifluoromethyl-pyrazolo[1,5-a]pyrimidine-2-carboxylic acid; (98 mg, 0.4 mmol), HOBT (59 mg, 0.44 mmol), EDAC (84 mg, 0.44 mmol) and diisopropylethylamine (57 mg, 0.44 mmol) in THF (5 ml) was stirred at room temperature for 30 min. and then added 2-methylpiperidine (38 mg, 0.44 mmol) and stirred for 16 hrs. The reaction mixture was quenched by addition of water (50 ml) and extracted with ethyl acetate (2 x 50 ml). The organic phase dried (MgSO₄), filtered and the volatiles evaporated *in* vacuo affording 106 mg (85%) of the title compound as a solid.
¹H NMR (400 MHz, CDCl₃) δ 1.63 (m, 2H), 1.71 (m, 4H), 2.70 (s, 3H), 3.76 (m, 4H), 7.03 (s, 1 H), 7.09 (s, 1 H).

### Example 4 (General method (B))

### (5-methyl-7-phenyl-pyrazolo[1,5-a]pyrimidine-2-carboxylic acid cyclohexyl-methyl amide

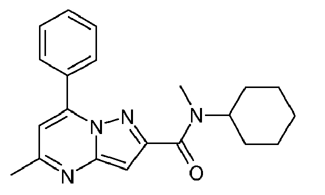

To 5-methyl-7-phenyl-pyrazolo[1,5-a]pyrimidine-2-carboxylic acid methyl ester (300 mg, 1.12 mmol) in ethanol (100 ml) was added 1 N NaOH (4 ml) and stirred at room temperature for 16 hrs. The solvent was removed in vacuo and to the residue was added 1 N HCl (6 ml). The solid 5-methyl-7-phenyl-pyrazolo[1,5-a]pyrimidine-2-carboxylic acid (284 mg, 99%) was collected by filtration and dried in vacuo. A mixture of 5-methyl-7-phenyl-pyrazolo[1,5-a]pyrimidine-2-carboxylic acid; (284 mg, 1.1 mmol), HOBT (167 mg, 1.23 mmol), EDAC (237 mg, 1.23 mmol) and diisopropylethylamine (159 mg, 1.23 mmol) in THF (5 ml) was stirred at room temperature for 30 min. and then added N-methyl-cyclohexylamine (127 mg, 1.21 mmol) and stirred for 16 hrs. The reaction mixture was quenched by addition of water and extracted with EtOAc (2 x 50 ml). The combined organic phases were dried (MgSO₄), filtered and the volatiles evaporated *in* vacuo affording 386 mg (94 %) of the title compound as a solid.
¹H NMR (400 MHz, CDCl₃) δ 1.05 (m, 2H), 1.55 (m, 2H) 1.65-1.80 (m, 4H), 2.67 (s, 3H), 3.00 (s, 3H) 3.26 (m, 1 H), 6.85 (s, 1 H) 6.96 (s, 1 H), 7.54 (m, 3H), 8.02 (m, 2H).
Mp:158 - 162 °C
The following compounds were made in a similar way as described in Example 4 above

| **No** | **Structure** | **MW** | **IUPAC name** |
|---|---|---|---|
| 4-2 | 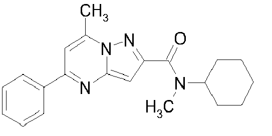 | | 7-Methyl-5-phenyl-pyrazolo[1,5-a]pyrimidine-2-carboxylic acid cyclohexyl-methyl-amide |
| 4-3 | 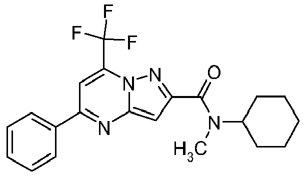 | 402.42 | 7-Phenyl-5-trifluoromethyl-pyrazolo[1,5-a]pyrimidine-2-carboxylic acid cyclohexyl-methyl-amide |
| 4-4 | 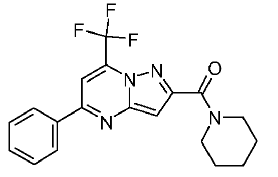 | 374.37 | (5-Phenyl-7-trifluoromethyl-pyrazolo[1,5-a]pyrimidin-2-yl)-piperidin-1-yl-methanone |
| 4-5 | 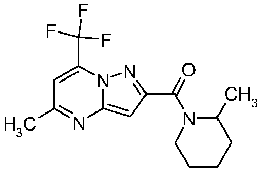 | 326.32 | (2-Methyl-piperidin-1-yl)-(5-methyl-7-trifluoromethyl-pyrazolo[1,5-a]pyrimidin-2-yl)-methanone |
| 4-6 | 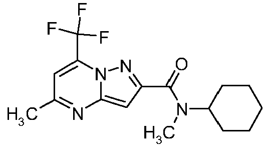 | 340.35 | 5-Methyl-7-trifluoromethyl-pyrazolo[1,5-a]pyrimidine-2-carboxylic acid cyclohexyl-methyl-amide |
| 4-7 | 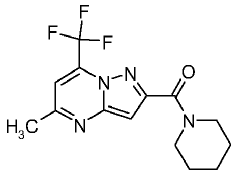 | 312.30 | (5-Methyl-7-trifluoromethyl-pyrazolo[1,5-a]pyrimidin-2-yl)-piperidin-1-yl-methanone |
| 4-8 | 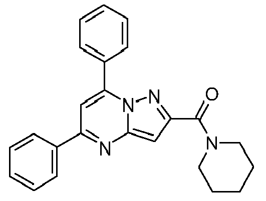 | 382.47 | (5,7-Diphenyl-pyrazolo[1,5-a]pyrimidin-2-yl)-piperidin-1-yl-methanone |
| 4-9 | 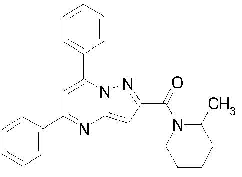 | 396.50 | (5,7-Diphenyl-pyrazolo[1,5-a]pyrimidin-2-yl)-(2-methyl-piperidin-1-yl)-methanone |
| 4-10 | 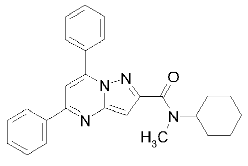 | 410.52 | 5,7-Diphenyl-pyrazolo[1,5-a]pyrimidine-2-carboxylic acid cyclohexyl-methyl-amide |
| 4-11 | 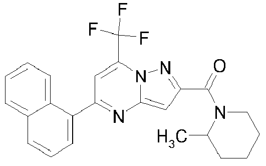 | 438.46 | (2-Methyl-piperidin-1-yl)-(5-naphthalen-1-yl-7-trifluoromethyl-pyrazolo[1,5-a]pyrimidin-2-yl)-methanone |
| 4-12 | 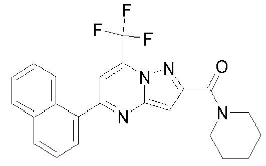 | 424.43 | (5-Naphthalen-1-yl-7-trifluoromethyl-pyrazolo[1,5-a]pyrimidin-2-yl)-piperidin-1-yl-methanone |
| 4-13 | 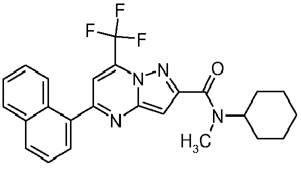 | 452.48 | 5-Naphthalen-1-yl-7-trifluoromethyl-pyrazolo[1,5-a]pyrimidine-2-carboxylic acid cyclohexyl-methyl-amide |
| 4-14 | 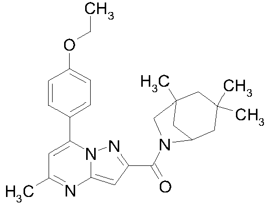 | 432.57 | [7-(4-Ethoxy-phenyl)-5-methyl-pyrazolo[1,5-a]pyrimidin-2-yl]-(1,3,3-trimethyl-6-aza-bicyclo[3.2.1]oct-6-yl)-methanone |
| 4-15 | 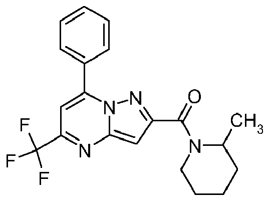 | 388.40 | (2-Methyl-piperidin-1-yl)-(7-phenyl-5-trifluoromethyl-pyrazolo[1,5-a]pyrimidin-2-yl)-methanone |
| 4-16 | 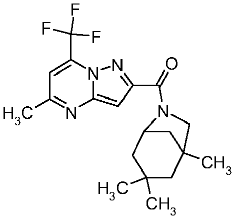 | 380.42 | (5-Methyl-7-trifluoromethyl-pyrazolo[1,5-a]pyrimidin-2-yl)-(1,3,3-trimethyl-6-aza-bicyclo[3.2.1]oct-6-yl)-methanone |
| 4-17 | 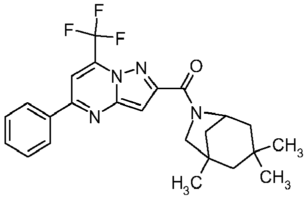 | 442.49 | (5-Phenyl-7-trifluoromethyl-pyrazolo[1,5-a]pyrimidin-2-yl)-(1,3,3-trimethyl-6-aza-bicyclo[3.2.1]oct-6-yl)-methanone |
| 4-18 | 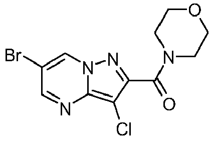 | 345.59 | (6-Bromo-3-chloro-pyrazolo[1,5-a]pyrimidin-2-yl)-morpholin-4-yl-methanone |
| 4-19 | 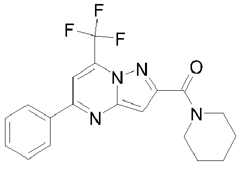 | 374.37 | (5-Phenyl-7-trifluoromethyl-pyrazolo[1,5-a]pyrimidin-2-yl)-piperidin-1-yl-methanone |
| 4-20 | 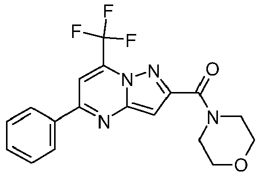 | 376.34 | Morpholin-4-yl-(5-phenyl-7-trifluoromethyl-pyrazolo[1,5-a]pyrimidin-2-yl)-methanone |
| 4-21 | 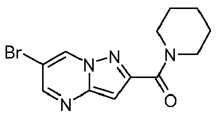 | 309.17 | (6-Bromo-pyrazolo[1,5-a]pyrimidin-2-yl)-piperidin-1-yl-methanone |
| 4-22 | 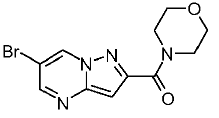 | 311.14 | (6-Bromo-pyrazolo[1,5-a]pyrimidin-2-yl)-morpholin-4-yl-methanone |
| 4-23 | 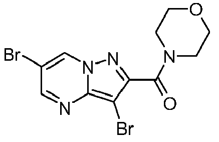 | 390.05 | (3,6-Dibromo-pyrazolo[1,5-a]pyrimidin-2-yl)-morpholin-4-yl-methanone |
| 4-24 | 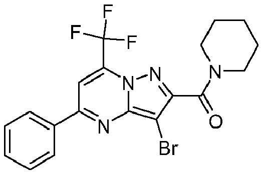 | 453.27 | (3-Bromo-5-phenyl-7-trifluoromethyl-pyrazolo[1,5-a]pyrimidin-2-yl)-piperidin-1-yl-methanone |
| 4-25 | 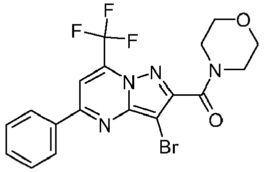 | 455.24 | (3-Bromo-5-phenyl-7-trifluoromethyl-pyrazolo[1,5-a]pyrimidin-2-yl)-morpholin-4-yl-methanone |
| 4-26 | 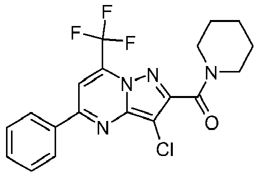 | 408.81 | (3-Chloro-5-phenyl-7-trifluoromethyl-pyrazolo[1,5-a]pyrimidin-2-yl)-piperidin-1-yl-methanone |
| 4-27 | 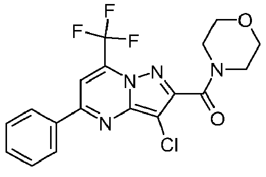 | 410.79 | (3-Chloro-5-phenyl-7-trifluoromethyl-pyrazolo[1,5-a]pyrimidin-2-yl)-morpholin-4-yl-methanone |
| 4-28 | 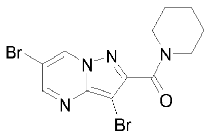 | 388.07 | (3,6-Dibromo-pyrazolo[1,5-a]pyrimidin-2-yl)-piperidin-1-yl-methanone |
| 4-29 | 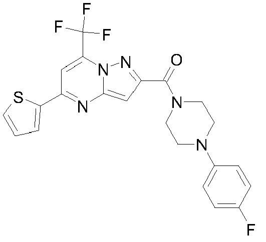 | 475.47 | [4-(4-Fluoro-phenyl)-piperazin-1-yl]-(5-thiophen-2-yl-7-trifluoromethyl-pyrazolo[1,5-a]pyrimidin-2-yl)-methanone |
| 4-30 | 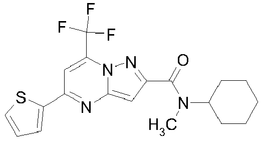 | 408.45 | 5-Thiophen-2-yl-7-trifluoromethyl-pyrazolo[1,5-a]pyrimidine-2-carboxylic acid cyclohexyl-methyl-amide |
| 4-31 | 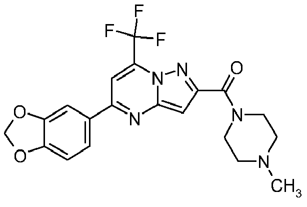 | 433.39 | (5-Benzo[1,3]dioxol-5-yl-7-trifluoromethyl-pyrazolo[1,5-a]pyrimidin-2-yl)-(4-methyl-piperazin-1-yl)-methanone |
| 4-32 | 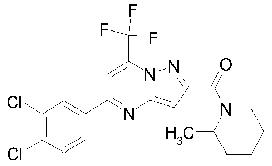 | 457.29 | [5-(3,4-Dichloro-phenyl)-7-trifluoromethyl-pyrazolo[1,5-a]pyrimidin-2-yl]-(2-methyl-piperidin-1-yl)-methanone |
| 4-33 | 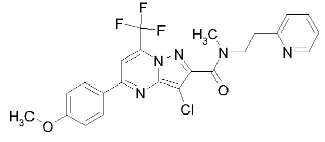 | 489.89 | 3-Chloro-5-(4-methoxy-phenyl)-7-trifluoromethyl-pyrazolo[1,5-a]pyrimidine-2-carboxylic acid methyl-(2-pyridin-2-yl-ethyl)-amide |
| 4-34 | 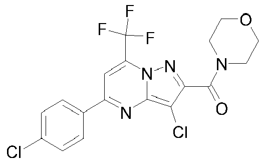 | 445.23 | [3-Chloro-5-(4-chloro-phenyl)-7-trifluoromethyl-pyrazolo[1,5-a]pyrimidin-2-yl]-morpholin-4-yl-methanone |
| 4-35 | 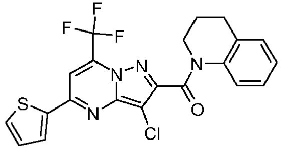 | 462.88 | (3-Chloro-5-thiophen-2-yl-7-trifluoromethyl-pyrazolo[1,5-a]pyrimidin-2-yl)-(3,4-dihydro-2H-quinolin-1-yl)-methanone |
| 4-36 | 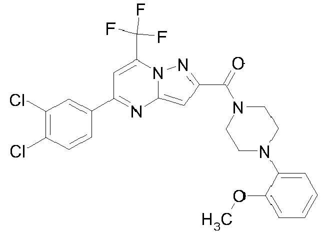 | 550.37 | [5-(3,4-Dichloro-phenyl)-7-trifluoromethyl-pyrazolo[1,5-a]pyrimidin-2-yl]-[4-(2-methoxy-phenyl)-piperazin-1-yl]-methanone |
| 4-37 | 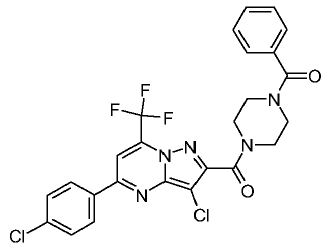 | 548.36 | (4-Benzoyl-piperazin-1-yl)-[3-chloro-5-(4-chloro-phenyl)-7-trifluoromethyl-pyrazolo[1,5-a]pyrimidin-2-yl]-methanone |
| 4-38 | 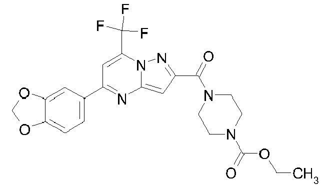 | 491.43 | 4-(5-Benzo[1,3]dioxol-5-yl-7-trifluoromethyl-pyrazolo[1,5-a]pyrimidine-2-carbonyl)-piperazine-1-carboxylic acid ethyl ester |
| 4-39 | 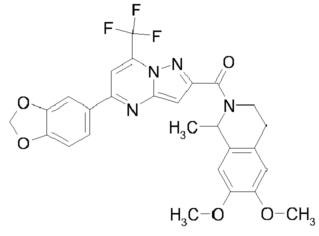 | 540.50 | (5-Benzo[1,3]dioxol-5-yl-7-trifluoromethyl-pyrazolo[1,5-a]pyrimidin-2-yl)-(6,7-dimethoxy-1-methyl-3,4-dihydro-1H-isoquinolin-2-yl)-methanone |
| 4-40 | 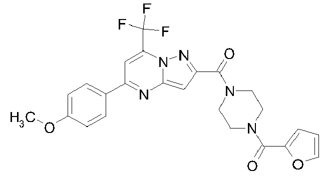 | 499.45 | [4-(Furan-2-carbonyl)-piperazin-1-yl]-[5-(4-methoxy-phenyl)-7-trifluoromethyl-pyrazolo[1,5-a]pyrimidin-2-yl]-methanone |
| 4-41 | 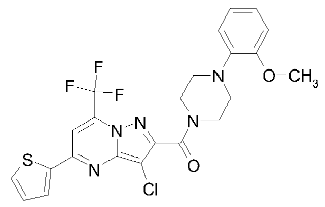 | 521.95 | (3-Chloro-5-thiophen-2-yl-7-trifluoromethyl-pyrazolo[1,5-a]pyrimidin-2-yl)-[4-(2-methoxy-phenyl)-piperazin-1-yl]-methanone |
| 4-42 | 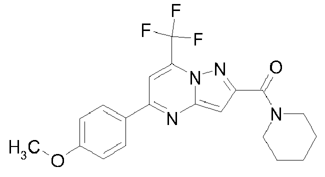 | 404.40 | [5-(4-Methoxy-phenyl)-7-trifluoromethyl-pyrazolo[1,5-a]pyrimidin-2-yl]-piperidin-1-yl-methanone |
| 4-43 | 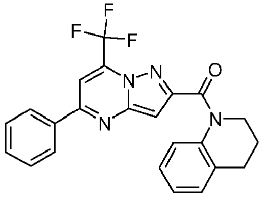 | 422.41 | (3,4-Dihydro-2H-quinolin-1-yl)-(5-phenyl-7-trifluoromethyl-pyrazolo[1,5-a]pyrimidin-2-yl)-methanone |
| 4-44 | 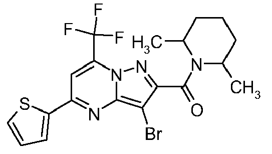 | 487.35 | (3-Bromo-5-thiophen-2-yl-7-trifluoromethyl-pyrazolo[1,5-a]pyrimidin-2-yl)-(2,6-dimethyl-piperidin-1-yl)-methanone |
| 4-45 | 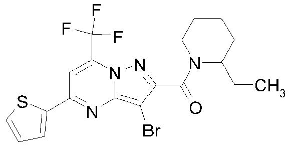 | 487.35 | (3-Bromo-5-thiophen-2-yl-7-trifluoromethyl-pyrazolo[1,5-a]pyrimidin-2-yl)-(2-ethyl-piperidin-1-yl)-methanone |
| 4-46 | 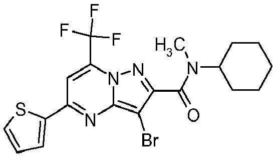 | 487.35 | 3-Bromo-5-thiophen-2-yl-7-trifluoromethyl-pyrazolo[1,5-a]pyrimidine-2-carboxylic acid cyclohexyl-methyl-amide |
| 4-47 | 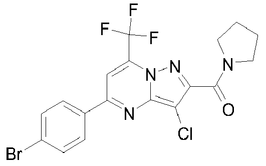 | 473.69 | [5-(4-Bromo-phenyl)-3-chloro-7-trifluoromethyl-pyrazolo[1,5-a]pyrimidin-2-yl]-pyrrolidin-1-yl-methanone |
| 4-48 | 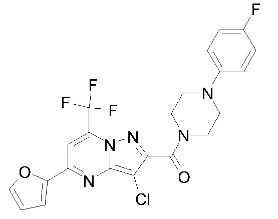 | 493.85 | (3-Chloro-5-furan-2-yl-7-trifluoromethyl-pyrazolo[1,5-a]pyrimidin-2-yl)-[4-(4-fluoro-phenyl)-piperazin-1-yl]-methanone |
| 4-49 | 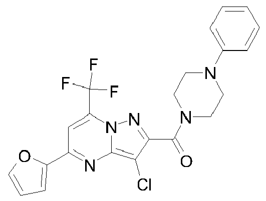 | 475.86 | (3-Chloro-5-furan-2-yl-7-trifluoromethyl-pyrazolo[1,5-a]pyrimidin-2-yl)-(4-phenyl-piperazin-1-yl)-methanone |
| 4-50 | 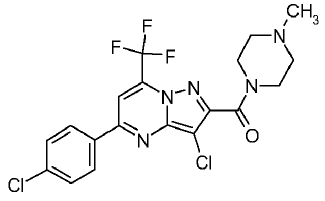 | 458.27 | [3-Chloro-5-(4-chloro-phenyl)-7-trifluoromethyl-pyrazolo[1,5-a]pyrimidin-2-yl]-(4-methyl-piperazin-1-yl)-methanone |
| 4-51 | 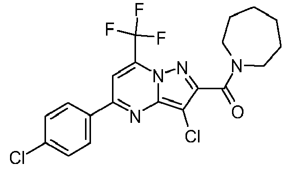 | 457.29 | Azepan-1-yl-[3-chloro-5-(4-chloro-phenyl)-7-trifluoromethyl-pyrazolo[1,5-a]pyrimidin-2-yl]-methanone |
| 4-52 | 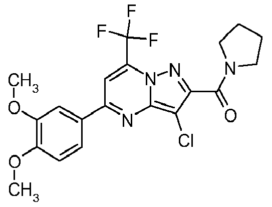 | 454.84 | [3-Chloro-5-(3,4-dimethoxy-phenyl)-7-trifluoromethyl-pyrazolo[1,5-a]pyrimidin-2-yl]-pyrrolidin-1-yl-methanone |
| 4-53 | 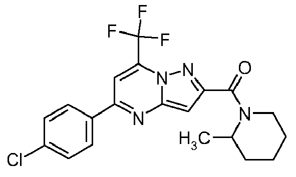 | 422.84 | [5-(4-Chloro-phenyl)-7-trifluoromethyl-pyrazolo[1,5-a]pyrimidin-2-yl]-(2-methyl-piperidin-1-yl)-methanone |
| 4-54 | 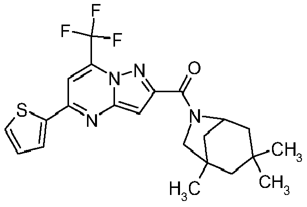 | 448.51 | (5-Thiophen-2-yl-7-trifluoromethyl-pyrazolo[1,5-a]pyrimidin-2-yl)-(1,3,3-trimethyl-6-aza-bicyclo[3.2.1]oct-6-yl)-methanone |
| 4-55 | 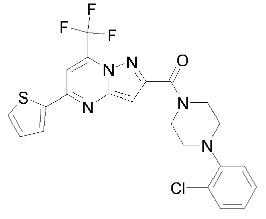 | 491.93 | [4-(2-Chloro-phenyl)-piperazin-1-yl]-(5-thiophen-2-yl-7-trifluoromethyl-pyrazolo[1,5-a]pyrimidin-2-yl)-methanone |
| 4-56 | 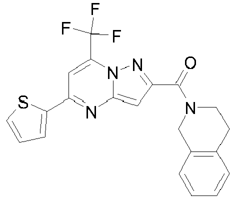 | 428.44 | (3,4-Dihydro-1H-isoquinolin-2-yl)-(5-thiophen-2-yl-7-trifluoromethyl-pyrazolo[1,5-a]pyrimidin-2-yl)-methanone |
| 4-57 | 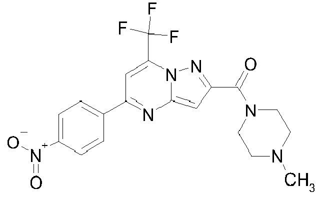 | 434.38 | (4-Methyl-piperazin-1-yl)-[5-(4-nitro-phenyl)-7-trifluoromethyl-pyrazolo[1,5-a]pyrimidin-2-yl]-methanone |
| 4-58 | 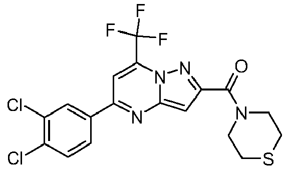 | 461.30 | [5-(3,4-Dichloro-phenyl)-7-trifluoromethyl-pyrazolo[1,5-a]pyrimidin-2-yl]-thiomorpholin-4-yl-methanone |
| 4-59 | 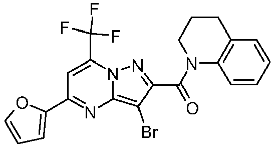 | 491.28 | (3-Bromo-5-furan-2-yl-7-trifluoromethyl-pyrazolo[1,5-a]pyrimidin-2-yl)-(3,4-dihydro-2H-quinolin-1-yl)-methanone |
| 4-60 | 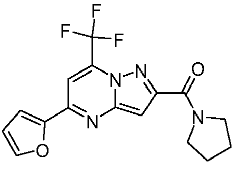 | 350.30 | (5-Furan-2-yl-7-trifluoromethyl-pyrazolo[1,5-a]pyrimidin-2-yl)-pyrrolidin-1-yl-methanone |
| 4-61 | 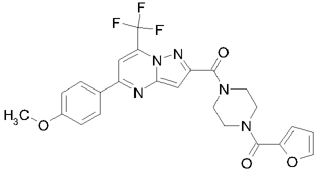 | 499.45 | [4-(Furan-2-carbonyl)-piperazin-1-yl]-[5-(4-methoxy-phenyl)-7-trifluoromethyl-pyrazolo[1,5-a]pyrimidin-2-yl]-methanone |
| 4-62 | 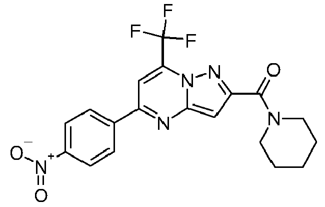 | 419.37 | [5-(4-Nitro-phenyl)-7-trifluoromethyl-pyrazolo[1,5-a]pyrimidin-2-yl]-piperidin-1-yl-methanone |
| 4-63 | 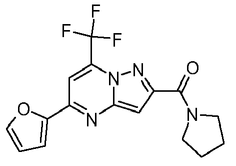 | 350.30 | (5-Furan-2-yl-7-trifluoromethyl-pyrazolo[1,5-a]pyrimidin-2-yl)-pyrrolidin-1-yl-methanone |
| 4-64 | 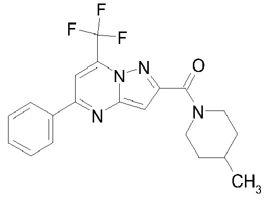 | 388.40 | (4-Methyl-piperidin-1-yl)-(5-phenyl-7-trifluoromethyl-pyrazolo[1,5-a]pyrimidin-2-yl)-methanone |
| 4-65 | 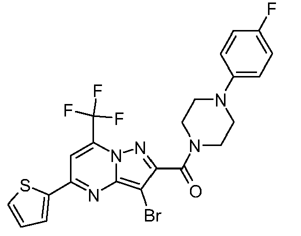 | 554.37 | (3-Bromo-5-thiophen-2-yl-7-trifluoromethyl-pyrazolo[1,5-a]pyrimidin-2-yl)-[4-(4-fluoro-phenyl)-piperazin-1-yl]-methanone |
| 4-66 | 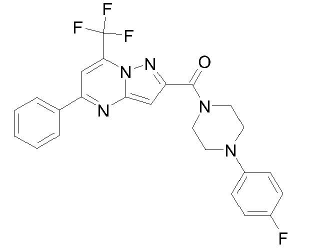 | 469.45 | [4-(4-Fluoro-phenyl)-piperazin-1-yl]-(5-phenyl-7-trifluoromethyl-pyrazolo[1,5-a]pyrimidin-2-yl)-methanone |
| 4-67 | 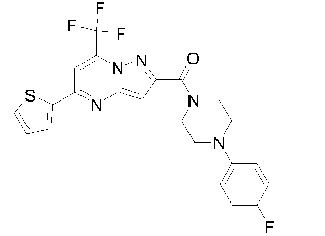 | 475.47 | [4-(4-Fluoro-phenyl)-piperazin-1-yl]-(5-thiophen-2-yl-7-trifluoromethyl-pyrazolo[1,5-a]pyrimidin-2-yl)-methanone |
| 4-68 | 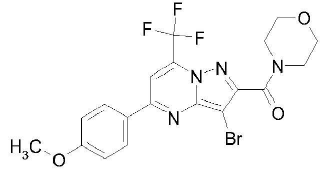 | 485.27 | [3-Bromo-5-(4-methoxy-phenyl)-7-trifluoromethyl-pyrazolo[1,5-a]pyrimidin-2-yl]-morpholin-4-yl-methanone |
| 4-69 | 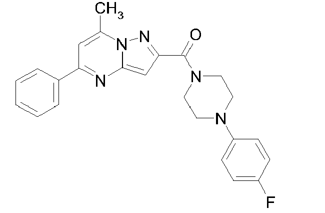 | 415.47 | [4-(4-Fluoro-phenyl)-piperazin-1-yl]-(7-methyl-5-phenyl-pyrazolo[1,5-a]pyrimidin-2-yl)-methanone |
| 4-70 | 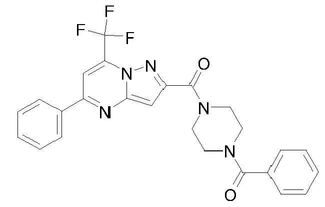 | 479.47 | (4-Benzoyl-piperazin-1-yl)-(5-phenyl-7-trifluoromethyl-pyrazolo[1,5-a]pyrimidin-2-yl)-methanone |
| 4-71 | 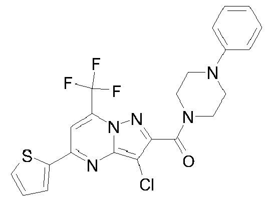 | 491.93 | (3-Chloro-5-thiophen-2-yl-7-trifluoromethyl-pyrazolo[1,5-a]pyrimidin-2-yl)-(4-phenyl-piperazin-1-yl)-methanone |
| 4-72 | 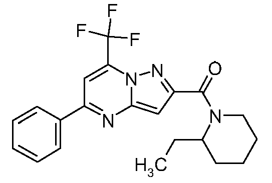 | 402.42 | (2-Ethyl-piperidin-1-yl)-(5-phenyl-7-trifluoromethyl-pyrazolo[1,5-a]pyrimidin-2-yl)-methanone |
| 4-73 | 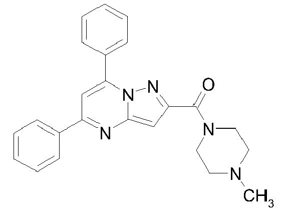 | 397.48 | (5,7-Diphenyl-pyrazolo[1,5-a]pyrimidin-2-yl)-(4-methyl-piperazin-1-yl)-methanone |
| 4-74 | 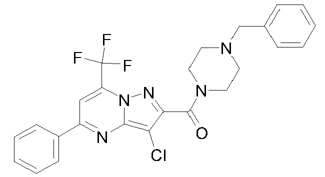 | 499.93 | (4-Benzyl-piperazin-1-yl)-(3-chloro-5-phenyl-7-trifluoromethyl-pyrazolo[1,5-a]pyrimidin-2-yl)-methanone |
| 4-75 | 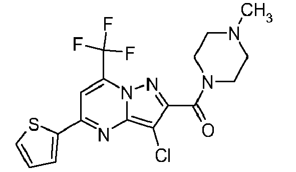 | 429.85 | (3-Chloro-5-thiophen-2-yl-7-trifluoromethyl-pyrazolo[1,5-a]pyrimidin-2-yl)-(4-methyl-piperazi n-1-yl)-methanone |
| 4-76 | 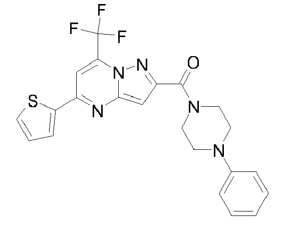 | 457.48 | (4-Phenyl-piperazin-1-yl)-(5-thiophen-2-yl-7-trifluoromethyl-pyrazolo[1,5-a]pyrimidin-2-yl)-methanone |
| 4-77 | 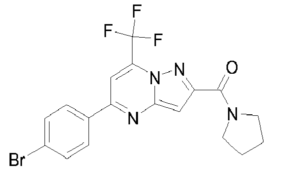 | 439.24 | [5-(4-Bromo-phenyl)-7-trifluoromethyl-pyrazolo[1,5-a]pyrimidin-2-yl]-pyrrolidin-1-yl-methanone |
| 4-78 | 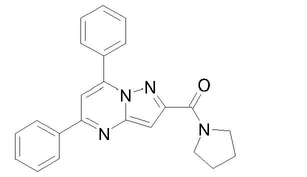 | 368.44 | (5,7-Diphenyl-pyrazolo[1,5-a]pyrimidin-2-yl)-pyrrolidin-1-yl-methanone |
| 4-79 | 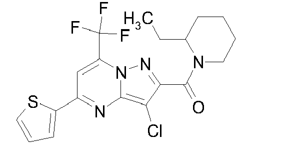 | 442.89 | (3-Chloro-5-thiophen-2-yl-7-trifluoromethyl-pyrazolo[1,5-a]pyrimidin-2-yl)-(2-ethyl-piperidin-1-yl)-methanone |
| 4-80 | 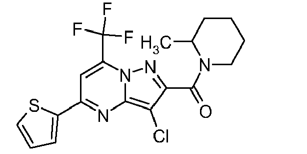 | 428.87 | (3-Chloro-5-thiophen-2-yl-7-trifluoromethyl-pyrazolo[1,5-a]pyrimidin-2-yl)-(2-methyl-piperidin-1-yl)-methanone |
| 4-81 | 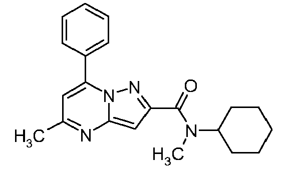 | 348.45 | 5-Methyl-7-phenyl-pyrazolo[1,5-a]pyrimidine-2-carboxylic acid cyclohexyl-methyl-amide |
| 4-82 | 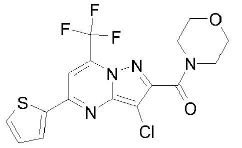 | 416.81 | (3-Chloro-5-thiophen-2-yl-7-trifluoromethyl-pyrazolo[1,5-a]pyrimidin-2-yl)-morpholin-4-yl-methanone |
| 4-83 | 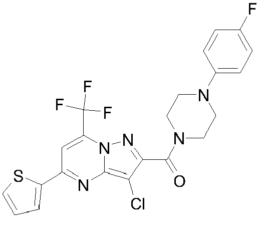 | 509.92 | (3-Chloro-5-thiophen-2-yl-7-trifluoromethyl-pyrazolo[1,5-a]pyrimidin-2-yl)-[4-(4-fluoro-phenyl)-piperazin-1-yl]-methanone |
| 4-84 | 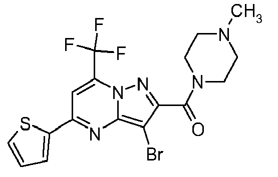 | 474.31 | (3-Bromo-5-thiophen-2-yl-7-trifluoromethyl-pyrazolo[1,5-a]pyrimidin-2-yl)-(4-methyl-piperazin-1-yl)-methanone |
| 4-85 | 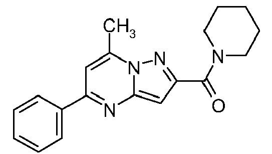 | 320.40 | (7-Methyl-5-phenyl-pyrazolo[1,5-a]pyrimidin-2-yl)-piperidin-1-yl-methanone |
| 4-86 | 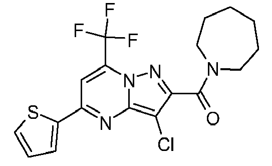 | 428.87 | Azepan-1-yl-(3-chloro-5-thiophen-2-yl-7-trifluoromethyl-pyrazolo[1,5-a]pyrimidin-2-yl)-methanone |
| 4-87 | 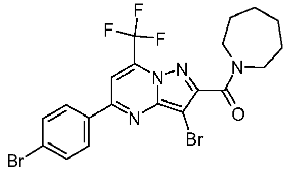 | 546.20 | Azepan-1-yl-[3-bromo-5-(4-bromo-phenyl)-7-trifluoromethyl-pyrazolo[1,5-a]pyrimidin-2-yl]-methanone |
| 4-88 | 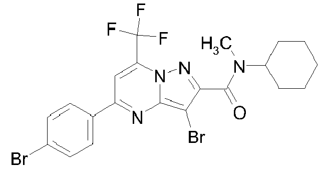 | 560.22 | 3-Bromo-5-(4-bromo-phenyl)-7-trifluoromethyl-pyrazolo[1,5-a]pyrimidine-2-carboxylic acid cyclo-hexyl-methyl-amide |
| 4-89 | 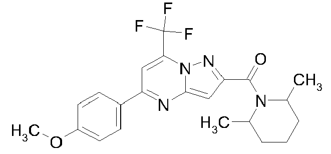 | 432.45 | (2,6-Dimethyl-piperidin-1-yl)-[5-(4-methoxy-phenyl)-7-trifluoromethyl-pyrazolo[1,5-a]pyrimidin-2-yl]-methanone |
| 4-90 | 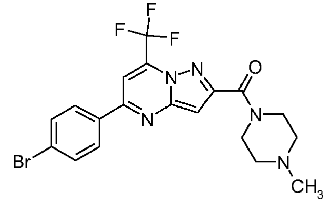 | 468.28 | [5-(4-Bromo-phenyl)-7-trifluoromethyl-pyrazolo[1,5-a]pyrimidin-2-yl]-(4-methyl-piperazin-1-yl)-methanone |
| 4-91 | 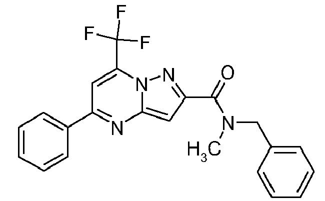 | 410.40 | 5-Phenyl-7-trifluoromethyl-pyrazolo[1,5-a]pyrimidine-2-carboxylic acid benzyl-methyl-amide |
| 4-92 | 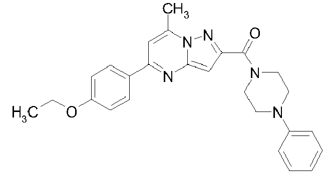 | 441.54 | [5-(4-Ethoxy-phenyl)-7-methyl-pyrazolo[1,5-a]pyrimidin-2-yl]-(4-phenyl-piperazin-1-yl)-methanone |
| 4-93 | 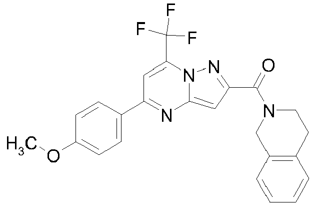 | 452.44 | (3,4-Dihydro-1 H-isoquinolin-2-yl)-[5-(4-methoxy-phenyl)-7-trifluoromethyl-pyrazolo[1,5-a]pyrimidin-2-yl]-methanone |
| 4-94 | 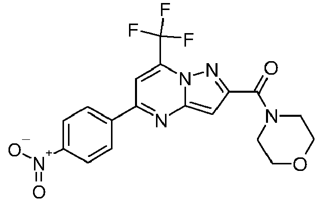 | 421.34 | Morpholin-4-yl-[5-(4-nitro-phenyl)-7-trifluoromethyl-pyrazolo[1,5-a]pyrimidin-2-yl]-methanone |
| 4-95 | 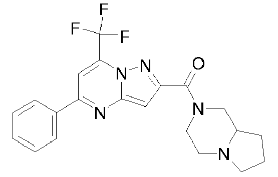 | 415.42 | (Hexahydro-pyrrolo[1,2-a]pyrazin-2-yl)-(5-phenyl-7-trifluoromethyl-pyrazolo[1,5-a]pyrimidin-2-yl)-methanone |
| 4-96 | 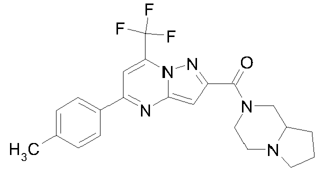 | 429.45 | (Hexahydro-pyrrolo[1,2-a]pyrazin-2-yl)-(5-p-tolyl-7-trifluoromethyl-pyrazolo[1,5-a]pyrimidin-2-yl)-methanone |
| 4-97 | 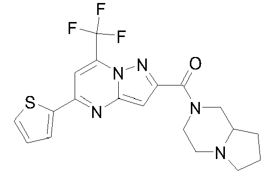 | 421.45 | (Hexahydro-pyrrolo[1,2-a]pyrazin-2-yl)-(5-thiophen-2-yl-7-trifluoromethyl-pyrazolo[1,5-a]pyrimidin-2-yl)-methanone |
| 4-98 | 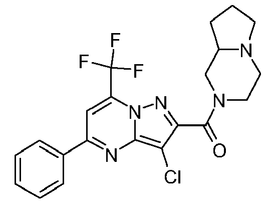 | 449.87 | (3-Chloro-5-phenyl-7-trifluoromethyl-pyrazolo[1,5-a]pyrimidin-2-yl)-(hexahydro-pyrrolo[1,2-a]pyrazin-2-yl)-methanone |
| 4-99 | 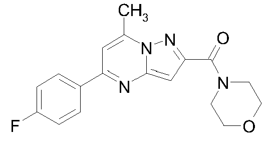 | 340.36 | [5-(4-Fluoro-phenyl)-7-methyl-pyrazolo[1,5-a]pyrimidin-2-yl]-morpholin-4-yl-methanone |
| 4-100 | 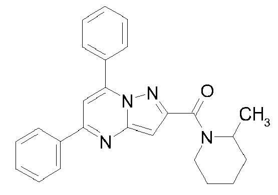 | 396.50 | (5,7-Diphenyl-pyrazolo[1,5-a]pyrimidin-2-yl)-(2-methyl-piperidin-1-yl)-methanone |
| 4-101 | 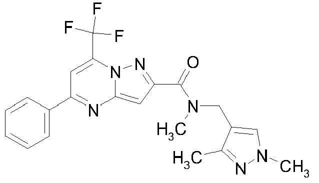 | 428.42 | 5-Phenyl-7-trifluoromethyl-pyrazolo[1,5-a]pyrimidine-2-carboxylic acid (1,3-dimethyl-1H-pyrazol-4-ylmethyl)-methyl-amide |
| 4-102 | 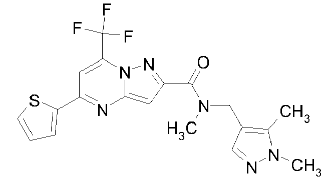 | 434.45 | 5-Thiophen-2-yl-7-trifluoromethyl-pyrazolo[1,5-a]pyrimidine-2-carboxylic acid (1,5-dimethyl-1H-pyrazol-4-ylmethyl)-methyl-amide |
| 4-103 | 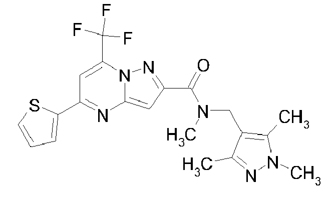 | 448.47 | 5-Thiophen-2-yl-7-trifluoromethyl-pyrazolo[1,5-a]pyrimidine-2-carboxylic acid methyl-(1,3,5-trimethyl-1 H-pyrazol-4-ylmethyl)-amide |
| 4-104 | 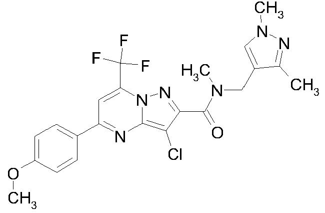 | 492.89 | 3-Chloro-5-(4-methoxy-phenyl)-7-trifluoromethyl-pyrazolo[1,5-a]pyrimidine-2-carboxylic acid (1,3-dimethyl-1H-pyrazol-4-ylmethyl)-methyl-amide |
| 4-105 | 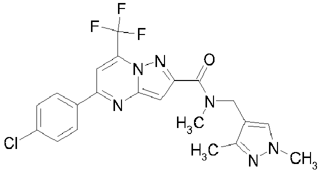 | 462.87 | 5-(4-Chloro-phenyl)-7-trifluoromethyl-pyrazolo[1,5-a]pyrimidine-2-carboxylic acid (1,3-dimethyl-1H-pyrazol-4-ylmethyl)-methyl-amide |
| 4-106 | 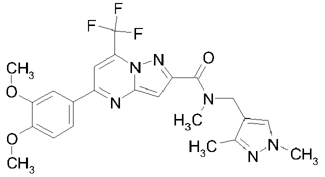 | 488.47 | 5-(3,4-Dimethoxy-phenyl)-7-trifluoromethyl-pyrazolo[1,5-a]pyrimidine-2-carboxylic acid (1,3-dimethyl-1H-pyrazol-4-ylmethyl)-methyl-amide |
| 4-107 | 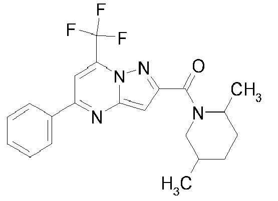 | 402.42 | (2,5-Dimethyl-piperidin-1-yl)-(5-phenyl-7-trifluoromethyl-pyrazolo[1,5-a]pyrimidin-2-yl)-methanone |
| 4-108 | 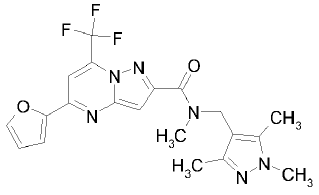 | 432.41 | 5-Furan-2-yl-7-trifluoromethyl-pyrazolo[1,5-a]pyrimidine-2-carboxylic acid methyl-(1,3,5-trimethyl-1H-pyrazol-4-ylmethyl)-amide |
| 4-110 | 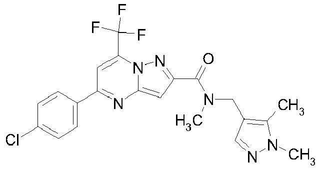 | 462.87 | 5-(4-Chloro-phenyl)-7-trifluoromethyl-pyrazolo[1,5-a]pyrimidine-2-carboxylic acid (1,5-dimethyl-1H-pyrazol-4-ylmethyl)-methyl-amide |
| 4-111 | 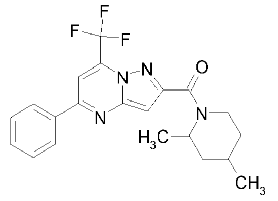 | 402.42 | (2,4-Dimethyl-piperidin-1-yl)-(5-phenyl-7-trifluoromethyl-pyrazolo[1,5-a]pyrimidin-2-yl)-methanone |
| 4-112 | 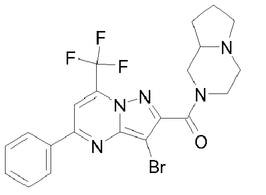 | 494.32 | (3-Bromo-5-phenyl-7-trifluoromethyl-pyrazolo[1,5-a]pyrimidin-2-yl)-(hexahydro-pyrrolo[1,2-a]pyrazin-2-yl)-methanone |
| 4-113 | 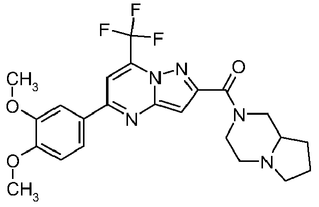 | 475.47 | [5-(3,4-Dimethoxy-phenyl)-7-trifluoromethyl-pyrazolo[1,5-a]pyrimidin-2-yl]-(hexahydro-pyrrolo[1,2-a]pyrazin-2-yl)-methanone |
| 4-114 | 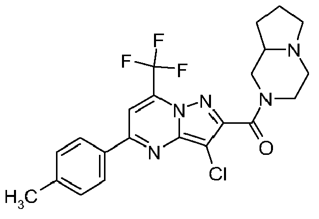 | 463.89 | (3-Chloro-5-p-tolyl-7-trifluoromethyl-pyrazolo[1,5-a]pyrimidin-2-yl)-(hexahydro-pyrrolo[1,2-a]pyrazin-2-yl)-methanone |
| 4-115 | 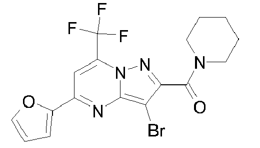 | 443.23 | (3-Bromo-5-furan-2-yl-7-trifluoromethyl-pyrazolo[1,5-a]pyrimidin-2-yl)-piperidin-1-yl-methanone |
| 4-116 | 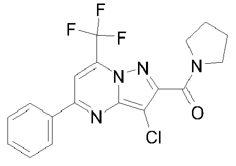 | 394.79 | (3-Chloro-5-phenyl-7-trifluoromethyl-pyrazolo[1,5-a]pyrimidin-2-yl)-pyrrolidin-1-yl-methanone |
| 4-117 | 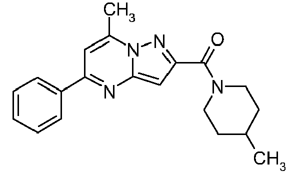 | 334.42 | (7-Methyl-5-phenyl-pyrazolo[1,5-a]pyrimidin-2-yl)-(4-methyl-piperidin-1-yl)-methanone |
| 4-118 | 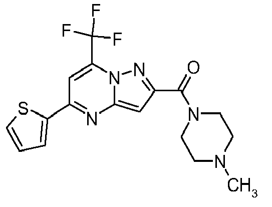 | 395.41 | (4-Methyl-piperazin-1-yl)-(5-thiophen-2-yl-7-trifluoromethyl-pyrazolo[1,5-a]pyrimidin-2-yl)-methanone |
| 4-119 | 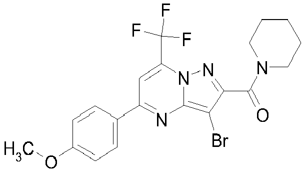 | 483.30 | [3-Bromo-5-(4-methoxy-phenyl)-7-trifluoromethyl-pyrazolo[1,5-a]pyrimidin-2-yl]-piperidin-1-yl-methanone |
| 4-120 | 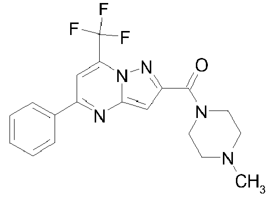 | 389.38 | (4-Methyl-piperazin-1-yl)-(5-phenyl-7-trifluoromethyl-pyrazolo[1,5-a]pyrimidin-2-yl)-methanone |
| 4-121 | 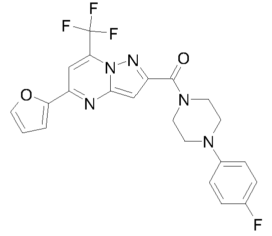 | 459.41 | [4-(4-Fluoro-phenyl)-piperazin-1-yl]-(5-furan-2-yl-7-trifluoromethyl-pyrazolo[1,5-a]pyrimidin-2-yl)-methanone |
| 4-122 | 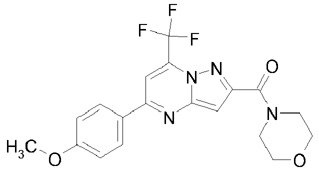 | 406.37 | [5-(4-Methoxy-phenyl)-7-trifluoromethyl-pyrazolo[1,5-a]pyrimidin-2-yl]-morpholin-4-yl-methanone |
| 4-123 | 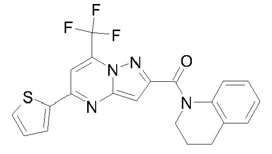 | 428.44 | (3,4-Dihydro-2H-quinolin-1-yl)-(5-thiophen-2-yl-7-trifluoromethyl-pyrazolo[1,5-a]pyrimidin-2-yl)-methanone |
| 4-124 | 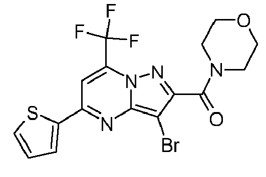 | 461.27 | (3-Bromo-5-thiophen-2-yl-7-trifluoromethyl-pyrazolo[1,5-a]pyrimidin-2-yl)-morpholin-4-yl-methanone |
| 4-125 | 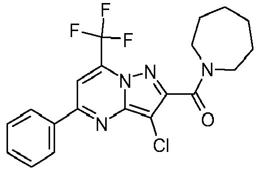 | 422.84 | Azepan-1-yl-(3-chloro-5-phenyl-7-trifluoromethyl-pyrazolo[1,5-a]pyrimidin-2-yl)-methanone |
| 4-126 | 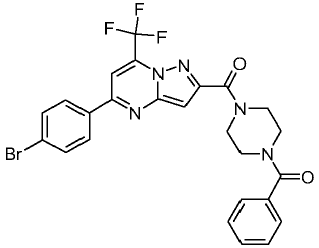 | 558.37 | (4-Benzoyl-piperazin-1-yl)-[5-(4-bromo-phenyl)-7-trifluoromethyl-pyrazolo[1,5-a]pyrimidin-2-yl]-methanone |
| 4-127 | 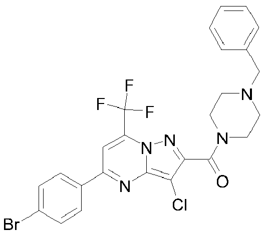 | 578.83 | (4-Benzyl-piperazin-1-yl)-[5-(4-bromo-phenyl)-3-chloro-7-trifluoromethyl-pyrazolo[1,5-a]pyrimidin-2-yl]-methanone |
| 4-128 | 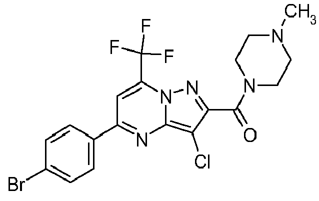 | 502.73 | [5-(4-Bromo-phenyl)-3-chloro-7-trifluoromethyl-pyrazolo[1,5-a]pyrimidin-2-yl]-(4-methyl-piperazin-1-yl)-methanone |
| 4-129 | 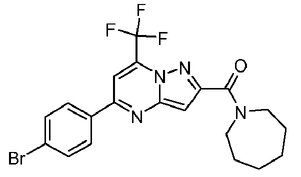 | 467.30 | Azepan-1-yl-[5-(4-bromo-phenyl)-7-trifluoromethyl-pyrazolo[1,5-a]pyrimidin-2-yl]-methanone |
| 4-130 | 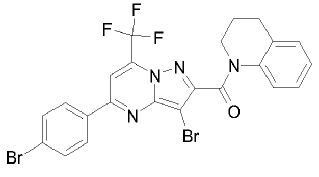 | 580.22 | [3-Bromo-5-(4-bromo-phenyl)-7-trifluoromethyl-pyrazolo[1,5-a]pyrimidin-2-yl]-(3,4-dihydro-2H-quinolin-1-yl)-methanone |
| 4-131 | 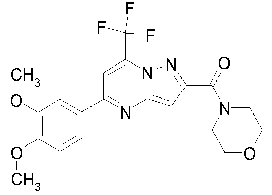 | 436.39 | [5-(3,4-Dimethoxy-phenyl)-7-trifluoromethyl-pyrazolo[1,5-a]pyrimidin-2-yl]-morpholin-4-yl-methanone |
| 4-132 | 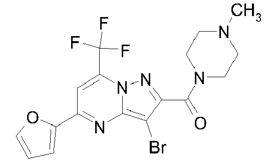 | 458.25 | (3-Bromo-5-furan-2-yl-7-trifluoromethyl-pyrazolo[1,5-a]pyrimidin-2-yl)-(4-methyl-piperazin-1-yl)-methanone |
| 4-133 | 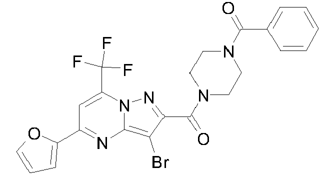 | 548.33 | (4-Benzoyl-pi perazin-1-yl)-(3-bromo-5-furan-2-yl-7-trifluoromethyl-pyrazolo[1,5-a]pyrimidin-2-yl)-methanone |
| 4-134 | 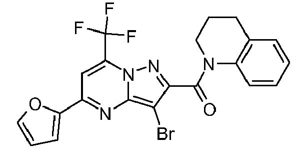 | 491.28 | (3-Bromo-5-furan-2-yl-7-trifluoromethyl-pyrazolo[1,5-a]pyrimidin-2-yl)-(3,4-dihydro-2H-quinolin-1-yl)-methanone |
| 4-135 | 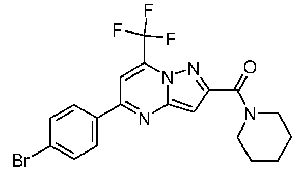 | 453.27 | [5-(4-Bromo-phenyl)-7-trifluoromethyl-pyrazolo[1,5-a]pyrimidin-2-yl]-piperidin-1-yl-methanone |
| 4-136 | 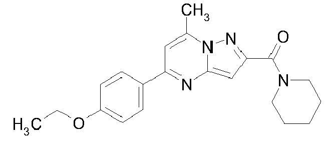 | 364.45 | [5-(4-Ethoxy-phenyl)-7-methyl-pyrazolo[1,5-a]pyrimidin-2-yl]-piperidin-1-yl-methanone |
| 4-137 | 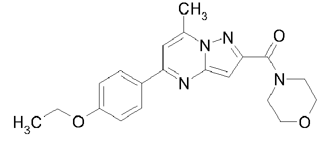 | 366.42 | [5-(4-Ethoxy-phenyl)-7-methyl-pyrazolo[1,5-a]pyrimidin-2-yl]-morpholin-4-yl-methanone |
| 4-138 | 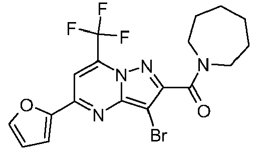 | 457.26 | Azepan-1-yl-(3-bromo-5-furan-2-yl-7-trifluoromethyl-pyrazolo[1,5-a]pyrimidin-2-yl)-methanone |
| 4-139 | 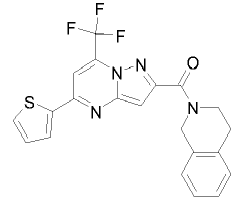 | 428.44 | (3,4-Dihydro-1 H-isoquinolin-2-yl)-(5-thiophen-2-yl-7-trifluoromethyl-pyrazolo[1,5-a]pyrimidin-2-yl)-methanone |
| 4-140 | 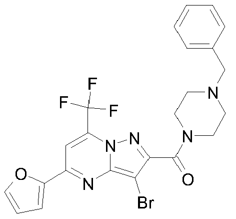 | 534.34 | (4-Benzyl-piperazin-1-yl)-(3-bromo-5-furan-2-yl-7-trifluoromethyl-pyrazolo[1,5-a]pyrimidin-2-yl)-methanone |
| 4-141 | 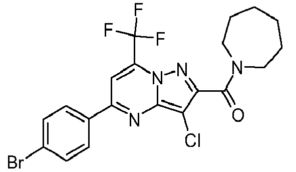 | 501.74 | Azepan-1-yl-[5-(4-bromo-phenyl)-3-chloro-7-trifluoromethyl-pyrazolo[1,5-a]pyrimidin-2-yl]-methanone |
| 4-142 | 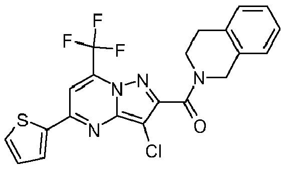 | 462.88 | (3-Chloro-5-thiophen-2-yl-7-trifluoromethyl-pyrazolo[1,5-a]pyrimidin-2-yl)-(3,4-dihydro-1 H-isoquinolin-2-yl)-methanone |
| 4-143 | 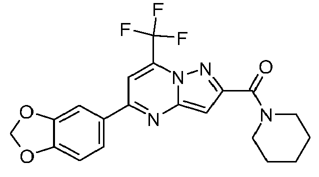 | 418.38 | (5-Benzo[1,3]dioxol-5-yl-7-trifluoromethyl-pyrazolo[1,5-a]pyrimidin-2-yl)-piperidin-1-yl-methanone |
| 4-144 | 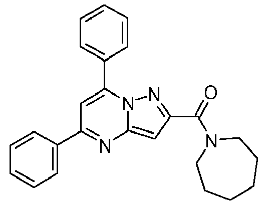 | 396.50 | Azepan-1-yl-(5,7-diphenyl-pyrazolo[1,5-a]pyrimidin-2-yl)-methanone |
| 4-145 | 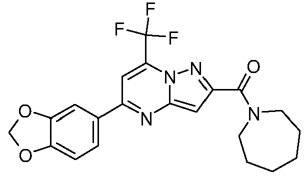 | 432.41 | Azepan-1-yl-(5-benzo[1,3]dioxol-5-yl-7-trifluoromethyl-pyrazolo[1,5-a]pyrimidin-2-yl)-methanone |
| 4-146 | 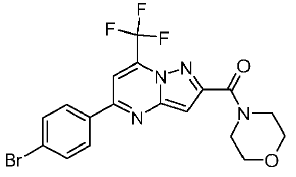 | 455.24 | [5-(4-Bromo-phenyl)-7-trifluoromethyl-pyrazolo[1,5-a]pyrimidin-2-yl]-morpholin-4-yl-methanone |
| 4-147 | 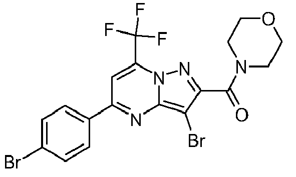 | 534.14 | [3-Bromo-5-(4-bromo-phenyl)-7-trifluoromethyl-pyrazolo[1,5-a] pyri mid i n-2-yl]-morphol i n-4-yl-methanone |
| 4-148 | 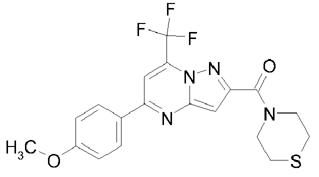 | 422.43 | [5-(4-Methoxy-phenyl)-7-trifluoromethyl-pyrazolo[1,5-a]pyrimidin-2-yl]-thiomorpholin-4-yl-methanone |
| 4-149 | 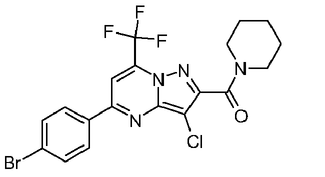 | 487.71 | [5-(4-Bromo-phenyl)-3-chloro-7-trifluoromethyl-pyrazolo[1,5-a]pyrimidin-2-yl]-piperidin-1-yl-methanone |
| 4-150 | 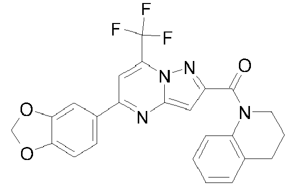 | 466.42 | (5-Benzo[1,3]dioxol-5-yl-7-trifluoromethyl-pyrazolo[1,5-a]pyrimidin-2-yl)-(3,4-dihydro-2H-quinolin-1-yl)-methanone |
| 4-151 | 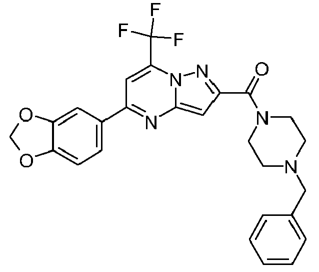 | 509.49 | (5-Benzo[1,3]dioxol-5-yl-7-trifluoromethyl-pyrazolo[1,5-a]pyrimidin-2-yl)-(4-benzyl-piperazin-1-yl)-methanone |
| 4-152 | 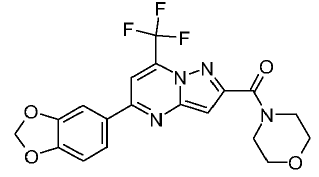 | 420.35 | (5-Benzo[1,3]dioxol-5-yl-7-trifluoromethyl-pyrazolo[1,5-a]pyrimidin-2-yl)-morpholin-4-yl-methanone |
| 4-153 | 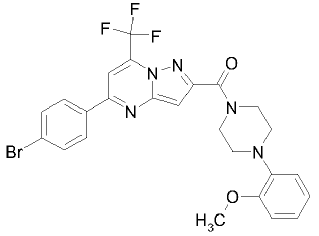 | 560.38 | [5-(4-Bromo-phenyl)-7-trifluoromethyl-pyrazolo[1,5-a]pyrimidin-2-yl]-[4-(2-methoxy-phenyl)-piperazin-1-yl]-methanone |
| 4-154 | 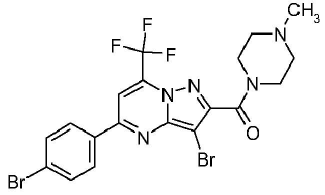 | 547.19 | [3-Bromo-5-(4-bromo-phenyl)-7-trifluoromethyl-pyrazolo[1,5-a]pyrimidin-2-yl]-(4-methyl-piperazin-1-yl)-methanone |
| 4-155 | 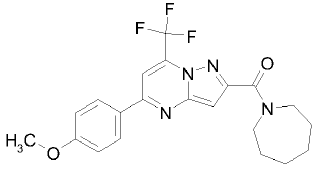 | 418.42 | Azepan-1-yl-[5-(4-methoxy-phenyl)-7-trifluoromethyl-pyrazolo[1,5-a]pyrimidin-2-yl]-methanone |
| 4-156 | 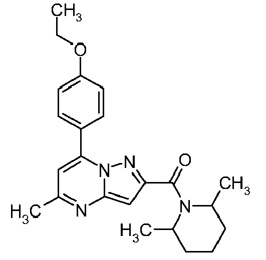 | 392.51 | (2,6-Dimethyl-piperidin-1-yl)-[7-(4-ethoxy-phenyl)-5-methyl-pyrazolo[1,5-a]pyrimidin-2-yl]-methanone |
| 4-157 | 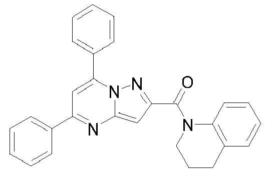 | 430.51 | (3,4-Dihydro-2H-quinolin-1-yl)-(5,7-diphenyl-pyrazolo[1,5-a]pyrimidin-2-yl)-methanone |
| 4-158 | 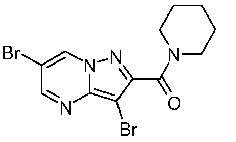 | 388.07 | (3,6-Dibromo-pyrazolo[1,5-a]pyrimidin-2-yl)-piperidin-1-yl-methanone |
| 4-159 | 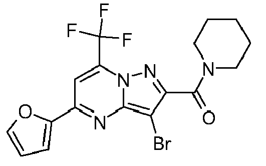 | 443.23 | (3-Bromo-5-furan-2-yl-7-trifluoromethyl-pyrazolo[1,5-a]pyrimidin-2-yl)-piperidin-1-yl-methanone |
| 4-160 | 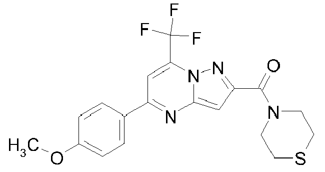 | 422.43 | [5-(4-Methoxy-phenyl)-7-trifluoromethyl-pyrazolo[1,5-a]pyrimidin-2-yl]-thiomorpholin-4-yl-methanone |
| 4-161 | 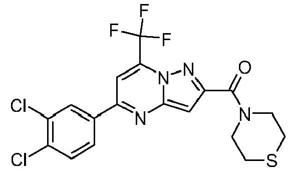 | 461.30 | [5-(3,4-Dichloro-phenyl)-7-trifluoromethyl-pyrazolo[1,5-a]pyrimidin-2-yl]-thiomorpholin-4-yl-methanone |
| 4-162 | 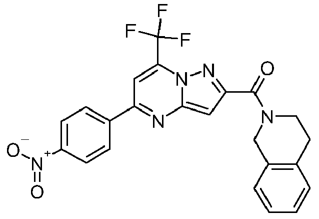 | 467.41 | (3,4-Dihydro-1H-isoquinolin-2-yl)-[5-(4-nitro-phenyl)-7-trifluoromethyl-pyrazolo[1,5-a]pyrimidin-2-yl]-methanone |
| 4-163 | 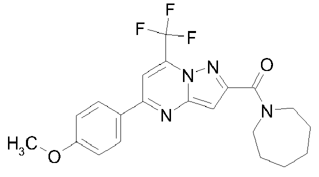 | 418.42 | Azepan-1-yl-[5-(4-methoxy-phenyl)-7-trifluoromethyl-pyrazolo[1,5-a]pyrimidin-2-yl]-methanone |
| 4-164 | 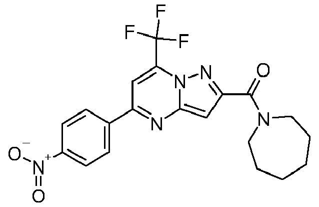 | 433.39 | Azepan-1-yl-[5-(4-nitro-phenyl)-7-trifluoromethyl-pyrazolo[1,5-a]pyrimidin-2-yl]-methanone |
| 4-165 | 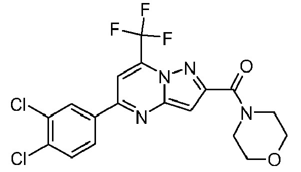 | 445.23 | [5-(3,4-Dichloro-phenyl)-7-trifluoromethyl-pyrazolo[1,5-a] pyrimidin-2-yl]-morpholin-4-yl-methanone |
| 4-166 | 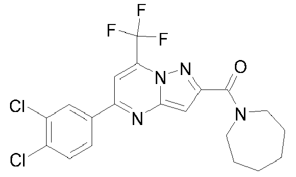 | 457.29 | Azepan-1-yl-[5-(3,4-dichloro-phenyl)-7-trifluoromethyl-pyrazolo[1,5-a]pyrimidin-2-yl]-methanone |
| 4-167 | 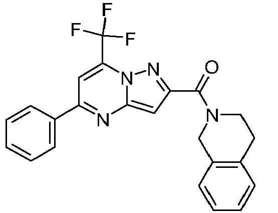 | 422.41 | (3,4-Dihydro-1 H-isoquinolin-2-yl)-(5-phenyl-7-trifluoromethyl-pyrazolo[1,5-a]pyrimidin-2-yl)-methanone |
| 4-168 | 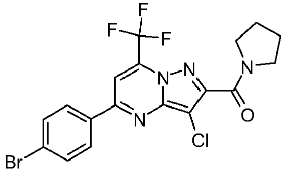 | 473.69 | [5-(4-Bromo-phenyl)-3-chloro-7-trifluoromethyl-pyrazolo[1,5-a]pyrimidin-2-yl]-pyrrolidin-1-yl-methanone |
| 4-169 | 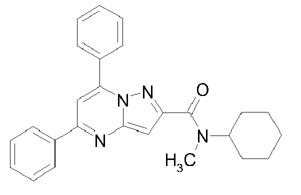 | 410.52 | 5,7-Diphenyl-pyrazolo[1,5-a]pyrimidine-2-carboxylic acid cyclohexyl-methyl-amide |
| 4-170 | 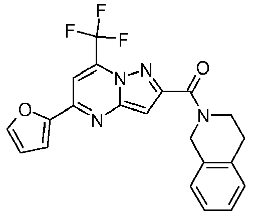 | 412.37 | (3,4-Dihydro-1H-isoquinolin-2-yl)-(5-furan-2-yl-7-trifluoromethyl-pyrazolo[1,5-a]pyrimidin-2-yl)-methanone |
| 4-171 | 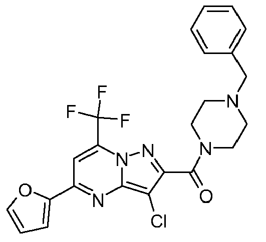 | 489.89 | (4-Benzyl-piperazin-1-yl)-(3-chloro-5-furan-2-yl-7-trifluoromethyl-pyrazolo[1,5-a]pyrimidin-2-yl)-methanone |
| 4-172 | 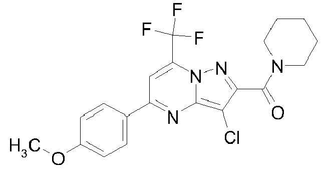 | 438.84 | [3-Chloro-5-(4-methoxy-phenyl)-7-trifluoromethyl-pyrazolo[1,5-a]pyrimidin-2-yl]-piperidin-1-yl-methanone |
| 4-173 | 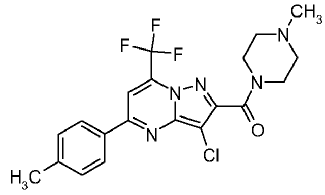 | 437.86 | (3-Chloro-5-p-tolyl-7-trifluoromethyl-pyrazolo[1,5-a]pyrimidin-2-yl)-(4-methyl-piperazin-1-yl)-methanone |
| 4-174 | 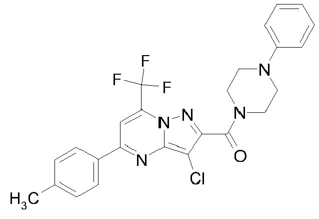 | 499.93 | (3-Chloro-5-p-tolyl-7-trifluoromethyl-pyrazolo[1,5-a]pyrimidin-2-yl)-(4-phenyl-piperazin-1-yl)-methanone |
| 4-175 | 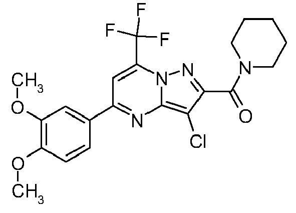 | 468.87 | [3-Chloro-5-(3,4-dimethoxy-phenyl)-7-trifluoromethyl-pyrazolo[1,5-a]pyrimidin-2-yl]-piperidin-1-yl-methanone |
| 4-176 | 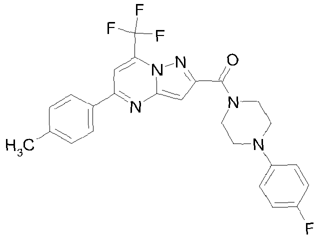 | 483.47 | [4-(4-Fluoro-phenyl)-piperazin-1-yl]-(5-p-tolyl-7-trifluoromethyl-pyrazolo[1,5-a]pyrimidin-2-yl)-methanone |
| 4-177 | 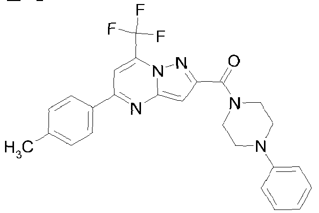 | 465.48 | (4-Phenyl-piperazin-1-yl)-(5-p-tolyl-7-trifluoromethyl-pyrazolo[1,5-a]pyrimidin-2-yl)-methanone |
| 4-178 | 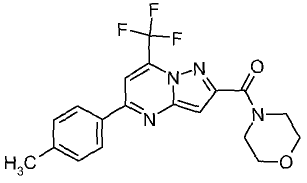 | 390.37 | Morpholin-4-yl-(5-p-tolyl-7-trifluoromethyl-pyrazolo[1,5-a]pyrimidin-2-yl)-methanone |
| 4-179 | 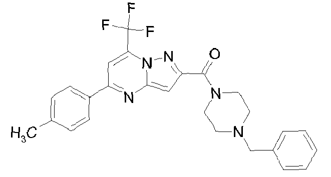 | 479.51 | (4-Benzyl-piperazin-1-yl)-(5-p-tolyl-7-trifluoromethyl-pyrazolo[1,5-a]pyrimidin-2-yl)-methanone |
| 4-180 | 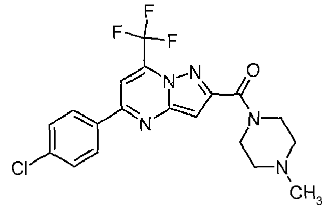 | 423.83 | [5-(4-Chloro-phenyl)-7-trifluoromethyl-pyrazolo[1,5-a]pyrimidin-2-yl]-(4-methyl-piperazin-1-yl)-methanone |
| 4-181 | 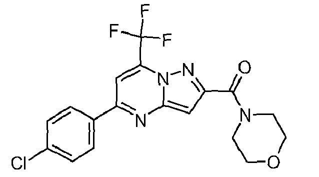 | 410.79 | [5-(4-Chloro-phenyl)-7-trifluoromethyl-pyrazolo[1,5-a]pyrimidin-2-yl]-morpholin-4-yl-methanone |
| 4-182 | 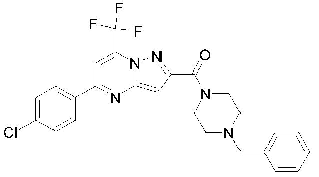 | 499.93 | (4-Benzyl-piperazin-1-yl)-[5-(4-chloro-phenyl)-7-trifluoromethyl-pyrazolo[1,5-a]pyrimidin-2-yl]-methanone |
| 4-183 | 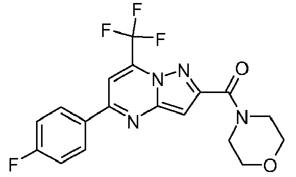 | 394.33 | [5-(4-Fluoro-phenyl)-7-trifluoromethyl-pyrazolo[1,5-a]pyrimidin-2-yl]-morpholin-4-yl-methanone |
| 4-184 | 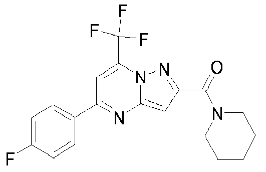 | 392.36 | [5-(4-Fluoro-phenyl)-7-trifluoromethyl-pyrazolo[1,5-a]pyrimidin-2-yl]-piperidin-1-yl-methanone |
| 4-185 | 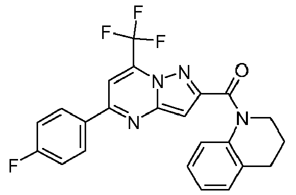 | 440.40 | (3,4-Dihydro-2H-quinolin-1-yl)-[5-(4-fluoro-phenyl)-7-trifluoromethyl-pyrazolo[1,5-a]pyrimidin-2-yl]-methanone |
| 4-186 | 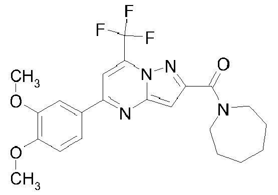 | 448.45 | Azepan-1-yl-[5-(3,4-dimethoxy-phenyl)-7-trifluoromethyl-pyrazolo[1,5-a]pyrimidin-2-yl]-methanone |
| 4-187 | 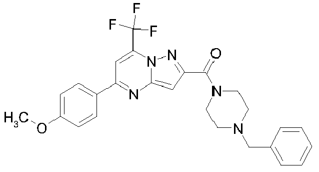 | 495.51 | (4-Benzyl-piperazin-1-yl)-[5-(4-methoxy-phenyl)-7-trifluoromethyl-pyrazolo[1,5-a]pyrimidin-2-yl]-methanone |
| 4-188 | 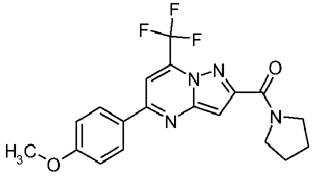 | 390.37 | [5-(4-Methoxy-phenyl)-7-trifluoromethyl-pyrazolo[1,5-a]pyrimidin-2-yl]-pyrrolidin-1-yl-methanone |
| 4-189 | 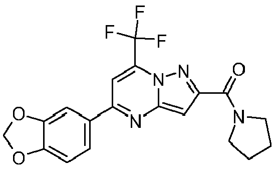 | 404.35 | (5-Benzo[1,3]dioxol-5-yl-7-trifluoromethyl-pyrazolo[1,5-a]pyrimidin-2-yl)-pyrrolidin-1-yl-methanone |
| 4-190 | 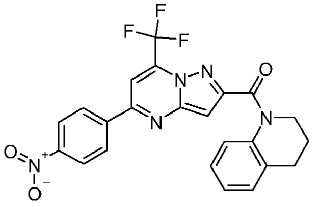 | 467.41 | (3,4-Di hydro-2H-quinol in-1-yl)-[5-(4-nitro-phenyl)-7-trifluoromethyl-pyrazolo[1,5-a]pyrimidin-2-yl]-methanone |
| 4-191 | 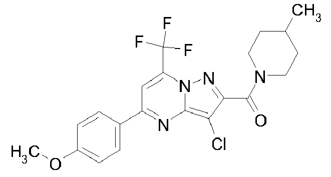 | 452.87 | [3-Chloro-5-(4-methoxy-phenyl)-7-trifluoromethyl-pyrazolo[1,5-a]pyrimidin-2-yl]-(4-methyl-piperidin-1-yl)-methanone |
| 4-192 | 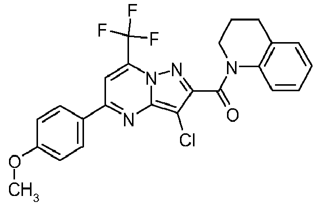 | 486.88 | [3-Chloro-5-(4-methoxy-phenyl)-7-trifluoromethyl-pyrazolo[1,5-a] pyrimidin-2-yl]-(3,4-dihydro-2H-quinolin-1-yl)-methanone |
| 4-193 | 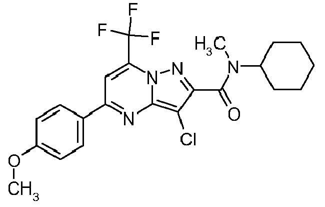 | 466.89 | 3-Chloro-5-(4-methoxy-phenyl)-7-trifluoromethyl-pyrazolo[1,5-a]pyrimidine-2-carboxylic acid cyclohexyl-methyl-amide |
| 4-194 | 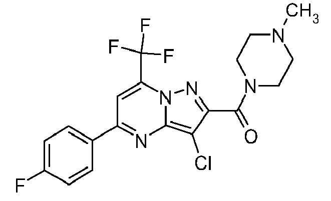 | 441.82 | [3-Chloro-5-(4-fluoro-phenyl)-7-trifluoromethyl-pyrazolo[1,5-a]pyrimidin-2-yl]-(4-methyl-piperazin-1-yl)-methanone |
| 4-195 | 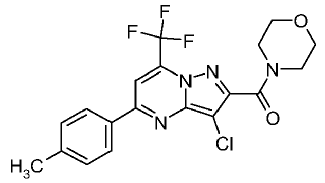 | 424.81 | (3-Chloro-5-p-tolyl-7-trifluoromethyl-pyrazolo[1,5-a]pyrimidin-2-yl)-morpholin-4-yl-methanone |
| 4-196 | 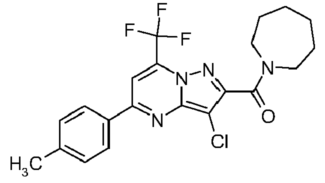 | 436.87 | Azepan-1-yl-(3-chloro-5-p-tolyl-7-trifluoromethyl-pyrazolo[1,5-a]pyrimidin-2-yl)-methanone |
| 4-197 | 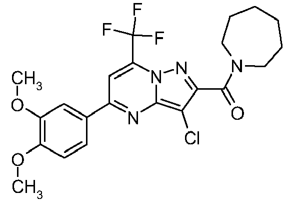 | 482.89 | Azepan-1-yl-[3-chloro-5-(3,4-dimethoxy-phenyl)-7-trifluoromethyl-pyrazolo[1,5-a]pyrimidin-2-yl]-methanone |
| 4-198 | 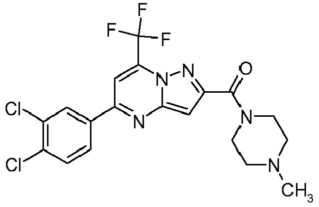 | 458.27 | [5-(3,4-Dichloro-phenyl)-7-trifluoromethyl-pyrazolo[1,5-a]pyrimidin-2-yl]-(4-methyl-piperazin-1-yl)-methanone |
| 4-199 | 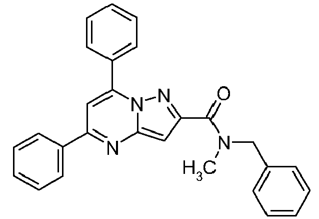 | 418.50 | 5,7-Diphenyl-pyrazolo[1,5-a]pyrimidine-2-carboxylic acid benzyl-methyl-amide |
| 4-200 | 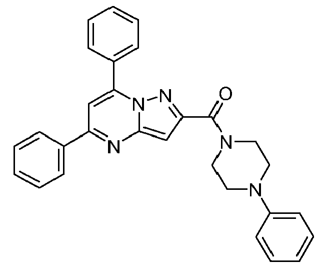 | 459.56 | (5,7-Diphenyl-pyrazolo[1,5-a]pyrimidin-2-yl)-(4-phenyl-piperazin-1-yl)-methanone |
| 4-201 | 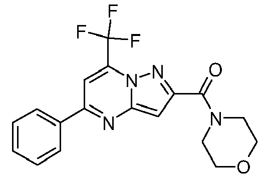 | 376.34 | Morpholin-4-yl-(5-phenyl-7-trifluoromethyl-pyrazolo[1,5-a]pyrimidin-2-yl)-methanone |
| 4-202 | 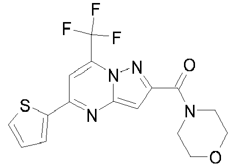 | 382.37 | Morpholin-4-yl-(5-thiophen-2-yl-7-trifluoromethyl-pyrazolo[1,5-a]pyrimidin-2-yl)-methanone |
| 4-203 | 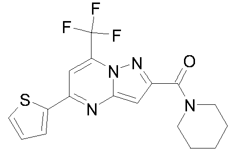 | 380.39 | Piperidin-1-yl-(5-thiophen-2-yl-7-trifluoromethyl-pyrazolo[1,5-a]pyrimidin-2-yl)-methanone |
| 4-204 | 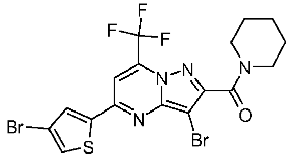 | 538.20 | [3-Bromo-5-(4-bromo-thiophen-2-yl)-7-trifluoromethyl-pyrazolo[1,5-a]pyrimidin-2-yl]-piperidin-1-yl-methanone |
| 4-205 | 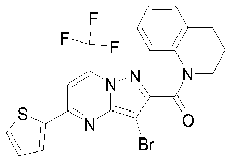 | 507.34 | (3-Bromo-5-thiophen-2-yl-7-trifluoromethyl-pyrazolo[1,5-a]pyrimidin-2-yl)-(3,4-dihydro-2H-quinolin-1-yl)-methanone |
| 4-206 | 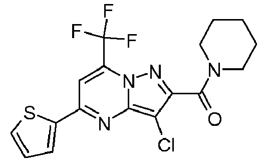 | 414.84 | (3-Chloro-5-thiophen-2-yl-7-trifluoromethyl-pyrazolo[1,5-a]pyrimidin-2-yl)-piperidin-1-yl-methanone |
| 4-207 | 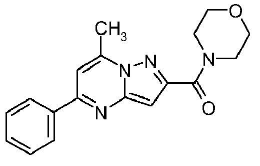 | 322.37 | (7-Methyl-5-phenyl-pyrazolo[1,5-a]pyrimidin-2-yl)-morpholin-4-yl-methanone |
| 4-208 | 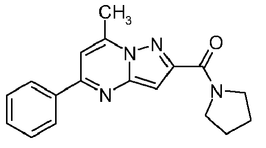 | 306.37 | (7-Methyl-5-phenyl-pyrazolo[1,5-a]pyrimidin-2-yl)-pyrrolidin-1-yl-methanone |
| 4-209 | 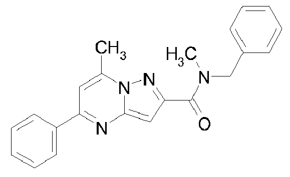 | 356.43 | 7-Methyl-5-phenyl-pyrazolo[1,5-a]pyrimidine-2-carboxylic acid benzyl-methyl-amide |
| 4-210 | 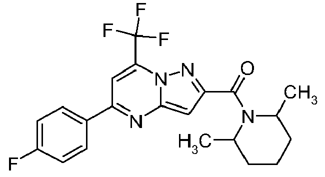 | 420.41 | (2,6-Dimethyl-piperidin-1-yl)-[5-(4-fluoro-phenyl)-7-trifluoromethyl-pyrazolo[1,5-a]pyrimidin-2-yl]-methanone |
| 4-211 | 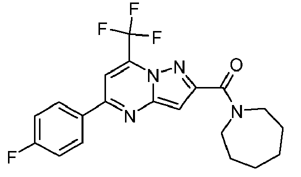 | 406.39 | Azepan-1-yl-[5-(4-fluoro-phenyl)-7-trifluoromethyl-pyrazolo[1,5-a]pyrimidin-2-yl]-methanone |
| 4-212 | 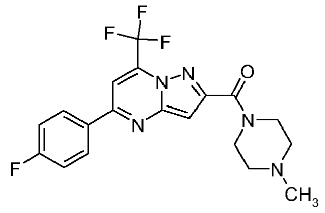 | 407.37 | [5-(4-Fluoro-phenyl)-7-trifluoromethyl-pyrazolo[1,5-a]pyrimidin-2-yl]-(4-methyl-piperazin-1-yl)-methanone |
| 4-213 | 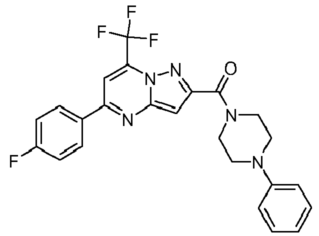 | 469.45 | [5-(4-Fluoro-phenyl)-7-trifluoromethyl-pyrazolo[1,5-a]pyrimidin-2-yl]-(4-phenyl-piperazin-1-yl)-methanone |
| 4-214 | 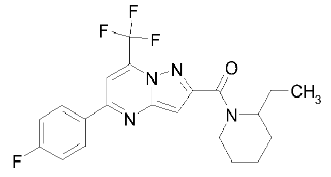 | 420.41 | (2-Ethyl-piperidin-1-yl)-[5-(4-fluoro-phenyl)-7-trifluoromethyl-pyrazolo[1,5-a]pyrimidin-2-yl]-methanone |

### Example 5

### (5-Phenyl-7-trifluoromethyl-pyrazolo[1,5-a]pyrimidin-3-yl)-(1,3,3-trimethyl-6-azabicyclo[3.2.1]oct-6-yl)-methanone

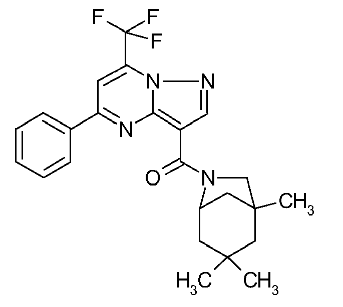

A mixture of 5-phenyl-7-trifluoromethyl-pyrazolo[1,5-a]pyrimidine-3-carboxylic acid (108 mg, 0.35 mmol), HOBT (52 mg, 0.39 mmol), EDAC (74 mg, 0.39 mmol) and DIPEA (50 mg, 0.39 mmol) in THF (5 ml) was stirred at room temperature for 30 min. and then added 1,3,3-trimethyl-6-aza-bicyclo[3.2.1]octane (59 mg, 0.39 mmol) and stirred for 16 hrs. The reaction mixture was quenched by addition of water and extracted with EtOAc (2 x 50 ml). The combined organic phases were dried (MgS0₄), filtered and the volatiles evaporated *in* vacuo affording 160 mg (~100 %) of the title compound as a solid.
LC/MS m/z: 443 H⁺
¹H NMR (400 MHz, CDCl₃) δ 0.89 - 1.19 (m, 9H), 1.22 - 1.95 (m, 5.5H), 2.26 (m, 0.5H), 3.36 (d, 0.5H), 3.54 (d, 0.5H), 3.73 (q, 1 H), 4.60 (m, 0.5 H), 4.76 (m, 0.5H), 7.56 (m, 3H), 7.71 (s, 1 H), 8.14 (t, 2H), 8.50 (d, 1H).
Calculated for C₂₄H₂₅F₃N₄O;
C 65.15 %; H 5.69 %; N 12.66 %; Found
C 65.27 %; H 5.97 %; N 12.32.
The following compounds were made in a similar way as described in example 5 above

| **No** | **Structure** | **MW** | **IUPAC name** |
|---|---|---|---|
| 5-2 | 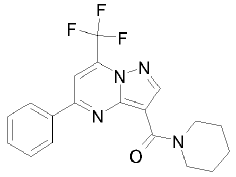 | 374.37 | (5-Phenyl-7-trifluoromethyl-pyrazolo[1,5-a]pyrimidin-3-yl)-piperidin-1-yl-methanone |
| 5-3 | 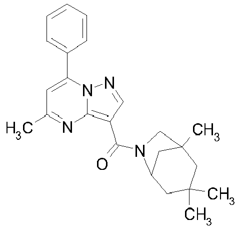 | 388.52 | (5-Methyl-7-phenyl-pyrazolo[1,5-a]pyrimidin-3-yl)-(1,3,3-trimethyl-6-aza-bicyclo[3.2.1]oct-6-yl)-methanone |
| 5-4 | 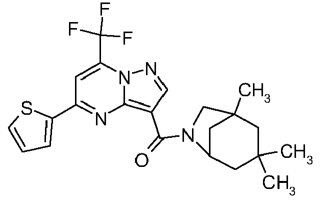 | 448.51 | (5-Thiophen-2-yl-7-trifluoromethyl-pyrazolo[1,5-a] pyrimidin-3-yl)-(1, 3, 3-tri methyl-6-aza-bicyclo[3.2.1]oct-6-yl)-methanone |
| 5-5 | 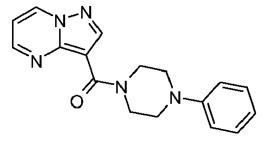 | 307.36 | (4-Phenyl-piperazin-1-yl)-pyrazolo[1,5-a]pyrimidin-3-yl-methanone |
| 5-6 | 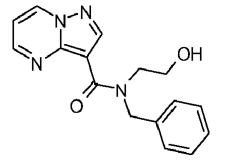 | 296.33 | Pyrazolo[1,5-a]pyrimidine-3-carboxylic acid ben-zyl-(2-hydroxy-ethyl)-amide |
| 5-7 | 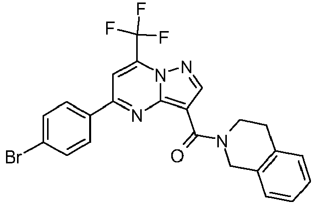 | 501.31 | [5-(4-Bromo-phenyl)-7-trifluoromethyl-pyrazolo[1,5-a]pyrimidin-3-yl]-(3,4-dihydro-1H-isoquinolin-2-yl)-methanone |
| 5-8 | 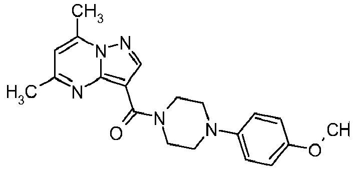 | 365.44 | (5,7-Dimethyl-pyrazolo[1,5-a]pyrimidin-3-yl)-[4-(4-methoxy-phenyl)-piperazin-1-yl]-methanone |
| 5-9 | 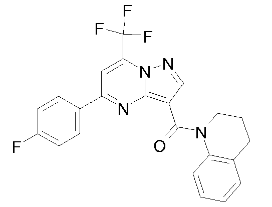 | 440.40 | (3,4-Dihydro-2H-quuinolin-1-yl)-[5-(4-fluoro-phenyl)-7-trifluoromethyl-pyrazolo[1,5-a]pyrimidin-3-yl]-methanone |
| 5-10 | 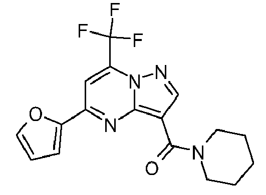 | 364.33 | (5-Furan-2-yl-7-trifluoromethyl-pyrazolo[1,5-a]pyrimidin-3-yl)-piperidin-1-yl-methanone |
| 5-11 | 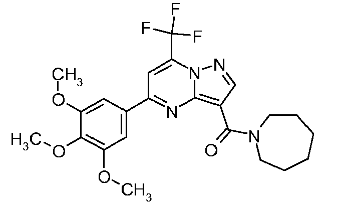 | 478.48 | Azepan-1-yl-[7-trifluoromethyl-5-(3,4,5-trimethoxy-phenyl)-pyrazolo[1,5-a]pyrimidin-3-yl]-methanone |
| 5-12 | 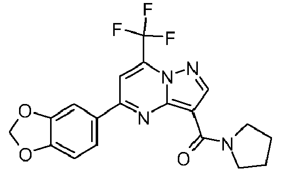 | 404.35 | (5-Benzo[1,3]dioxol-5-yl-7-trifluoromethyl-pyrazolo[1,5-a]pyrimidin-3-yl)-pyrrolidin-1-yl-methanone |
| 5-13 | 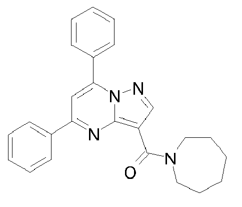 | 396.50 | Azepan-1-yl-(5,7-diphenyl-pyrazolo[1,5-a]pyrimidin-3-yl)-methanone |
| 5-14 | 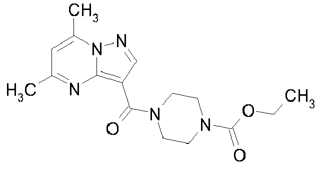 | 331.38 | 4-(5,7-Dimethyl-pyrazolo[1,5-a]pyrimidine-3-carbonyl)-piperazine-1-carboxylic acid ethyl ester |
| 5-15 | 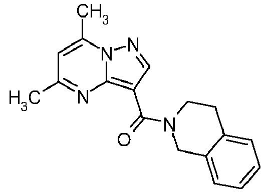 | 306.37 | (3,4-Dihydro-1H-isoquinolin-2-yl)-(5,7-dimethyl-pyrazolo[1,5-a]pyrimidin-3-yl)-methanone |
| 5-16 | 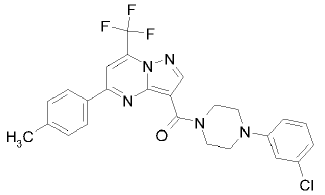 | 499.93 | [4-(3-Chloro-phenyl)-piperazin-1-yl]-(5-p-tolyl-7-trifluoromethyl-pyrazolo[1,5-a]pyrimidin-3-yl)-methanone |
| 5-17 | 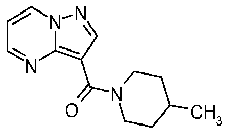 | 244.30 | (4-Methyl-piperidin-1-yl)-pyrazolo[1,5-a]pyrimidin-3-yl-methanone |
| 5-18 | 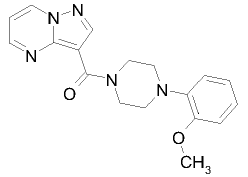 | 337.38 | [4-(2-Methoxy-phenyl)-piperazin-1-yl]-pyrazolo[1,5-a]pyrimidin-3-yl-methanone |
| 5-19 | 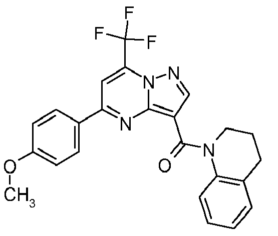 | 452.44 | (3,4-Dihydro-2H-quinolin-1-yl)-[5-(4-methoxy-phenyl)-7-trifluoromethyl-pyrazolo[1,5-a]pyrimidin-3-yl]-methanone |
| 5-20 | 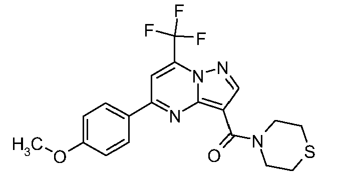 | 422.43 | [5-(4-Methoxy-phenyl)-7-trifluoromethyl-pyrazolo[1,5-a]pyrimidin-3-yl]-thiomorpholin-4-yl-methanone |
| 5-21 | 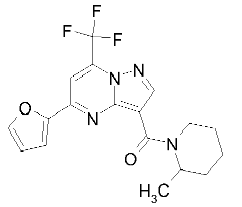 | 378.36 | (5-Furan-2-yl-7-trifluoromethyl-pyrazolo[1,5-a]pyrimidin-3-yl)-(2-methyl-piperidin-1-yl)-methanone |
| 5-22 | 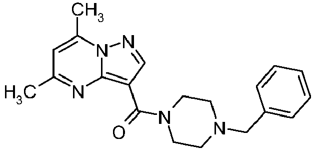 | 349.44 | (4-Benzyl-piperazin-1-yl)-(5,7-dimethyl-pyrazolo[1,5-a]pyrimidin-3-yl)-methanone |
| 5-23 | 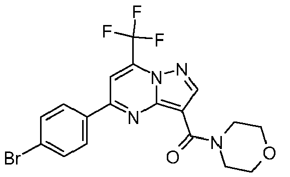 | 455.24 | [5-(4-Bromo-phenyl)-7-trifluoromethyl-pyrazolo[1,5-a] pyrimidin-3-yl]-morpholin-4-yl-methanone |
| 5-24 | 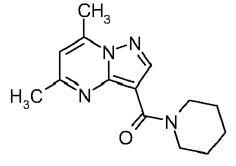 | 258.33 | (5,7-Dimethyl-pyrazolo[1,5-a]pyrimidin-3-yl)-piperidin-1-yl-methanone |
| 5-25 | 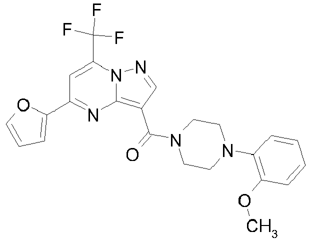 | 471.44 | (5-Furan-2-yl-7-trifluoromethyl-pyrazolo[1,5-a]pyrimidin-3-yl)-[4-(2-methoxy-phenyl)-piperazin-1-yl]-methanone |
| 5-26 | 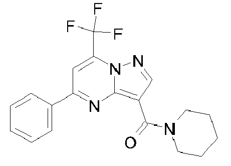 | 374.37 | (5-Phenyl-7-trifluoromethyl-pyrazolo[1,5-a]pyrimidin-3-yl)-piperidin-1-yl-methanone |
| 5-27 | 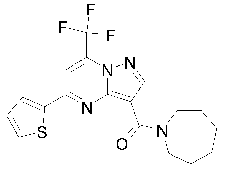 | 394.42 | Azepan-1-yl-(5-thiophen-2-yl-7-trifluoromethyl-pyrazolo[1,5-a]pyrimidin-3-yl)-methanone |
| 5-28 | 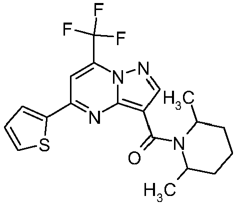 | 408.45 | (2,6-Dimethyl-piperidin-1-yl)-(5-thiophen-2-yl-7-trifluoromethyl-pyrazolo[1,5-a]pyrimidin-3-yl)-methanone |
| 5-29 | 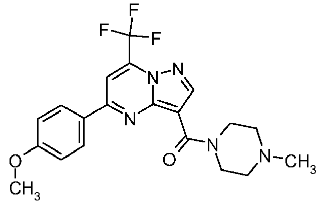 | 419.41 | [5-(4-Methoxy-phenyl)-7-trifluoromethyl-pyrazolo[1,5-a]pyrimidin-3-yl]-(4-methyl-piperazin-1-yl)-methanone |
| 5-30 | 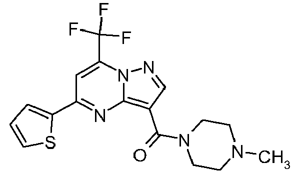 | 395.41 | (4-Methyl-piperazin-1-yl)-(5-thiophen-2-yl-7-trifluoromethyl-pyrazolo[1,5-a]pyrimidin-3-yl)-methanone |
| 5-31 | 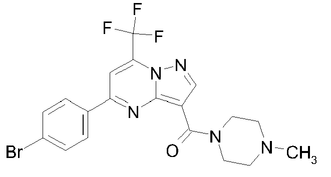 | 468.28 | [5-(4-Bromo-phenyl)-7-trifluoromethyl-pyrazolo[1,5-a]pyrimidin-3-yl]-(4-methyl-piperazin-1-yl)-methanone |
| 5-32 | 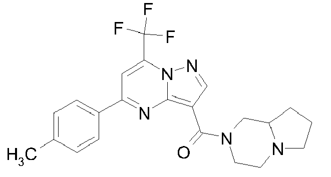 | 429.45 | (Hexahydro-pyrrolo[1,2-a]pyrazin-2-yl)-(5-p-tolyl-7-trifluoromethyl-pyrazolo[1,5-a]pyrimidin-3-yl)-methanone |
| 5-33 | 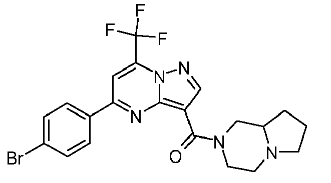 | 494.32 | [5-(4-Bromo-phenyl)-7-trifluoromethyl-pyrazolo[1,5-a]pyrimidin-3-yl]-(hexahydro-pyrrolo[1,2-a]pyrazin-2-yl)-methanone |
| 5-34 | 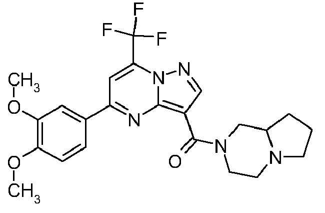 | 475.47 | [5-(3,4-Dimethoxy-phenyl)-7-trifluoromethyl-pyrazolo[1,5-a]pyrimidin-3-yl]-(hexahydro-pyrrolo[1,2-a]pyrazin-2-yl)-methanone |
| 5-35 | 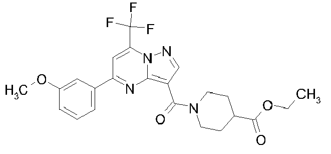 | 476.46 | 1-[5-(3-Methoxy-phenyl)-7-trifluoromethyl-pyrazolo[1,5-a]pyrimidine-3-carbonyl]-piperidine-4-carboxylic acid ethyl ester |
| 5-36 | 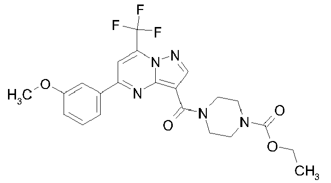 | 477.45 | 4-[5-(3-Methoxy-phenyl)-7-trifluoromethyl-pyrazolo[1,5-a]pyrimidine-3-carbonyl]-piperazine-1-carboxylic acid ethyl ester |
| 5-37 | 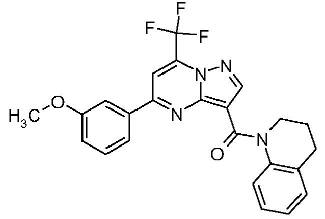 | 452.44 | (3,4-Dihydro-2H-quinolin-1-yl)-[5-(3-methoxy-phenyl)-7-trifluoromethyl-pyrazolo[1,5-a]pyrimidin-3-yl]-methanone |
| 5-38 | 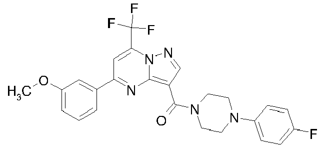 | 499.47 | [4-(4-Fluoro-phenyl)-piperazin-1-yl]-[5-(3-methoxy-phenyl)-7-trifluoromethyl-pyrazolo[1,5-a]pyrimidin-3-yl]-methanone |
| 5-39 | 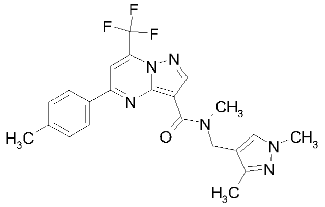 | 442.45 | 5-p-Tolyl-7-trifluoromethyl-pyrazolo[1,5-al]pyrimidine-3-carboxylic acid (1,3-dimethyl-1-H-pyrazol-4-ylmethyl)-methyl-amide |
| 5-40 | 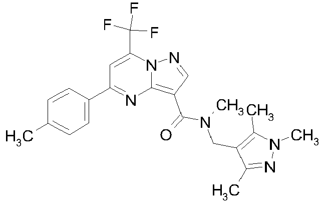 | 456.47 | 5-p-Tolyl-7-trifluoromethyl-pyrazolo[1,5-a]pyrimidine-3-carboxylic acid methyl-(1,3,5-trimethyl-1 H-pyrazol-4-ylmethyl)-amide |
| 5-41 | 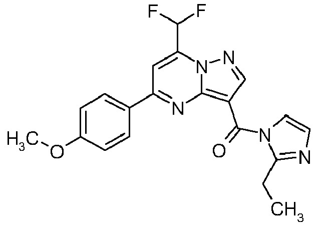 | 397.39 | [7-Difluoromethyl-5-(4-methoxy-phenyl)-pyrazolo[1,5-a]pyrimidin-3-yl]-(2-ethyl-imidazol-1-yl)-methanone |
| 5-42 | 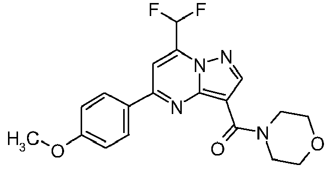 | 388.38 | [7-Difluoromethyl-5-(4-methoxy-phenyl)-pyrazolo[1,5-a]pyrimidin-3-yl]-morpholin-4-yl-methanone |
| 5-43 | 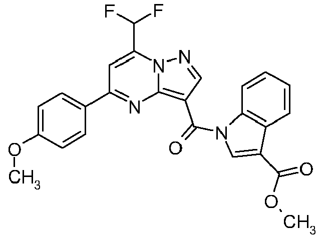 | 476.44 | 1-[7-Difluoromethyl-5-(4-methoxy-phenyl)-pyrazolo[1,5-a]pyrimidine-3-carbonyl]-1H-indole-3-carboxylic acid methyl ester |
| 5-44 | 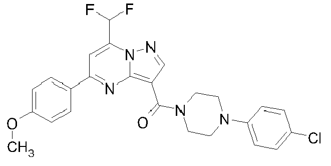 | 497.94 | [4-(4-Chloro-phenyl)-piperazin-1-yl]-[7-difluoromethyl-5-(4-methoxy-phenyl)-pyrazolo[1,5-a]pyrimidin-3-yl]-methanone |
| 5-45 | 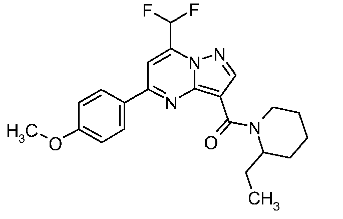 | 414.46 | [7-Difluoromethyl-5-(4-methoxy-phenyl)-pyrazolo[1,5-a]pyrimidin-3-yl]-(2-ethyl-piperidin-1-yl)-methanone |
| 5-46 | 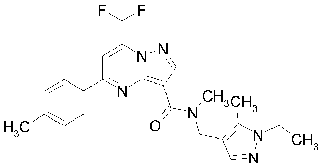 | 438.48 | 7-Difluoromethyl-5-p-tolyl-pyrazolo[1,5-a]pyrimidine-3-carboxylic acid (1-ethyl-5-methyl-1 H-pyrazol-4-ylmethyl)-methyl-amide |
| 5-47 | 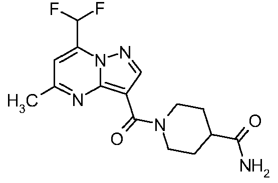 | 337.33 | 1-(7-Difluoromethyl-5-methyl-pyrazolo[1,5-a]pyrimidine-3-carbonyl)-piperidine-4-carboxylic acid amide |
| 5-48 | 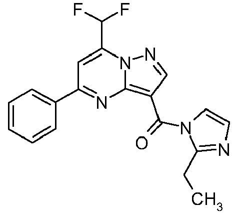 | 367.36 | (7-Difluoromethyl-5-phenyl-pyrazolo[1,5-a]pyrimidin-3-yl)-(2-ethyl-imidazol-1-yl)-methanone |
| 5-49 | 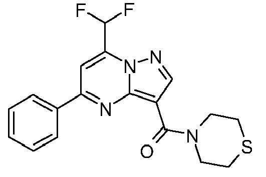 | 374.42 | (7-Difluoromethyl-5-phenyl-pyrazolo[1,5-a]pyrimidin-3-yl)-thiomorpholin-4-yl-methanone |
| 5-50 | 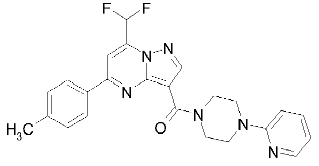 | 448.48 | (7-Difluoromethyl-5-p-tolyl-pyrazolo[1,5-a]pyrimidin-3-yl)-(4-pyridin-2-yl-piperazin-1-yl)-methanone |
| 5-51 | 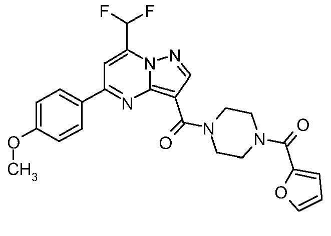 | 481.46 | [7-Difluoromethyl-5-(4-methoxy-phenyl)-pyrazolo[1,5-a]pyrimidin-3-yl]-[4-(furan-2-carbonyl)-piperazin-1-yl]-methanone |
| 5-52 | 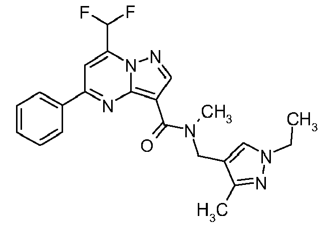 | 424.46 | 7-Difluoromethyl-5-phenyl-pyrazolo[1,5-a]pyrimidine-3-carboxylic acid (1-ethyl-3-methyl-1H-pyrazol-4-ylmethyl)-methyl-amide |
| 5-53 | 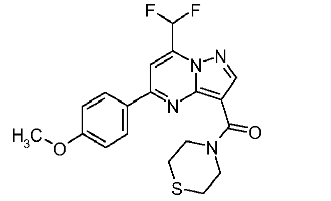 | 404.44 | [7-Difluoromethyl-5-(4-methoxy-phenyl)-pyrazolo[1,5-a]pyrimidin-3-yl]-thiomorpholin-4-yl-methanone |
| 5-54 | 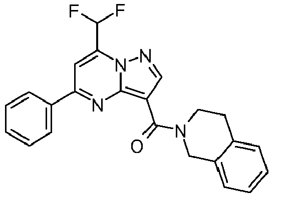 | 404.42 | (7-Difluoromethyl-5-phenyl-pyrazolo[1,5-a]pyrimidin-3-yl)-(3,4-dihydro-1 H-isoquinolin-2-yl)-methanone |
| 5-55 | 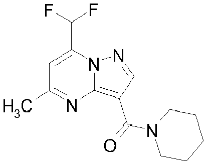 | 294.31 | (7-Difluoromethyl-5-methyl-pyrazolo[1,5-a]pyrimidin-3-yl)-piperidin-1-yl-methanone |
| 5-56 | 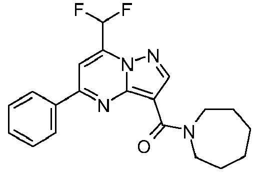 | 370.41 | Azepan-1-yl-(7-difluoromethyl-5-phenyl-pyrazolo[1,5-a]pyrimidin-3-yl)-methanone |
| 5-57 | 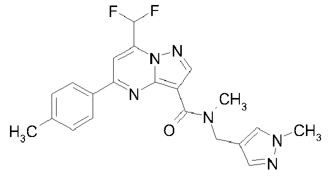 | 410.43 | 7-Difluoromethyl-5-p-tolyl-pyrazolo[1,5-a]pyrimidine-3-carboxylic acid methyl-(1-methyl-1 H-pyrazol-4-ylmethyl)-amide |
| 5-58 | 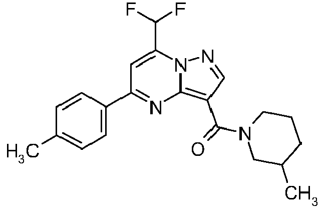 | 384.43 | (7-Difluoromethyl-5-p-tolyl-pyrazolo[1,5-a]pyrimidin-3-yl)-(3-methyl-piperidin-1-yl)-methanone |
| 5-59 | 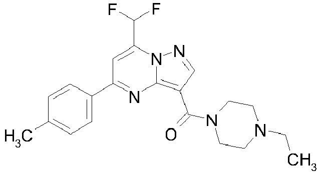 | 399.45 | (7-Difluoromethyl-5-p-tolyl-pyrazolo[1,5-a]pyrimidin-3-yl)-(4-ethyl-piperazin-1-yl)-methanone |
| 5-60 | 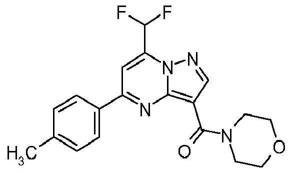 | 372.38 | (7-Difluoromethyl-5-p-tolyl-pyrazolo[1,5-a]pyrimidin-3-yl)-morpholin-4-yl-methanone |
| 5-61 | 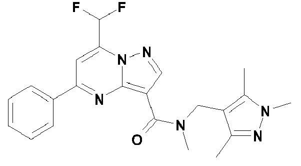 | 424.46 | 7-Difluoromethyl-5-phenyl-pyrazolo[1,5-a]pyrimidine-3-carboxylic acid methyl-(1,3,5-trimethyl-1H-pyrazol-4-ylmethyl)-amide |
| 5-62 | 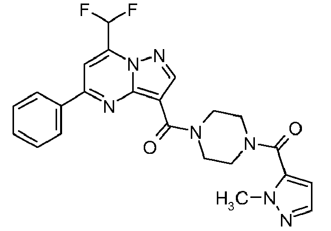 | 465.47 | (7-Difluoromethyl-5-phenyl-pyrazolo[1,5-a] pyrimidin-3-yl)-[4-(2-methyl-2H-pyrazole-3-carbonyl)-piperazin-1-yl]-methanone |
| 5-63 | 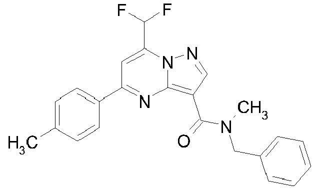 | 406.44 | 7-Difluoromethyl-5-p-tolyl-pyrazolo[1,5-a]pyrimidine-3-carboxylic acid benzyl-methyl-amide |
| 5-64 | 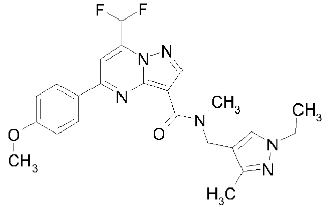 | 454.48 | 7-Difluoromethyl-5-(4-methoxy-phenyl)-pyrazolo[1,5-a]pyrimidine-3-carboxylic acid (1-ethyl-3-methyl-1H-pyrazol-4-ylmethyl)-methyl-amide |
| 5-65 | 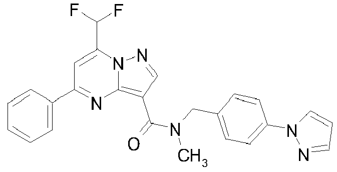 | 458.47 | 7-Difluoromethyl-5-phenyl-pyrazolo[1,5-a]pyrimidine-3-carboxylic acid methyl-(4-pyrazol-1-yl-benzyl)-amide |
| 5-66 | 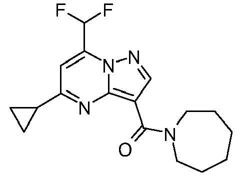 | 334.37 | Azepan-1-yl-(5-cyclopropyl-7-difluoromethyl-pyrazolo[1,5-a]pyrimidin-3-yl)-methanone |
| 5-67 | 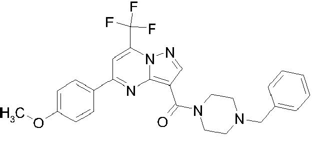 | 495.51 | (4-Benzyl-piperazin-1-yl)-[5-(4-methoxy-phenyl)-7-trifluoromethyl-pyrazolo[1,5-a]pyrimidin-3-yl]-methanone |
| 5-68 | 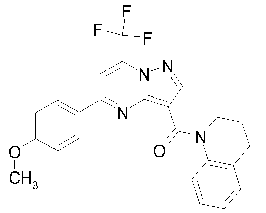 | 452.44 | (3,4-Dihydro-2H-quinolin-1-yl)-[5-(4-methoxy-phenyl)-7-trifluoromethyl-pyrazolo[1,5-a]pyrimidin-3-yl]-methanone |
| 5-69 | 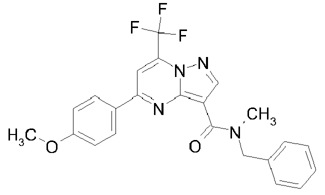 | 440.43 | 5-(4-Methoxy-phenyl)-7-trifluoromethyl-pyrazolo[1,5-a]pyrimidine-3-carboxylic acid ben-zyl-methyl-amide |
| 5-70 | 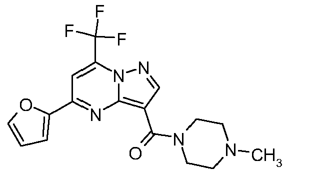 | 379.34 | (5-Furan-2-yl-7-trifluoromethyl-pyrazolo[1,5-a]pyrimidin-3-yl)-(4-methyl-piperazin-1-yl)-methanone |
| 5-71 | 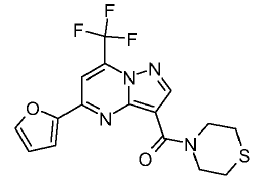 | 382.37 | (5-Furan-2-yl-7-trifluoromethyl-pyrazolo[1,5-a]pyrimidin-3-yl)-thiomorpholin-4-yl-methanone |
| 5-72 | 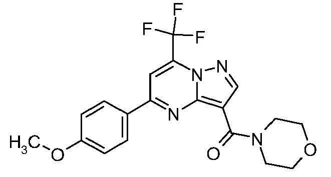 | 406.37 | [5-(4-Methoxy-phenyl)-7-trifluoromethyl-pyrazolo[1,5-a]pyrimidin-3-yl]-morpholin-4-yl-methanone |
| 5-73 | 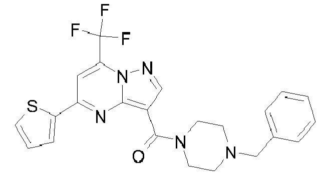 | 471.51 | (4-Benzyl-piperazin-1-yl)-(5-thiophen-2-yl-7-trifluoromethyl-pyrazolo[1,5-a]pyrimidin-3-yl)-methanone |
| 5-74 | 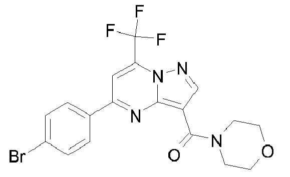 | 455.24 | [5-(4-Bromo-phenyl)-7-trifluoromethyl-pyrazolo[1,5-a]pyrimidin-3-yl]-morpholin-4-yl-methanone |
| 5-75 | 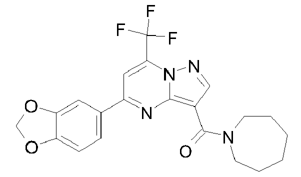 | 432.41 | Azepan-1-yl-(5-benzo[1,3]dioxol-5-yl-7-trifluoromethyl-pyrazolo[1,5-a]pyrimidin-3-yl)-methanone |
| 5-76 | 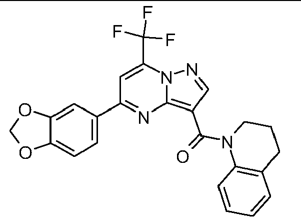 | 466.42 | (5-Benzo[1,3]dioxol-5-yl-7-trifluoromethyl-pyrazolo[1,5-a]pyrimidin-3-yl)-(3,4-dihydro-2H-quinolin-1-yl)-methanone |
| 5-77 | 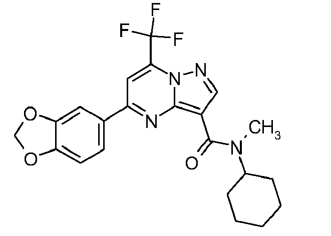 | 446.43 | 5-Benzo[1,3]dioxol-5-yl-7-trifluoromethyl-pyrazolo[1,5-a]pyrimidine-3-carboxylic acid cyclohexyl-methyl-amide |
| 5-78 | 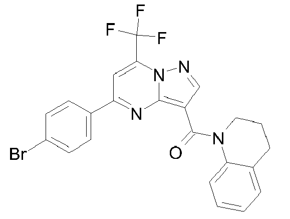 | 501.31 | [5-(4-Bromo-phenyl)-7-trifluoromethyl-pyrazolo[1,5-a]pyrimidin-3-yl]-(3,4-d i hydro-2H-quinolin-1-yl)-methanone |
| 5-79 | 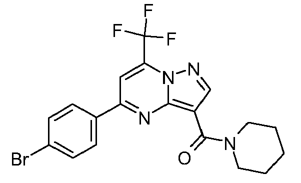 | 453.27 | [5-(4-Bromo-phenyl)-7-trifluoromethyl-pyrazolo[1,5-a]pyrimidin-3-yl]-piperidin-1-yl-methanone |
| 5-80 | 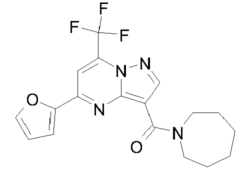 | 378.36 | Azepan-1-yl-(5-furan-2-yl-7-trifluoromethyl-pyrazolo[1,5-a]pyrimidin-3-yl)-methanone |
| 5-81 | 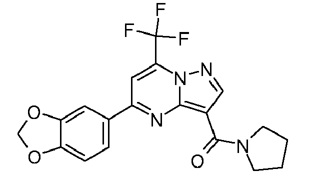 | 404.35 | (5-Benzo[1,3]dioxol-5-yl-7-trifluoromethyl-pyrazolo[1,5-a]pyrimidin-3-yl)-pyrrolidin-1-yl-methanone |
| 5-82 | 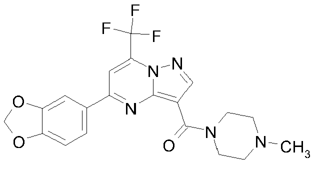 | 433.39 | (5-Benzo[1,3]dioxol-5-yl-7-trifluoromethyl-pyrazolo[1,5-a]pyrimidin-3-yl)-(4-methyl-piperazin-1-yl)-methanone |
| 5-83 | 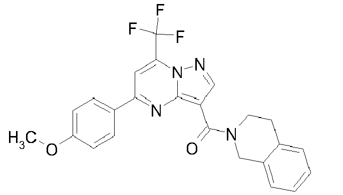 | 452.44 | (3,4-Dihydro-1 H-isoquinolin-2-yl)-[5-(4-methoxy-phenyl)-7-trifluoromethyl-pyrazolo[1,5-a]pyrimidin-3-yl]-methanone |
| 5-84 | 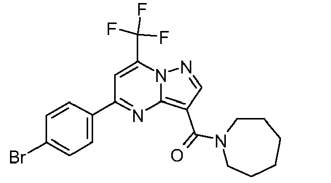 | 467.30 | Azepan-1-yl-[5-(4-bromo-phenyl)-7-trifluoromethyl-pyrazolo[1,5-a]pyrimidin-3-yl]-methanone |
| 5-85 | 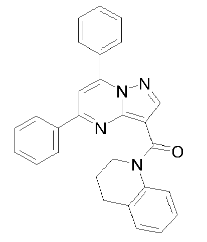 | 430.51 | (3,4-Dihydro-2H-quinolin-1-yl)-(5,7-diphenyl-pyrazolo[1,5-a]pyrimidin-3-yl)-methanone |
| 5-86 | 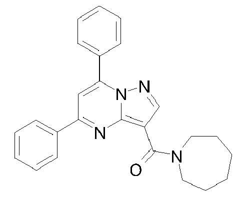 | 396.50 | Azepan-1-yl-(5,7-diphenyl-pyrazolo[1,5-a]pyrimidin-3-yl)-methanone |
| 5-87 | 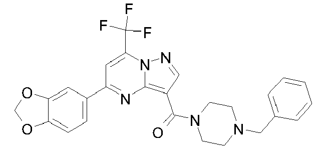 | 509.49 | (5-Benzo[1,3]dioxol-5-yl-7-trifluoromethyl-pyrazolo[1,5-a]pyrimidin-3-yl)-(4-benzyl-piperazin-1-yl)-methanone |
| 5-88 | 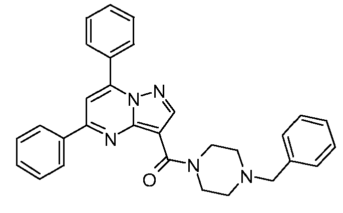 | 473.58 | (4-Benzyl-piperazin-1-yl)-(5,7-diphenyl-pyrazolo[1,5-a]pyrimidin-3-yl)-methanone |
| 5-89 | 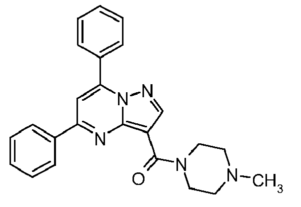 | 397.48 | (5,7-Diphenyl-pyrazolo[1,5-a]pyrimidin-3-yl)-(4-methyl-piperazin-1-yl)-methanone |
| 5-90 | 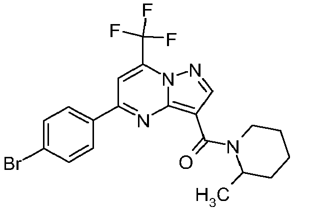 | 467.30 | [5-(4-Bromo-phenyl)-7-trifluoromethyl-pyrazolo[1,5-a]pyrimidin-3-yl]-(2-methyl-piperidin-1-yl)-methanone |
| 5-91 | 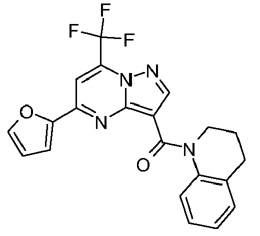 | 412.37 | (3,4-Dihydro-2H-quinolin-1-yl)-(5-furan-2-yl-7-trifluoromethyl-pyrazolo[1,5-a]pyrimidin-3-yl)-methanone |
| 5-92 | 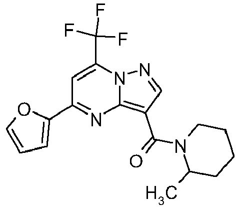 | 378.36 | (5-Furan-2-yl-7-trifluoromethyl-pyrazolo[1,5-a]pyrimidin-3-yl)-(2-methyl-piperidin-1-yl)-methanone |
| 5-93 | 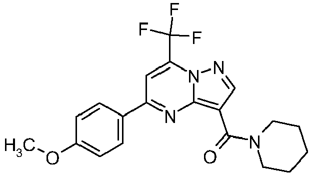 | 404.40 | [5-(4-Methoxy-phenyl)-7-trifluoromethyl-pyrazolo[1,5-a]pyrimidin-3-yl]-piperidin-1-yl-methanone |
| 5-94 | 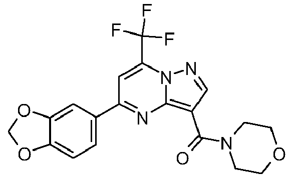 | 420.35 | (5-Benzo[1,3]dioxol-5-yl-7-trifluoromethyl-pyrazolo[1,5-a]pyrimidin-3-yl)-morpholin-4-yl-methanone |
| 5-95 | 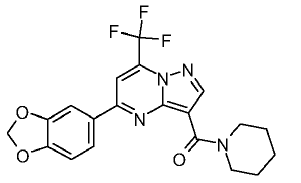 | 418.38 | (5-Benzo[1,3]dioxol-5-yl-7-trifluoromethyl-pyrazolo[1,5-a]pyrimidin-3-yl)-piperidin-1-yl-methanone |
| 5-96 | 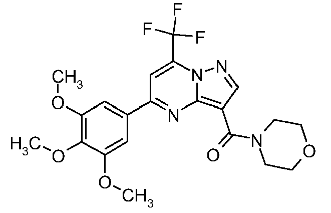 | 466.42 | Morpholin-4-yl-[7-trifluoromethyl-5-(3,4,5-trimethoxy-phenyl)-pyrazolo[1,5-a]pyrimidin-3-yl]-methanone |
| 5-97 | 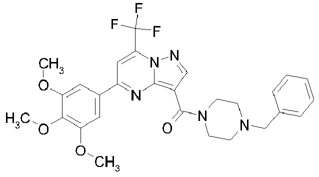 | 555.56 | (4-Benzyl-piperazin-1-yl)-[7-trifluoromethyl-5-(3,4,5-trimethoxy-phenyl)-pyrazolo[1,5-a]pyrimidin-3-yl]-methanone |
| 5-98 | 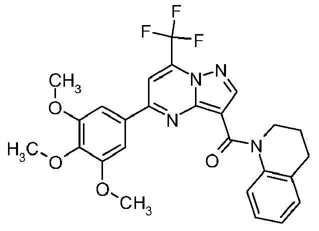 | 512.49 | (3,4-Dihydro-2H-quinolin-1-yl)-[7-trifluoromethyl-5-(3,4,5-trimethoxy-phenyl)-pyrazolo[1,5-a]pyrimidin-3-yl]-methanone |
| 5-99 | 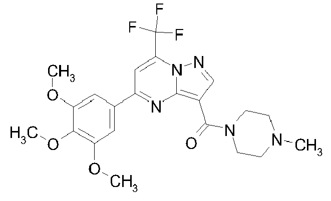 | 479.46 | (4-Methyl-piperazin-1-yl)-[7-trifluoromethyl-5-(3,4,5-trimethoxy-phenyl)-pyrazolo[1,5-a]pyrimidin-3-yl]-methanone |
| 5-100 | 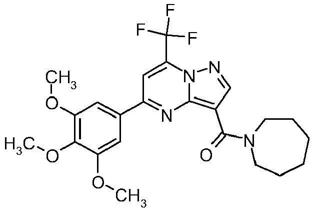 | 478.48 | Azepan-1-yl-[7-trifluoromethyl-5-(3,4,5-trimethoxy-phenyl)-pyrazolo[1,5-a]pyrimidin-3-yl]-methanone |
| 5-101 | 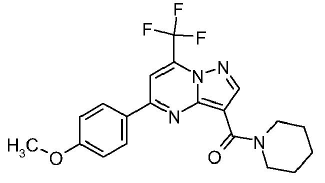 | 404.40 | [5-(4-Methoxy-phenyl)-7-trifluoromethyl-pyrazolo[1,5-a]pyrimidin-3-yl]-piperidin-1-yl-methanone |
| 5-102 | 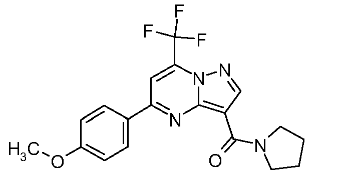 | 390.37 | [5-(4-Methoxy-phenyl)-7-trifluoromethyl-pyrazolo[1,5-a]pyrimidin-3-yl]-pyrrolidin-1-yl-methanone |
| 5-103 | 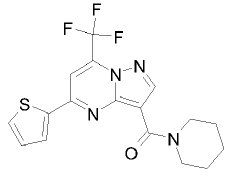 | 380.39 | Piperidin-1-yl-(5-thiophen-2-yl-7-trifluoromethyl-pyrazolo[1,5-a]pyrimidin-3-yl)-methanone |
| 5-104 | 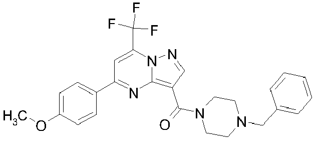 | 495.51 | (4-Benzyl-piperazin-1-yl)-[5-(4-methoxy-phenyl)-7-trifluoromethyl-pyrazolo[1,5-a]pyrimidin-3-yl]-methanone |
| 5-105 | 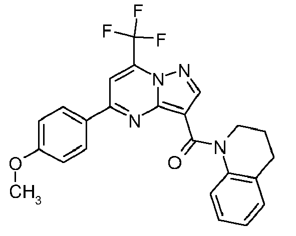 | 452.44 | (3,4-Di hydro-2H-quinolin-1-yl)-[5-(4-methoxy-phenyl)-7-trifluoromethyl-pyrazolo[1,5-a]pyrimidin-3-yl]-methanone |
| 5-106 | 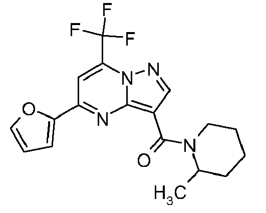 | 378.36 | (5-Furan-2-yl-7-trifluoromethyl-pyrazolo[1,5-a]pyrimidin-3-yl)-(2-methyl-piperidin-1-yl)-methanone |
| 5-107 | 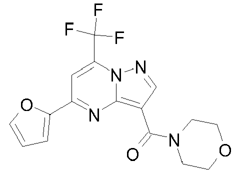 | 366.30 | (5-Furan-2-yl-7-trifluoromethyl-pyrazolo[1,5-a]pyrimidin-3-yl)-morpholin-4-yl-methanone |
| 5-108 | 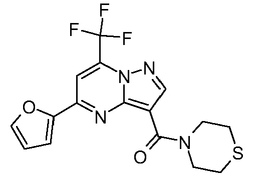 | 382.37 | (5-Furan-2-yl-7-trifluoromethyl-pyrazolo[1,5-a]pyrimidin-3-yl)-thiomorpholin-4-yl-methanone |
| 5-109 | 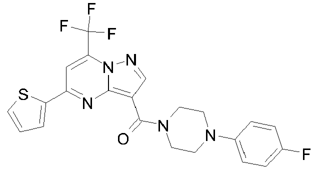 | 475.47 | [4-(4-Fluoro-phenyl)-piperazin-1-yl]-(5-thiophen-2-yl-7-trifluoromethyl-pyrazolo[1,5-a]pyrimidin-3-yl)-methanone |
| 5-110 | 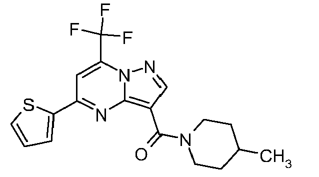 | 394.42 | (4-Methyl-piperidin-1-yl)-(5-thiophen-2-yl-7-trifluoromethyl-pyrazolo[1,5-a]pyrimidin-3-yl)-methanone |
| 5-111 | 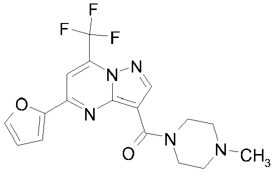 | 379.34 | (5-Furan-2-yl-7-trifluoromethyl-pyrazolo[1,5-a]pyrimidin-3-yl)-(4-methyl-piperazin-1-yl)-methanone |
| 5-112 | 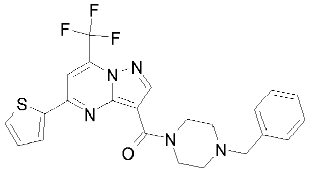 | 471.51 | (4-Benzyl-piperazin-1-yl)-(5-thiophen-2-yl-7-trifluoromethyl-pyrazolo[1,5-a]pyrimidin-3-yl)-methanone |
| 5-113 | 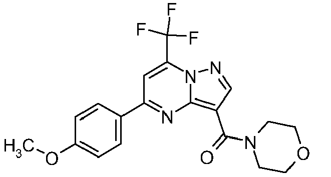 | 406.37 | [5-(4-Methoxy-phenyl)-7-trifluoromethyl-pyrazolo[1,5-a] pyrimidin-3-yl]-morpholin-4-yl-methanone |
| 5-114 | 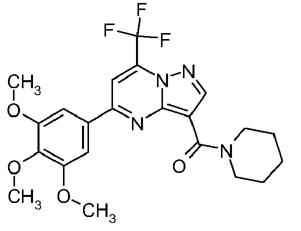 | 464.45 | Piperidin-1-yl-[7-trifluoromethyl-5-(3,4,5-trimethoxy-phenyl)-pyrazolo[1,5-a]pyrimidin-3-yl]-methanone |
| 5-115 | 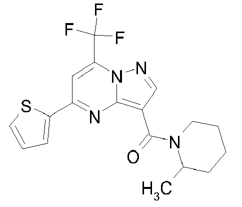 | 394.42 | (2-Methyl-piperidin-1-yl)-(5-thiophen-2-yl-7-trifluoromethyl-pyrazolo[1,5-a]pyrimidin-3-yl)-methanone |
| 5-116 | 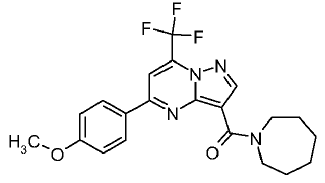 | 418.42 | Azepan-1-yl-[5-(4-methoxy-phenyl)-7-trifluoromethyl-pyrazolo[1,5-a]pyrimidin-3-yl]-methanone |
| 5-117 | 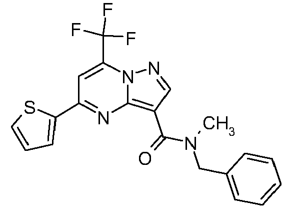 | 416.43 | 5-Thiophen-2-yl-7-trifluoromethyl-pyrazolo[1,5-a]pyrimidine-3-carboxylic acid benzyl-methyl-amide |
| 5-118 | 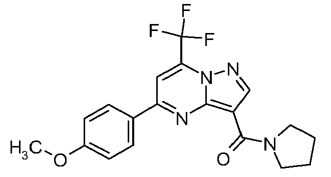 | 390.37 | [5-(4-Methoxy-phenyl)-7-trifluoromethyl-pyrazolo[1,5-a]pyrimidin-3-yl]-pyrrolidin-1-yl-methanone |
| 5-119 | 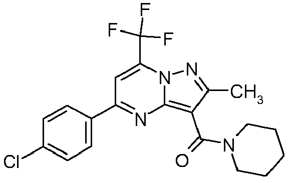 | 422.84 | [5-(4-Chloro-phenyl)-2-methyl-7-trifluoromethyl-pyrazolo[1,5-a]pyrimidin-3-yl]-piperidin-1-yl-methanone |
| 5-120 | 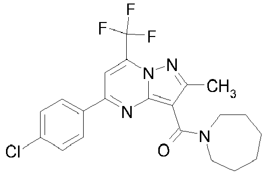 | 436.87 | Azepan-1-yl-[5-(4-chloro-phenyl)-2-methyl-7-trifluoromethyl-pyrazolo[1,5-a]pyrimidin-3-yl]-methanone |
| 5-121 | 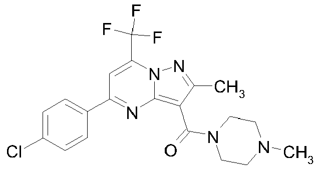 | 437.86 | [5-(4-Chloro-phenyl)-2-methyl-7-trifluoromethyl-pyrazolo[1,5-a]pyrimidin-3-yl]-(4-methyl-piperazin-1-yl)-methanone |
| 5-122 | 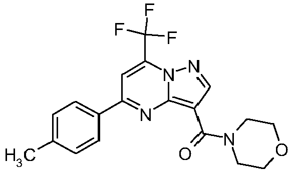 | 390.37 | Morpholin-4-yl-(5-p-tolyl-7-trifluoromethyl-pyrazolo[1,5-a]pyrimidin-3-yl)-methanone |
| 5-123 | 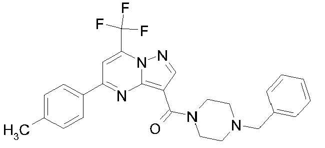 | 479.51 | (4-Benzyl-piperazin-1-yl)-(5-p-tolyl-7-trifluoromethyl-pyrazolo[1,5-a]pyrimidin-3-yl)-methanone |
| 5-124 | 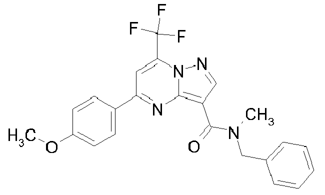 | 440.43 | 5-(4-Methoxy-phenyl)-7-trifluoromethyl-pyrazolo[1,5-a]pyrimidine-3-carboxylic acid ben-zyl-methyl-amide |
| 5-125 | 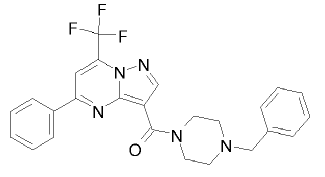 | 465.48 | (4-Benzyl-piperazin-1-yl)-(5-phenyl-7-trifluoromethyl-pyrazolo[1,5-a]pyrimidin-3-yl)-methanone |
| 5-126 | 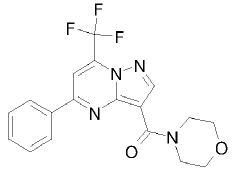 | 376.34 | Morpholin-4-yl-(5-phenyl-7-trifluoromethyl-pyrazolo[1,5-a]pyrimidin-3-yl)-methanone |

### PHARMACOLOGICAL METHODS

### 11 βHSD1 enzyme assay

### Materials

³H-cortisone and anti-rabbit lg coated scintillation proximity assay (SPA) beads were purchased from Amersham Pharmacia Biotech, β-NADPH was from Sigma and rabbit anticortisol antibodies were from Fitzgerald. An extract of yeast transformed with h-11βHSD1 (Hult et al., FEBS Lett; 441, 25 (1998)) was used as the source of enzyme. The test compounds were dissolved in DMSO (10 mM). All dilutions were performed in a buffer containing 50 mM TRIS-HCl (Sigma Chemical Co), 4 mM EDTA (Sigma Chemical Co), 0.1 % BSA (Sigma Chemical Co), 0.01% Tween-20 (Sigma Chemical Co) and 0.005% bacitracin (Novo Nordisk A/S), pH=7.4. Optiplate 96 wells plates were supplied by Packard. The amount of ³H-cortisol bound to the SPA beads was measured on TopCount NXT, Packard.

### Methods

h-11βHSD1, 120 nM ³H-cortisone, 4 mM β-NADPH, antibody (1:200), serial dilutions of test compound and SPA particles (2 mg/well) were added to the wells. The reaction was initiated by mixing the different components and was allowed to proceed under shaking for 60 min at 30°C. The reaction was stopped be the addition of 10 fold excess of a stopping buffer containing 500 µM carbenoxolone and 1 µM cortisone. Data was analysed using GraphPad Prism software.

**Table 1**

| Inhibition of 11 βHSD1 by compounds of the invention | |
|---|---|
| Example no. | 11βHSD1 |
| | IC₅₀ values (µM) |
| 3 | 4.7 |
| 4 | 0.34 |

While the invention has been described and illustrated with reference to certain preferred embodiments thereof, those skilled in the art will appreciate that various changes, modifications, and substitutions can be made therein without departing from the spirit and scope of the present invention. Likewise, the specific pharmacological responses observed may vary according to and depending on the particular active compound selected or whether there are present pharmaceutical carriers, as well as the type of formulation and mode of administration employed, and such expected variations or differences in the results are contemplated in accordance with the objects and practices of the present invention. Accordingly, the invention is not to be limited as by the appended claims.

The features disclosed in the foregoing description and/or in the claims may both separately and in any combination thereof be material for realising the invention in diverse forms thereof.

Preferred features of the invention:
1. Use of a substituted pyrazolo[1 ,5-a]pyrimidine, a prodrug thereof, or a salt thereof with a pharmaceutically acceptable acid or base, or any optical isomer or mixture of optical isomers, including a racemic mixture or any tautomeric forms, for
   a) modulation of the activity of 11 βHSD1; or
   b) inhibition of 11βHSD1,
   in a patient in need thereof.
2. Use of a substituted pyrazolo[1,5-*a*]pyrimidine, a prodrug thereof, or a salt thereof with a pharmaceutically acceptable acid or base, or any optical isomer or mixture of optical isomers, including a racemic mixture or any tautomeric forms, for the preparation of a pharmaceutical composition for the treatment, prevention and/or prophylaxis of any disorder and disease where it is desirable to
   a) modulate the activity of 11 βHSD1; or
   b) inhibit 11βHSD1,
   in a patient in need thereof.
3. The use according to clause 1 or 2, wherein the substituted pyrazolo[1 ,5-a]pyrimidine or a prodrug thereof is of the general formula (I) 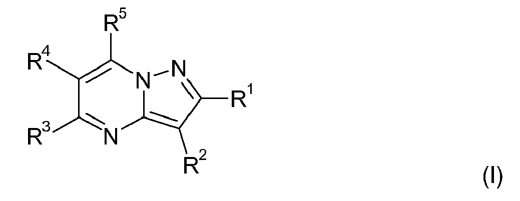 wherein
   R¹ and R² independently are hydrogen, halo, C(=O)NR⁶R⁷, CO₂R¹⁵, NR⁶C(=O)R¹¹, OR¹² or SR¹²;
   R³ and R⁵ independently are hydrogen, NR¹³R¹⁴, trihalomethyl, trihalomethoxy, C₁-C₆alkyl, C₂-C₆alkenyl, C₂-C₆alkynyl, C₁-C₆alkyloxy, C₁-C₆alkylthio, aryl, arylC₁-C₆alkyl, hetaryl or hetarylC₁-C₆alkyl, wherein alkyl, alkynyl, alkenyl, aryl, hetaryl, arylalkyl or hetarylalkyl groups independently are optionally substituted with one or more of R⁸;
   R⁴ is hydrogen, cyano, halo, CO₂R¹⁵, C(=O)NR⁶R⁷, C₁-C₆alkyl, C₂-C₆alkenyl, C₂-C₆alkynyl, aryl, hetaryl, arylC₁-C₆alkyl, hetarylC₁-C₆alkyl, hydroxy, C₁-C₆alkyloxy, arylC₁-C₆alkyloxy, hetarylC₁-C₆alkyloxy, or C₁-C₆alkyloxyC₁-C₆alkyl, wherein the alkyl, alkynyl, alkenyl, cycloalkyl, aryl, hetaryl, arylalkyl or hetarylalkyl groups independently are optionally substituted with one or more of R⁹;
   R⁶ and R⁷ independently are C₁-C₆alkyl, C₃-C₁₀cycloalkyl, hetC₃-C₁₀cycloalkyl, arylC₁-C₆alkyl or hetarylC₁-C₆alkyl, wherein the alkyl, cycloalkyl, hetcycloalkyl, arylalkyl, and hetarylalkyl groups independently are optionally substituted with one or more of R¹⁰; or
   R⁶ and R⁷ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆-alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
   R⁸ and R⁹ independently are hydrogen, halo, hydroxy, oxo, cyano, nitro, C₃-C₁₀cycloalkyl, C₃-C₁₀hetocycloalkyl, C₁-C₆alkyl, C₁-C₆alkyloxy, trihalomethyl, trihalomethoxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆-alkylcarboxy, hetarylC₁-C₆alkylcarboxy, C₁-C₆alkylcarbonylamino or arylC₁-C₆alkylcarbonylamino;
   R¹⁰ is hydrogen, halo, cyano, nitro, hydroxy, oxo, C₃-C₁₀cydoalkyl, C₃-C₁₀hetcycloalkyl, C₁-C₆alkyl, C₁-C₆alkyloxy, trihalomethyl, trihalometh dialkylamino oxy, arylC₁-C₆alkyloxy, hetaryl-C₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;
   R¹¹ is C₁-C₆alkyl, C₂-C₆alkenyl, C₂-C₆alkynyl, arylC₁-C₆alkyl, hetarylC₁-C₆alkyl, C₁-C₆alkylcarbonylC₁-C₆alkyl, C₁-C₆alkyloxy, aryloxy, C₁-C₆alkyloxy, arylcarbonyl, arylC₁-C₆alkylcarbonyl, hetarylcarbonylC₁-C₆alkyl, wherein the alkyl, alkenyl, alkynyl, aryl and hetaryl groups independently are optionally substituted with one or more of R⁹;
   R¹² is C₁-C₆alkylcarbonylaminoC₁-C₆alkyl, arylcarbonylaminoC₁-C₆alkyl or arylC₁-C₆alkylcarbonylaminoC₁-C₆alkyl;
   R¹³and R¹⁴ independently are hydrogen, oxo, C₃-C₁₀cycloalkyl, C₁-C₆alkyl, C₁-C₆alkylcarbonyl, arylcarbonyl, hetarylcarbonyl, arylC₁-C₆alkylcarbonyl, hetarylC₁-C₆alkylcarbonyl;
   R¹⁵ is hydrogen, C₃-C₁₀cycloalkyl, C₁-C₆alkyl, aryl, hetaryl, arylC₁-C₆alkyl, hetarylC₁-C₆alkyl, C₁-C₆alkyloxyalkyl or arylC₁-C₆alkyloxyalkyl; or
   a salt thereof with a pharmaceutically acceptable acid or base, or any optical isomer or mixture of optical isomers, including a racemic mixture, or any tautomeric forms.
4. The use according to any of the clauses 1-3, wherein the substituted pyrazolo[1,5-*a*]pyrimidine or a prodrug thereof is of the general formula (1) 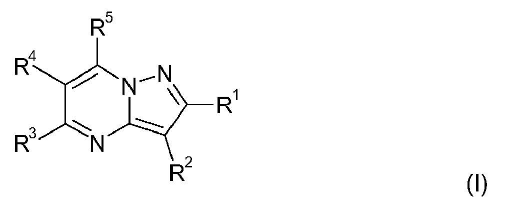 wherein
   R¹ and R² independently are hydrogen, halo, C(=O)NR⁶R⁷, NC(=O)R¹¹, OR¹² or SR¹²;
   R³ and R⁵ independently are hydrogen, NR¹³R¹⁴, trihalomethyl, trihalomethoxy, C₁-C₆alkyl, C₂-C₆alkenyl, C₂-C₆alkynyl, C₁-C₆alkyloxy, C₁-C₆alkylthio, aryl, arylC₁-C₆alkyl, hetaryl or hetaryl-C₁-C₆alkyl, wherein alkyl, alkynyl, alkenyl, aryl, hetaryl, arylalkyl or hetarylalkyl groups independently are optionally substituted with one or more of R⁸;
   R⁴ is hydrogen, cyano, halo, CO₂R¹⁵, C(=O)NR⁶R⁷, C₁-C₆alkyl, C₂-C₆alkenyl, C₂-C₆alkynyl, aryl, hetaryl, arylC₁-C₆alkyl, hetarylC₁-C₆alkyl, hydroxy, C₁-C₆alkyloxy, arylC₁-C₆alkyloxy, hetarylC₁-C₆alkyloxy, or C₁-C₆alkyloxyC₁-C₆alkyl, wherein the alkyl, alkynyl, alkenyl, cycloalkyl, aryl, hetaryl, arylalkyl or hetarylalkyl groups independently are optionally substituted with one or more of R⁹;
   R⁶ and R⁷ independently are C₁-C₆alkyl, C₃-C₁₀cycloalkyl, hetC₃-C₁₀cycloalkyl, arylC₁-C₆alkyl or hetarylC₁-C₆alkyl, wherein the alkyl, cycloalkyl, hetcycloalkyl, arylalkyl, and hetarylalkyl groups independently are optionally substituted with one or more of R¹⁰; or
   R⁶ and R⁷ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
   R⁸ and R⁹ independently are hydrogen, halo, hydroxy, oxo, cyano, nitro, C₃-C₁₀cycloalkyl, C₃-C₁₀hetocycloalkyl, C₁-C₆alkyl, C₁-C₆alkyloxy, trihalomethyl, trihalomethoxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆-alkylcarboxy, hetarylC₁-C₆alkylcarboxy, C₁-C₆alkylcarbonylamino or arylC₁-C₆alkylcarbonylamino;
   R¹⁰ is hydrogen, halo, cyano, nitro, hydroxy, oxo, C₃-C₁₀cycloalkyl, C₃-C₁₀hetcycloalkyl, C₁-C₆alkyl, C₁-C₆alkyloxy, trihalomethyl, trihalometh dialkylamino oxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;
   R¹¹ is C₁-C₆alkyl, C₂-C₆alkenyl, C₂-C₆alkynyl, arylC₁-C₆alkyl, hetarylC₁-C₆alkyl, C₁-C₆alkylcarbonylC₁-C₆alkyl, C₁-C₆alkyl, arylcarbonyl, arylC₁-C₆alkylcarbonyl, hetarylcarbonylC₁-C₆-alkyl, wherein the alkyl, alkenyl, alkynyl, aryl and hetaryl groups independently are optionally substituted with one or more of R⁹;
   R¹² is C₁-C₆alkylcarbonylaminoC₁-C₆alkyl, arylcarbonylaminoC₁-C₆alkyl or arylC₁-C₆alkylcarbonylaminoC₁-C₆alkyl;
   R¹³and R¹⁴ independently are hydrogen, oxo, C₃-C₁₀ cycloalkyl, C₁-C₆alkyl, C₁-C₆alkyloxy, trihalomethyl, trihalomethoxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy;
   R¹⁵ is hydrogen, C₃-C₁₀cycloalkyl, C₁-C₆alkyl, aryl, hetaryl, arylC₁-C₆alkyl, hetarylC₁-C₆alkyl, C₁-C₆alkyloxyalkyl or arylC₁-C₆alkyloxyalkyl; or
   a salt thereof with a pharmaceutically acceptable acid or base, or any optical isomer or mixture of optical isomers, including a racemic mixture, or any tautomeric forms.
5. The use according to any of the clauses 1-4, wherein in formula (I) R¹ and R² independently are hydrogen and/or C(=O)NR⁶R⁷.
6. The use according to any of the clauses 1-5 wherein in formula (I) R³ and R⁵ independently are hydrogen, trihalomethyl, C₁-C₆alkyl, C₁-C₆alkyloxy, aryl, arylC₁-C₆alkyl, hetaryl or hetarylC₁-C₆alkyl, wherein alkyl, aryl, hetaryl, arylalkyl or hetarylalkyl groups independently are optionally substituted with one or more of R⁸.
7. The use according to any of the clauses 1-6, wherein in formula (I) R⁴ is hydrogen, cyano, halo, C(=O)NR⁶R⁷, C₁-C₆alkyl, aryl, hetaryl, arylC₁-C₆alkyl, hetarylC₁-C₆alkyl, hydroxy, C₁-C₆alkyloxy, arylC₁-C₆alkyloxy, hetarylC₁-C₆alkyloxy, wherein the alkyl, aryl, hetaryl, arylalkyl or hetarylalkyl groups independently are optionally substituted with one or more of R⁹.
8. The use according to any of the clauses 1-7, wherein in formula (I) R⁶ and R⁷ independently are C₁-C₆alkyl, C₃-C₁₀cycloalkyl, hetC₃-C₁₀cycloalkyl, arylC₁-C₆alkyl or hetarylC₁-C₆alkyl, wherein the alkyl, cycloalkyl, hetcycloalkyl, arylalkyl, and hetarylalkyl groups independently are optionally substituted with one or more of R¹⁰.
9. The use according to any of the clauses 1-7, wherein in formula (I)
   R⁶ and R⁷ together with the nitrogen to which they are attached, are forming a saturated or partially saturated cyclic, bicyclic or tricyclic ring system containing from 4 to 10 carbon atoms and from 0 to 2 additional heteroatoms selected from nitrogen or oxygen, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
10. The use according to any of the clauses 1-9, wherein in formula (I) R⁸ and R⁹ independently are hydrogen, halo, hydroxy, oxo, cyanoC₃-C₁₀cycloalkyl, C₃-C₁₀hetocycloalkyl, C₁-C₆alkyl, C₁-C₆alkyloxy, trihalomethyl, arylC₁-C₆alkyloxy, hetarylC₁-C₆alkyloxy.
11. The use according to any of the clauses 1-10 wherein in formula (I) R¹⁰ is hydrogen, halo, cyano, nitro, hydroxy, oxo, C₃-C₁₀cycloalkyl, C₃-C₁₀hetcycloalkyl, C₁-C₆alkyl, C₁-C₆alkyloxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy.
12. The use according to any of the clauses 1-11, wherein in formula (I) R¹¹ is C₁-C₆alkyl, arylC₁-C₆alkyl, hetarylC₁-C₆alkyl, C₁-C₆alkyloxy, aryloxy, C₁-C₆alkyloxy, wherein the alkyl, aryl and hetaryl groups independently are optionally substituted with one or more of R⁹.
13. The use according to any of the clauses 1-12, wherein in formula (I) R¹² is C₁-C₆alkylcarbonylaminoC₁-C₆alkyl, arylcarbonylaminoC₁-C₆alkyl or arylC₁-C₆alkylcarbonylaminoC₁-C₆alkyl.
14. The use according to any of the clauses 1-13, wherein in formula (I) R¹³and R¹⁴ independently are hydrogen, C₃-C₁₀cycloalkyl, C₁-C₆alkyl, C₁-C₆alkylcarbonyl, arylcarbonyl, hetarylcarbonyl, arylC₁-C₆alkylcarbonyl.
15. The use according to any of the clauses 1-14, wherein in formula (I) R¹⁵ is hydrogen, C₃-C₁₀cycloalkyl, C₁-C₆alkyl, aryl, hetaryl, arylC₁-C₆alkyl, hetarylC₁-C₆alkyl, C₁-C₆alkyloxyalkyl or arylC₁-C₆alkyloxyalkyl.
16. The use according to any of the clauses 1-15, wherein the substituted pyrazolo[1,5-a]pyrimidine or a prodrug thereof is selected from the group consisting of:
   (3-Bromo-5-thiophen-2-yl-7-trifluoromethyl-pyrazolo[1,5-a]pyrimidin-2-yl)-(2,6-dimethylpiperidin-1-yl-)methanone;
   (3-Bromo-5-thiophen-2-yl-7-trifluoromethyl-pyrazolo[1,5-a]pyrimidin-2-yl)-(2-ethyl-piperidin-1-yl-)methanone;
   (5-Thiophen-2-yl-7-trifluoromethyl-pyrazolo[1,5-a]pyrimidin-2-yl)-(1,3,3-trimethyl-6-azabicyclo[3.2.1]oct-6-yl)methanone;
   (3-Chloro-5-thiophen-2-yl-7-trifluoromethyl-pyrazolo[1,5-a]pyrimidin-2-yl)-(2-methyl-piperidin-1-yl-)methanone;
   5-Methyl-7-phenyl-pyrazolo[1,5-a]pyrimidine-2-carboxylic acid cyclohexyl amide;
   Azepan-1-yl-(3-chloro-5-thiophen-2-yl-7-trifluoromethyl-pyrazolo[1,5-a]pyrimidin-2-yl)-methanone;
   Azepan-1-yl-(3-chloro-5-thiophen-2-yl-7-trifluoromethyl-pyrazolo[1,5-a]pyrimidin-3-yl)-methanone;
   (2,6-Dimethyl-piperidin-1-yl-)-(3-chloro-5-thiophen-2-yl-7-trifluoromethyl-pyrazolo[1,5-*a*]pyrimidin-3-yl)methanone;
   3-Bromo-5-furan-2-yl-7-trifluoromethyl-pyrazolo[1,5-*a*]pyrimidin-2-carboxylic acid cyclohexylamide;
   (7-Methyl-5-phenyl-pyrazolo[1,5-a]pyrimidin-3-yl)-(4-methyl-piperidin-1-yl)methanone;
   5-(-4-Methoxy-phenyl-7-trifluoromethyl-pyrazolo[1,5-a]pyrimidine-3-carboxylic acid benzyl-methyl amide;
   [5-(4-Ethoxy-phenyl)-7-methyl-pyrazolo[1,5-*a*]pyrimidine-2-yl]-piperidin-1-yl-methanone;
   Azepan-1-yl-(3-bromo-5-furan-2-yl-7-trifluoromethyl-pyrazolo[1,5-*a*]pyrimidin-2-yl)methanone;
   5-Benzo[1,3]dioxol-5-yl-7-trifluoromethyl-pyrazolo[1,5-*a*]pyrimidine-3-carboxylic acid cyclohexyl-methyl amide;
   (3,4-Dihydro-1 H-isoquinolin-2-yl)-[5-(-4-Methoxy-phenyl-7-trifluoromethyl-pyrazolo[1,5-a]pyrimidin-3-yl]methanone;
   Azepan-1-yl-[5-(4-bromo-phenyl)-7-trifluoromethyl-pyrazolo[1,5-a]pyrimidin-3-yl)methanone;
   Azepan-1-yl-(5,7-diphenyl)-pyrazolo[1,5-a]pyrimidin-3-yl)methanone;
   [5-(4-Bromo-phenyl)-7-trifluoromethyl-pyrazolo[1,5-*a*]pyrimidin-3-yl]-(2-methyl-piperidin-1-yl)-methanone;
   (5-Furan-2-yl-7-trifluoromethyl-pyrazolo[1,5-*a*]pyrimidin-3-yl)-(2-methyl-piperidin-1-yl)-methanone;
   (2,6-Dimethyl-piperidin-1-yl-)-[7-(4-ethoxy-phenyl)-5-methyl-pyrazolo[1,5-*a*]pyrimidin-2-yl] methanone;
   5-(4-Ethoxy-phenyl)-7-methyl-pyrazolo[1,5-a]pyrimidine-2-carboxylic acid cyclohexylamide;
   Piperidin-1-yl-(5-thiophen-2-yl-7-trifluoromethyl-pyrazolo[1,5-a]pyrimidin-3-yl)-methanone;
   (5-Furan-2-yl-7-trifluoromethyl-pyrazolo[1,5-a]pyrimidin-3-yl)-(2-methyl-piperidin-1-yl)-methanone;
   (2-Methyl-piperidin-1-yl)-(5-thiophen-2-yl-7-trifluoromethyl-pyrazolo[1,5-a]pyrimidin-3-yl)-methanone;
   Azepan-1-yl-[5-(4-methoxy-phenyl)-7-trifluoromethyl-pyrazolo[1,5-a]pyrimidin-3-yl]-methanone;
   5-Thiophen-2-yl -7-trifluoromethyl-pyrazolo[1,5-a]pyrimidine-3-carboxylic acid benzyl-methyl-amide;
   5-p-Tolyl-7-trifluoromethyl-pyrazolo[1,5-a]pyrimidine-3-carboxylic acid (1,3-dimethyl-1H-pyrazol-4-yl-methyl)methyl amide;
   5-Methyl-7-phenyl-pyrazolo[1,5-a]pyrimidine-2-carboxylic acid cyclohexyl-methyl amide; 7-Methyl-5-phenyl-pyrazolo[1,5-a]pyrimidine-2-carboxylic acid cyclohexyl-methyl amide;
   (5-Phenyl-7-trifluoromethyl-pyrazolo[1,5-a]pyrimidin-2-yl)-piperidin-1-yl-methanone;
   5-Naphthalen-1-yl-7-trifluoromethyl-pyrazolo[1,5-a]pyrimidine-2-carboxylic acid cyclohexyl-methyl amide;
   5-Naphthalen-1-yl-7-trifluoromethyl-pyrazolo[1,5-*a*]pyrimidine-2-carboxylic acid cyclohexyl amide;
   (5-Naphthalen-1-yl-7-trifluoromethyl-pyrazolo[1,5-a]pyrimidin-2-yl)-piperidin-1-yl-methanone
   (2-Methyl-piperidin-1-yl)-(5-naphthalen-1-yl-7-trifluoromethyl-pyrazolo[1,5-a]pyrimidin-2-yl)-methanone;
   5,7-Diphenyl-pyrazolo[1,5-a]pyrimidine-2-carboxylic acid cyclohexyl amide; 5,7-Diphenyl-pyrazolo[1,5-a]pyrimidine-2-carboxylic acid cyclohexyl-methyl amide;
   (5,7-Diphenyl-pyrazolo[1,5-a]pyrimidin-2-yl)-(2-methyl-piperidin-1-yl)methanone;
   (5,7-Diphenyl-pyrazolo[1,5-a]pyrimidin-2-yl)-piperidin-1-yl-methanone;
   5-Methyl-7-trifluoromethyl-pyrazolo[1,5-a]pyrimidine-2-carboxylic acid cyclohexyl amide;
   (5-Methyl-7-trifluoromethyl-pyrazolo[1,5-a]pyrimidin-2-yl)-piperidin-1-yl-methanone;
   5-Methyl-7-trifluoromethyl-pyrazolo[1,5-*a*]pyrimidine-2-carboxylic acid cyclohexyl-methyl-amide;
   (2-Methyl-piperidin-1-yl)-(5-methyl-7-trifluoromethyl-pyrazolo[1,5-a]pyrimidin-2-yl)-methanone;
   5-Phenyl-7-trifluoromethyl-pyrazolo[1,5-a!]pyrimidine-2-carboxylic acid cyclohexyl amide;
   (5-Phenyl-7-trifluoromethyl-pyrazolo[1,5-a]pyrimidin-2-yl)-piperidin-1-yl-methanone;
   7-Phenyl-5-trifluoromethyl-pyrazolo[1,5-a]pyrimidine-2-carboxylic acid cyclohexyl-methyl-amide;
   5-Phenyl-7-trifluoromethyl-pyrazolo[1,5-a]pyrimidine-3-carboxylic acid cyclohexyl amide;
   (5-Phenyl-7-trifluoromethyl-pyrazolo[1,5-a] pyrimidin-3-yl)-piperidin-1-yl-methanone; or
   a salt thereof with a pharmaceutically acceptable acid or base, or any optical isomer or mixture of optical isomers, including a racemic mixture, or any tautomeric forms.
17. The use according to any of the clauses 1-16, for the preparation of a pharmaceutical composition for the treatment, prevention and/or prophylaxis any conditions, disorders and diseases that are influenced by intracellular glucocorticoid levels.
18. The use according to any of the clauses 1-16 for the preparation of a pharmaceutical composition for the treatment, prevention and/or prophylaxis of conditions, disorders or diseases of the metabolic syndrome, insulin resistance, dyslipidemia, hypertension and obesity.
19. The use according to any of the clauses 1-16, for the preparation of a composition for the treatment, prevention and/or prophylaxis of type 2 diabetes, impaired glucose tolerance (IGT), impaired fasting glucose (IFG).
20. The use according to any of the clauses 1-16, for the preparation of a pharmaceutical composition for the delaying or prevention of the progression from IGT to type 2 diabetes.
21. The use according to any of the clauses 1-16, for the preparation of a pharmaceutical composition for the delaying or prevention of the progression of the metabolic syndrome into type 2 diabetes.
22. The use according to any of the clauses 1-16, for the preparation of a pharmaceutical composition for the treatment, prevention and/or prophylaxis of diabetic late complications including cardiovascular diseases; arteriosclerosis; atherosclerosis.
23. The use according to any of the clauses 1-16, for the preparation of a pharmaceutical composition for the treatment, prevention and/or prophylaxis of neurodegenerative and psychiatric disorders.
24. The use according to any of the clauses 1-16, for the preparation of a pharmaceutical composition for the treatment, prevention and/or prophylaxis of adverse effects of glucocorticoid receptor agonist treatment or therapy.
25. The use according to any of the clauses 1-24, wherein the pharmaceutical composition is for oral, nasal, buccal, transdermal, pulmonal or parenteral administration.
26. The use according to any of the clauses 1-25 wherein the pharmaceutical composition is preferably in unit dosage form, comprising from about 0.05 mg to about 2000 mg/day, preferably from about 0.1 mg to about 1000 mg and especially preferred from about 0.5 mg to about 500 mg per day of the compound.
27. A method for the treatment, prevention and/or prophylaxis of conditions, disorders or diseases wherein a modulation or an inhibition of the activity of 11 βHSD1 is beneficial, the method comprising administering to a subject in need thereof an effective amount of a compound according to the invention.
28. The method according to clause 27 wherein the treatment, prevention and/or prophylaxis is of any conditions, disorders and diseases that are influenced by intracellular glucocorticoid levels.
29. The method according to clause 27 or 28, wherein the condition, disorder or disease is the metabolic syndrome, insulin resistance, dyslipidemia, hypertension and obesity.
30. The method according to clause 27 or 28, wherein the condition, disorder or disease is type 2 diabetes, impaired glucose tolerance (IGT), impaired fasting glucose (IFG).
31. The method according to clause 27 or 28, for the delaying or prevention of the progression from IGT to type 2 diabetes.
32. The method according to clause 27 or 28, for the delaying or prevention of the progression of the metabolic syndrome into type 2 diabetes.
33. The method according to clause 27 or 28, wherein the condition, disorder or disease is diabetic late complications including cardiovascular diseases; arteriosclerosis; atherosclerosis.
34. The method according to clause 27 or 28, wherein the condition, disorder or disease is neurodegenerative and psychiatric disorders.
35. The method according to clause 27 or 28, for the treatment, prevention and/or prophylaxis of adverse effects of glucocorticoid receptor agonist treatment or therapy.
36. A substituted pyrazolo[1,5-a]pyrimidine or a prodrug thereof the general formula (I) which is:
   5-Methyl-7-trifluoromethyl-pyrazolo[1,5-a]pyrimidine-2-carboxylic acid methyl ester;
   5-Methyl-7-phenyl-pyrazolo[1,5-a]pyrimidine-2-carboxylic acid methyl ester;
   7-Methyl-5-phenyl-pyrazolo[1,5-a]pyrimidine-2-carboxylic acid methyl ester;
   (5-methyl-7-trifluoromethyl-pyrazolo[1,5-a]pyrimidin-2-yl)- (piperidin-1-yl)methanone;
   (5-methyl-7-phenyl-pyrazolo[1,5-a]pyrimidine-2-carboxylic acid cyclohexyl-methyl amide; or
   a salt thereof with a pharmaceutically acceptable acid or base, or any optical isomer or mixture of optical isomers, including a racemic mixture, or any tautomeric forms.
37. A substituted pyrazolo[1,5-a]pyrimidine or a prodrug thereof the general formula (I) which is:
   7-Methyl-5-phenyl-pyrazolo[1,5-a]pyrimidine-2-carboxylic acid cyclohexyl-methyl-amide;
   7-Phenyl-5-trifluoromethyl-pyrazolo[1,5-a]pyrimidine-2-carboxylic acid cyclohexyl-methyl-amide;
   (5-Phenyl-7-trifluoromethyl-pyrazolo[1,5-a]pyrimidin-2-yl)-piperidin-1-yl-methanone;
   (2-Methyl-piperidin-1-yl)-(5-methyl-7-trifluoromethyl-pyrazolo[1,5-a]pyrimidin-2-yl)-methanone;
   5-Methyl-7-trifluoromethyl-pyrazolo[1,5-a]pyrimidine-2-carboxylic acid cyclohexyl-methyl-amide;
   (5-Methyl-7-trifluoromethyl-pyrazolo[1,5-a]pyrimidin-2-yl)-piperidin-1-yl-methanone;
   (5,7-Diphenyl-pyrazolo[1,5-a]pyrimidin-2-yl)-piperidin-1-yl-methanone;
   (5,7-Diphenyl-pyrazolo[1,5-a]pyrimidin-2-yl)-(2-methyl-piperidin-1-yl)-methanone;
   5,7-Diphenyl-pyrazolo[1,5-a]pyrimidine-2-carboxylic acid cyclohexyl-methyl-amide;
   (2-Methyl-piperidin-1-yl)-(5-naphthalen-1-yl-7-trifluoromethyl-pyrazolo[1,5-a]pyrimidin-2-yl)-methanone;
   (5-Naphthalen-1-yl-7-trifluoromethyl-pyrazolo[1,5-a]pyrimidin-2-yl)-piperidin-1-yl-methanone;
   5-Naphthalen-1-yl-7-trifluoromethyl-pyrazolo[1,5-a]pyrimidine-2-carboxylic acid cyclohexyl-methyl-amide;
   [7-(4-Ethoxy-phenyl)-5-methyl-pyrazolo[1,5-a]pyrimidin-2-yl]-(1,3,3-trimethyl-6-azabicyclo[3.2.1]oct-6-yl)-methanone;
   (2-Methyl-piperidin-1-yl)-(7-phenyl-5-trifluoromethyl-pyrazolo[1,5-a]pyrimidin-2-yl)-methanone;
   (5-Methyl-7-trifluoromethyl-pyrazolo[1,5-a]pyrimidin-2-yl)-(1,3,3-trimethyl-6-azabicyclo[3.2.1]oct-6-yl)-methanone;
   (5-Phenyl-7-trifluoromethyl-pyrazolo[1,5-a]pyrimidin-2-yl)-(1,3,3-trimethyl-6-azabicyclo[3.2.1]oct-6-yl)-methanone;
   (6-Bromo-3-chloro-pyrazolo[1,5-a]pyrimidin-2-yl)-morpholin-4-yl-methanone;
   (5-Phenyl-7-trifluoromethyl-pyrazolo[1,5-a]pyrimidin-2-yl)-piperidin-1-yl-methanone;
   Morpholin-4-yl-(5-phenyl-7-trifluoromethyl-pyrazolo[1,5-a]pyrimidin-2-yl)-methanone;
   (6-Bromo-pyrazolo[1,5-a]pyrimidin-2-yl)-piperidin-1-yl-methanone;
   (6-Bromo-pyrazolo[1,5-a]pyrimidin-2-yl)-morpholin-4-yl-methanone;
   (3,6-Dibromo-pyrazolo[1,5-a]pyrimidin-2-yl)-morpholin-4-yl-methanone;
   (3-Bromo-5-phenyl-7-trifluoromethyl-pyrazolo[1,5-a]pyrimidin-2-yl)-piperidin-1-yl-methanone;
   (3-Bromo-5-phenyl-7-trifluoromethyl-pyrazolo[1,5-a]pyrimidin-2-yl)-morpholin-4-yl-methanone;
   (3-Chloro-5-phenyl-7-trifluoromethyl-pyrazolo[1,5-a]pyrimidin-2-yl)-piperidin-1-yl-methanone;
   (3-Chloro-5-phenyl-7-trifluoromethyl-pyrazolo[1,5-a]pyrimidin-2-yl)-morpholin-4-yl-methanone;
   (3,6-Dibromo-pyrazolo[1,5-a]pyrimidin-2-yl)-piperidin-1-yl-methanone;
   [4-(4-Fluoro-phenyl)-piperazin-1-yl]-(5-thiophen-2-yl-7-trifluoromethyl-pyrazolo[1,5-a]pyrimidin-2-yl)-methanone;
   5-Thiophen-2-yl-7-trifluoromethyl-pyrazolo[1,5-a]pyrimidine-2-carboxylic acid cyclohexyl-methyl-amide;
   (5-Benzo[1,3]dioxol-5-yl-7-trifluoromethyl-pyrazolo[1,5-a]pyrimidin-2-yl)-(4-methyl-piperazin-1-yl)-methanone;
   [5-(3,4-Dichloro-phenyl)-7-trifluoromethyl-pyrazolo[1,5-a]pyrimidin-2-yl]-(2-methyl-piperidin-1-yl)-methanone;
   3-Chloro-5-(4-methoxy-phenyl)-7-trifluoromethyl-pyrazolo[1,5-a]pyrimidine-2-carboxylic acid methyl-(2-pyridin-2-yl-ethyl)-amide;
   [3-Chloro-5-(4-chloro-phenyl)-7-trifluoromethyl-pyrazolo[1,5-a]pyrimidin-2-yl]-morpholin-4-yl-methanone;
   (3-Chloro-5-thiophen-2-yl-7-trifluoromethyl-pyrazolo[1,5-a]pyrimidin-2-yl)-(3,4-dihydro-2*H-*quinolin-1-yl)-methanone;
   [5-(3,4-Dichloro-phenyl)-7-trifluoromethyl-pyrazolo[1,5-a]pyrimidin-2-yl]-[4-(2-methoxyphenyl)-piperazin-1-yl]-methanone;
   (4-Benzoyl-piperazin-1-yl)-[3-chloro-5-(4-chloro-phenyl)-7-trifluoromethyl-pyrazolo[1,5-a]pyrimidin-2-yl]-methanone;
   4-(5-Benzo[1,3]dioxol-5-yl-7-trifluoromethyl-pyrazolo[1,5-a]pyrimidine-2-carbonyl)-piperazine-1-carboxylic acid ethyl ester;
   (5-Benzo[1,3]dioxol-5-yl-7-trifluoromethyl-pyrazolo[1,5-a]pyrimidin-2-yl)-(6,7-dimethoxy-1-methyl-3,4-dihydro-1H-isoquinolin-2-yl)-methanone;
   [4-(Furan-2-carbonyl)-piperazin-1-yl]-[5-(4-methoxy-phenyl)-7-trifluoromethyl-pyrazolo[1,5-a]pyrimidin-2-yl]-methanone;
   (3-Chloro-5-thiophen-2-yl-7-trifluoromethyl-pyrazolo[1,5-a]pyrimidin-2-yl)-[4-(2-methoxyphenyl)-piperazin-1-yl]-methanone;
   [5-(4-Methoxy-phenyl)-7-trifluoromethyl-pyrazolo[1,5-a]pyrimidin-2-yl]-piperidin-1-yl-methanone;
   (3,4-Dihydro-2*H*-quinolin-1-yl)-(5-phenyl-7-trifluoromethyl-pyrazolo[1,5-a]pyrimidin-2-yl)-methanone;
   (3-Bromo-5-thiophen-2-yl-7-trifluoromethyl-pyrazolo[1,5-a]pyrimidin-2-yl)-(2,6-dimethylpiperidin-1-yl)-methanone;
   (3-Bromo-5-thiophen-2-yl-7-trifluoromethyl-pyrazolo[1,5-a]pyrimidin-2-yl)-(2-ethyl-piperidin-1-yl)-methanone;
   3-Bromo-5-thiophen-2-yl-7-trifluoromethyl-pyrazolo[1,5-a]pyrimidine-2-carboxylic acid cyclohexyl-methyl-amide;
   [5-(4-Bromo-phenyl)-3-chloro-7-trifluoromethyl-pyrazolo[1,5-a]pyrimidin-2-yl]-pyrrolidin-1-yl-methanone;
   (3-Chloro-5-furan-2-yl-7-trifluoromethyl-pyrazolo[1,5-a]pyrimidin-2-yl)-[4-(4-fluoro-phenyl)-piperazin-1-yl]-methanone;
   (3-Chloro-5-furan-2-yl-7-trifluoromethyl-pyrazolo[1,5-a]pyrimidin-2-yl)-(4-phenyl-piperazin-1-yl)-methanone;
   [3-Chloro-5-(4-chloro-phenyl)-7-trifluoromethyl-pyrazolo[1,5-a]pyrimidin-2-yl]-(4-methylpiperazin-1-yl)-methanone;
   Azepan-1 -yl-[3-chloro-5-(4-chloro-phenyl)-7-trifluoromethyl-pyrazolo[1,5-a]pyrimidin-2-yl]-methanone;
   [3-Chloro-5-(3,4-dimethoxy-phenyl)-7-trifluoromethyl-pyrazolo[1,5-a]pyrimidin-2-yl]-pyrrolidin-1-yl-methanone;
   [5-(4-Chloro-phenyl)-7-trifluoromethyl-pyrazolo[1,5-a]pyrimidin-2-yl]-(2-methyl-piperidin-1-yl)-methanone;
   (5-Thiophen-2-yl-7-trifluoromethyl-pyrazolo[1,5-a]pyrimidin-2-yl)-(1,3,3-trimethyl-6-azabicyclo[3.2.1]oct-6-yl)-methanone;
   [4-(2-Chloro-phenyl)-piperazin-1-yl]-(5-thiophen-2-yl-7-trifluoromethyl-pyrazolo[1,5-a]pyrimidin-2-yl)-methanone;
   (3,4-Dihydro-1 H-isoquinolin-2-yl)-(5-thiophen-2-yl-7-trifluoromethyl-pyrazolo[1,5-a]pyrimidin-2-yl)-methanone;
   (4-Methyl-piperazin-1-yl)-[5-(4-nitro-phenyl)-7-trifluoromethyl-pyrazolo[1,5-a]pyrimidin-2-yl]-methanone;
   [5-(3,4-Dichloro-phenyl)-7-trifluoromethyl-pyrazolo[1,5-a]pyrimidin-2-yl]-thiomorpholin-4-yl-methanone;
   (3-Bromo-5-furan-2-yl-7-trifluoromethyl-pyrazolo[1,5-a]pyrimidin-2-yl)-(3,4-dihydro-2*H-*quinolin-1-yl)-methanone;
   (5-Furan-2-yl-7-trifluoromethyl-pyrazolo[1,5-a]pyrimidin-2-yl)-pyrrolidin-1-yl-methanone;
   [4-(Furan-2-carbonyl)-piperazin-1-yl]-[5-(4-methoxy-phenyl)-7-trifluoromethyl-pyrazolo[1,5-a]pyrimidin-2-yl]-methanone;
   [5-(4-Nitro-phenyl)-7-trifluoromethyl-pyrazolo[1,5-a]pyrimidin-2-yl]-piperidin-1-yl-methanone;
   (5-Furan-2-yl-7-trifluoromethyl-pyrazolo[1,5-a]pyrimidin-2-yl)-pyrrolidin-1-yl-methanone;
   (4-Methyl-piperidin-1-yl)-(5-phenyl-7-trifluoromethyl-pyrazolo[1,5-a]pyrimidin-2-yl)-methanone;
   (3-Bromo-5-thiophen-2-yl-7-trifluoromethyl-pyrazolo[1,5-a]pyrimidin-2-yl)-[4-(4-fluoro-phenyl)-piperazin-1-yl]-methanone;
   [4-(4-Fluoro-phenyl)-piperazin-1-yl]-(5-phenyl-7-trifluoromethyl-pyrazolo[1,5-a]pyrimidin-2-yl)-methanone;
   [4-(4-Fluoro-phenyl)-piperazin-1-yl]-(5-thiophen-2-yl-7-trifluoromethyl-pyrazolo[1,5-a]pyrimidin-2-yl)-methanone;
   [3-Bromo-5-(4-methoxy-phenyl)-7-trifluoromethyl-pyrazolo[1,5-a]pyrimidin-2-yl]-morpholin-4-yl-methanone;
   [4-(4-Fluoro-phenyl)-piperazin-1-yl]-(7-methyl-5-phenyl-pyrazolo[1,5-a]pyrimidin-2-yl)-methanone;
   (4-Benzoyl-piperazin-1-yl)-(5-phenyl-7-trifluoromethyl-pyrazolo[1,5-a]pyrimidin-2-yl)-methanone;
   (3-Chloro-5-thiophen-2-yl-7-trifluoromethyl-pyrazolo[1,5-a]pyrimidin-2-yl)-(4-phenyl-piperazin-1-yl)-methanone;
   (2-Ethyl-piperidin-1-yl)-(5-phenyl-7-trifluoromethyl-pyrazolo[1,5-a]pyrimidin-2-yl)-methanone;
   (5,7-Diphenyl-pyrazolo[1,5-a]pyrimidin-2-yl)-(4-methyl-piperazin-1-yl)-methanone;
   (4-Benzyl-piperazin-1-yl)-(3-chloro-5-phenyl-7-trifluoromethyl-pyrazolo[1,5-a]pyrimidin-2-yl)-methanone;
   (3-Chloro-5-thiophen-2-yl-7-trifluoromethyl-pyrazolo[1,5-a]pyrimidin-2-yl)-(4-methyl-piperazin-1-yl)-methanone;
   (4-Phenyl-piperazin-1-yl)-(5-thiophen-2-yl-7-trifluoromethyl-pyrazolo[1,5-a]pyrimidin-2-yl)-methanone;
   [5-(4-Bromo-phenyl)-7-trifluoromethyl-pyrazolo[1,5-a]pyrimidin-2-yl]-pyrrolidin-1-yl-methanone;
   (5,7-Diphenyl-pyrazolo[1,5-a]pyrimidin-2-yl)-pyrrolidin-1-yl-methanone;
   (3-Chloro-5-thiophen-2-yl-7-trifluoromethyl-pyrazolo[1,5-a]pyrimidin-2-yl)-(2-ethyl-piperidin-1-yl)-methanone;
   (3-Chloro-5-thiophen-2-yl-7-trifluoromethyl-pyrazolo[1,5-a]pyrimidin-2-yl)-(2-methyl-piperidin-1-yl)-methanone;
   5-Methyl-7-phenyl-pyrazolo[1,5-a]pyrimidine-2-carboxylic acid cyclohexyl-methyl-amide;
   (3-Chloro-5-thiophen-2-yl-7-trifluoromethyl-pyrazolo[1,5-a]pyrimidin-2-yl)-morpholin-4-yl-methanone;
   (3-Chloro-5-thiophen-2-yl-7-trifluoromethyl-pyrazolo[1,5-a]pyrimidin-2-yl)-[4-(4-fluoro-phenyl)-piperazin-1-yl]-methanone;
   (3-Bromo-5-thiophen-2-yl-7-trifluoromethyl-pyrazolo[1,5-a]pyrimidin-2-yl)-(4-methyl-piperazin-1-yl)-methanone;
   (7-Methyl-5-phenyl-pyrazolo[1,5-a]pyrimidin-2-yl)-piperidin-1-yl-methanone;
   Azepan-1-yl-(3-chloro-5-thiophen-2-yl-7-trifluoromethyl-pyrazolo[1,5-a]pyrimidin-2-yl)-methanone;
   Azepan-1-yl-[3-bromo-5-(4-bromo-phenyl)-7-trifluoromethyl-pyrazolo[1,5-a]pyrimidin-2-yl]-methanone;
   3-Bromo-5-(4-bromo-phenyl)-7-trifluoromethyl-pyrazolo[1,5-a]pyrimidine-2-carboxylic acid cyclohexyl-methyl-amide;
   (2,6-Dimethyl-piperidin-1-yl)-[5-(4-methoxy-phenyl)-7-trifluoromethyl-pyrazolo[1,5-a]pyrimidin-2-yl]-methanone;
   [5-(4-Bromo-phenyl)-7-trifluoromethyl-pyrazolo[1,5-a]pyrimidin-2-yl]-(4-methyl-piperazin-1-yl)-methanone;
   5-Phenyl-7-trifluoromethyl-pyrazolo[1,5-a]pyrimidine-2-carboxylic acid benzyl-methyl-amide;
   [5-(4-Ethoxy-phenyl)-7-methyl-pyrazolo[1,5-a]pyrimidin-2-yl]-(4-phenyl-piperazin-1-yl)-methanone;
   (3,4-Dihydro-1 *H*-isoquinolin-2-yl)-[5-(4-methoxy-phenyl)-7-trifluoromethyl-pyrazolo[1,5-a]pyrimidin-2-yl]-methanone;
   Morpholin-4-yl-[5-(4-nitro-phenyl)-7-trifluoromethyl-pyrazolo[1,5-a]pyrimidin-2-yl]-methanone;
   (Hexahydro-pyrrolo[1,2-a]pyrazin-2-yl)-(5-phenyl-7-trifluoromethyl-pyrazolo[1,5-a]pyrimidin-2-yl)-methanone;
   (Hexahydro-pyrrolo[1,2-a]pyrazin-2-yl)-(5-p-tolyl-7-trifluoromethyl-pyrazolo[1,5-a]pyrimidin-2-yl)-methanone;
   (Hexahydro-pyrrolo[1,2-a]pyrazin-2-yl)-(5-thiophen-2-yl-7-trifluoromethyl-pyrazolo[1,5-a]pyrimidin-2-yl)-methanone;
   (3-Chloro-5-phenyl-7-trifluoromethyl-pyrazolo[1,5-a]pyrimidin-2-yl)-(hexahydro-pyrrolo[1,2-a]pyrazin-2-yl)-methanone;
   [5-(4-Fluoro-phenyl)-7-methyl-pyrazolo[1,5-a]pyrimidin-2-yl]-morpholin-4-yl-methanone;
   (5,7-Diphenyl-pyrazolo[1,5-a]pyrimidin-2-yl)-(2-methyl-piperidin-1-yl)-methanone;
   5-Phenyl-7-trifluoromethyl-pyrazolo[1,5-a]pyrimidine-2-carboxylic acid (1,3-dimethyl-1 H-pyrazol-4-ylmethyl)-methyl-amide;
   5-Thiophen-2-yl-7-trifluoromethyl-pyrazolo[1,5-a]pyrimidine-2-carboxylic acid (1,5-dimethyl-1 H-pyrazol-4-ylmethyl)-methyl-amide;
   5-Thiophen-2-yl-7-trifluoromethyl-pyrazolo[1,5-a]pyrimidine-2-carboxylic acid methyl-(1,3,5-trimethyl-1 H-pyrazol-4-ylmethyl)-amide;
   3-Chloro-5-(4-methoxy-phenyl)-7-trifluoromethyl-pyrazolo[1,5-a]pyrimidine-2-carboxylic acid (1,3-dimethyl-1 H-pyrazol-4-ylmethyl)-methyl-amide;
   5-(4-Chloro-phenyl)-7-trifluoromethyl-pyrazolo[1,5-a]pyrimidine-2-carboxylic acid (1,3-dimethyl-1 H-pyrazol-4-ylmethyl)-methyl-amide;
   5-(3,4-Dimethoxy-phenyl)-7-trifluoromethyl-pyrazolo[1,5-a]pyrimidine-2-carboxylic acid (1,3-dimethyl-1 H-pyrazol-4-ylmethyl)-methyl-amide;
   (2,5-Dimethyl-piperidin-1-yl)-(5-phenyl-7-trifluoromethyl-pyrazolo[1,5-a]pyrimidin-2-yl)-methanone;
   5-Furan-2-yl-7-trifluoromethyl-pyrazolo[1,5-a]pyrimidine-2-carboxylic acid methyl-(1,3,5-trimethyl-1 *H*-pyrazol-4-ylmethyl)-amide;
   5-(4-Chloro-phenyl)-7-trifluoromethyl-pyrazolo[1,5-a]pyrimidine-2-carboxylic acid (1,5-dimethyl-1 *H*-pyrazol-4-ylmethyl)-methyl-amide;
   (2,4-Dimethyl-piperidin-1-yl)-(5-phenyl-7-trifluoromethyl-pyrazolo[1,5-a]pyrimidin-2-yl)-methanone;
   (3-Bromo-5-phenyl-7-trifluoromethyl-pyrazolo[1,5-a]pyrimidin-2-yl)-(hexahydro-pyrrolo[1,2-a]pyrazin-2-yl)-methanone;
   [5-(3,4-Dimethoxy-phenyl)-7-trifluoromethyl-pyrazolo[1,5-a]pyrimidin-2-yl]-(hexahydropyrrolo[1,2-a]pyrazin-2-yl)-methanone;
   (3-Chloro-5-p-tolyl-7-trifluoromethyl-pyrazolo[1,5-a]pyrimidin-2-yl)-(hexahydro-pyrrolo[1,2-a]pyrazin-2-yl)-methanone;
   (3-Bromo-5-furan-2-yl-7-trifluoromethyl-pyrazolo[1,5-a]pyrimidin-2-yl)-piperidin-1-yl-methanone;
   (3-Chloro-5-phenyl-7-trifluoromethyl-pyrazolo[1,5-a]pyrimidin-2-yl)-pyrrolidin-1-yl-methanone;
   (7-Methyl-5-phenyl-pyrazolo[1,5-a]pyrimidin-2-yl)-(4-methyl-piperidin-1-yl)-methanone;
   (4-Methyl-piperazin-1-yl)-(5-thiophen-2-yl-7-trifluoromethyl-pyrazolo[1,5-a]pyrimidin-2-yl)-methanone;
   [3-Bromo-5-(4-methoxy-phenyl)-7-trifluoromethyl-pyrazolo[1,5-a]pyrimidin-2-yl]-piperidin-1-yl-methanone;
   (4-Methyl-piperazin-1-yl)-(5-phenyl-7-trifluoromethyl-pyrazolo[1,5-a]pyrimidin-2-yl)-methanone;
   [4-(4-Fluoro-phenyl)-piperazin-1-yl]-(5-furan-2-yl-7-trifluoromethyl-pyrazolo[1,5-a]pyrimidin-2-yl)-methanone;
   [5-(4-Methoxy-phenyl)-7-trifluoromethyl-pyrazolo[1,5-a]pyrimidin-2-yl]-morpholin-4-yl-methanone;
   (3,4-Dihydro-2*H*-quinolin-1-yl)-(5-thiophen-2-yl-7-trifluoromethyl-pyrazolo[1,5-a]pyrimidin-2-yl)-methanone;
   (3-Bromo-5-thiophen-2-yl-7-trifluoromethyl-pyrazolo[1,5-a]pyrimidin-2-yl)-morpholin-4-yl-methanone;
   Azepan-1-yl-(3-chloro-5-phenyl-7-trifluoromethyl-pyrazolo[1,5-a]pyrimidin-2-yl)-methanone;
   (4-Benzoyl-piperazin-1-yl)-[5-(4-bromo-phenyl)-7-trifluoromethyl-pyrazolo[1,5-a]pyrimidin-2-yl]-methanone;
   (4-Benzyl-piperazin-1-yl)-[5-(4-bromo-phenyl)-3-chloro-7-trifluoromethyl-pyrazolo[1,5-a]pyrimidin-2-yl]-methanone;
   [5-(4-Bromo-phenyl)-3-chloro-7-trifluoromethyl-pyrazolo[1,5-a]pyrimidin-2-yl]-(4-methylpiperazin-1-yl)-methanone;
   Azepan-1-yl-[5-(4-bromo-phenyl)-7-trifluoromethyl-pyrazolo[1,5-a]pyrimidin-2-yl]-methanone;
   [3-Bromo-5-(4-bromo-phenyl)-7-trifluoromethyl-pyrazolo[1,5-a]pyrimidin-2-yl]-(3,4-dihydro-2H-quinolin-1-yl)-methanone;
   [5-(3,4-Dimethoxy-phenyl)-7-trifluoromethyl-pyrazolo[1,5-a]pyrimidin-2-yl]-morpholin-4-yl-methanone;
   (3-Bromo-5-furan-2-yl-7-trifluoromethyl-pyrazolo[1,5-a]pyrimidin-2-yl)-(4-methyl-piperazin-1-yl)-methanone;
   (4-Benzoyl-piperazin-1-yl)-(3-bromo-5-furan-2-yl-7-trifluoromethyl-pyrazolo[1,5-a]pyrimidin-2-yl)-methanone;
   (3-Bromo-5-furan-2-yl-7-trifluoromethyl-pyrazolo[1,5-a]pyrimidin-2-yl)-(3,4-dihydro-2H-quinolin-1-yl)-methanone;
   [5-(4-Bromo-phenyl)-7-trifluoromethyl-pyrazolo[1,5-a]pyrimidin-2-yl]-piperidin-1-yl-methanone;
   [5-(4-Ethoxy-phenyl)-7-methyl-pyrazolo[1,5-a]pyrimidin-2-yl]-piperidin-1-yl-methanone;
   [5-(4-Ethoxy-phenyl)-7-methyl-pyrazolo[1,5-a]pyrimidin-2-yl]-morpholin-4-yl-methanone;
   Azepan-1-yl-(3-bromo-5-furan-2-yl-7-trifluoromethyl-pyrazolo[1,5-a]pyrimidin-2-yl)-methanone;
   (3,4-Dihydro-1 H-isoquinolin-2-yl)-(5-thiophen-2-yl-7-trifluoromethyl-pyrazolo[1,5-a]pyrimidin-2-yl)-methanone;
   (4-Benzyl-piperazin-1-yl)-(3-bromo-5-furan-2-yl-7-trifluoromethyl-pyrazolo[1,5-a]pyrimidin-2-yl)-methanone;
   Azepan-1-yl-[5-(4-bromo-phenyl)-3-chloro-7-trifluoromethyl-pyrazolo[1,5-a]pyrimidin-2-yl]-methanone;
   (3-Chloro-5-thiophen-2-yl-7-trifluoromethyl-pyrazolo[1,5-a]pyrimidin-2-yl)-(3,4-dihydro-1 H-isoquinolin-2-yl)-methanone;
   (5-Benzo[1,3]dioxol-5-yl-7-trifluoromethyl-pyrazolo[1,5-a]pyrimidin-2-yl)-piperidin-1-yl-methanone;
   Azepan-1-yl-(5,7-diphenyl-pyrazolo[1,5-a]pyrimidin-2-yl)-methanone;
   Azepan-1-yl-(5-benzo[1,3]dioxol-5-yl-7-trifluoromethyl-pyrazolo[1,5-a]pyrimidin-2-yl)-methanone;
   [5-(4-Bromo-phenyl)-7-trifluoromethyl-pyrazolo[1,5-a]pyrimidin-2-yl]-morpholin-4-yl-methanone;
   [3-Bromo-5-(4-bromo-phenyl)-7-trifluoromethyl-pyrazolo[1,5-a]pyrimidin-2-yl]-morpholin-4-yl-methanone;
   [5-(4-Methoxy-phenyl)-7-trifluoromethyl-pyrazolo[1,5-a]pyrimidin-2-yl]-thiomorpholin-4-yl-methanone;
   [5-(4-Bromo-phenyl)-3-chloro-7-trifluoromethyl-pyrazolo[1,5-a]pyrimidin-2-yl]-piperidin-1-yl-methanone;
   (5-Benzo[1,3]dioxol-5-yl-7-trifluoromethyl-pyrazolo[1,5-a]pyrimidin-2-yl)-(3,4-dihydro-2H-quinolin-1-yl)-methanone;
   (5-Benzo[1,3]dioxol-5-yl-7-trifluoromethyl-pyrazolo[1,5-a]pyrimidin-2-yl)-(4-benzyl-piperazin-1-yl)-methanone;
   (5-Benzo[1,3]dioxol-5-yl-7-trifluoromethyl-pyrazolo[1,5-a]pyrimidin-2-yl)-morpholin-4-yl-methanone;
   [5-(4-Bromo-phenyl)-7-trifluoromethyl-pyrazolo[1,5-a]pyrimidin-2-yl]-[4-(2-methoxy-phenyl)-piperazin-1-yl]-methanone;
   [3-Bromo-5-(4-bromo-phenyl)-7-trifluoromethyl-pyrazolo[1,5-a]pyrimidin-2-yl]-(4-methylpiperazin-1-yl)-methanone;
   Azepan-1-yl-[5-(4-methoxy-phenyl)-7-trifluoromethyl-pyrazolo[1,5-a]pyrimidin-2-yl]-methanone;
   (2,6-Dimethyl-piperidin-1-yl)-[7-(4-ethoxy-phenyl)-5-methyl-pyrazolo[1,5-a]pyrimidin-2-yl]-methanone;
   (3,4-Dihydro-2*H*-quinolin-1-yl)-(5,7-diphenyl-pyrazolo[1,5-a]pyrimidin-2-yl)-methanone;
   (3,6-Dibromo-pyrazolo[1,5-a]pyrimidin-2-yl)-piperidin-1-yl-methanone;
   (3-Bromo-5-furan-2-yl-7-trifluoromethyl-pyrazolo[1,5-a]pyrimidin-2-yl)-piperidin-1-yl-methanone;
   [5-(4-Methoxy-phenyl)-7-trifluoromethyl-pyrazolo[1,5-a]pyrimidin-2-yl]-thiomorpholin-4-yl-methanone;
   [5-(3,4-Dichloro-phenyl)-7-trifluoromethyl-pyrazolo[1,5-a]pyrimidin-2-yl]-thiomorpholin-4-yl-methanone;
   (3,4-Dihydro-1 H-isoquinolin-2-yl)-[5-(4-nitro-phenyl)-7-trifluoromethyl-pyrazolo[1,5-a]pyrimidin-2-yl]-methanone;
   Azepan-1-yl-[5-(4-methoxy-phenyl)-7-trifluoromethyl-pyrazolo[1,5-a]pyrimidin-2-yl]-methanone;
   Azepan-1-yl-[5-(4-nitro-phenyl)-7-trifluoromethyl-pyrazolo[1,5-a]pyrimidin-2-yl]-methanone;
   [5-(3,4-Dichloro-phenyl)-7-trifluoromethyl-pyrazolo[1,5-a]pyrimidin-2-yl]-morpholin-4-yl-methanone;
   Azepan-1-yl-[5-(3,4-dichloro-phenyl)-7-trifluoromethyl-pyrazolo[1,5-a]pyrimidin-2-yl]-methanone;
   (3,4-Dihydro-1 H-isoquinolin-2-yl)-(5-phenyl-7-trifluoromethyl-pyrazolo[1,5-a]pyrimidin-2-yl)-methanone;
   [5-(4-Bromo-phenyl)-3-chloro-7-trifluoromethyl-pyrazolo[1,5-a]pyrimidin-2-yl]-pyrrolidin-1-yl-methanone;
   5,7-Diphenyl-pyrazolo[1,5-a]pyrimidine-2-carboxylic acid cyclohexyl-methyl-amide;
   (3,4-Dihydro-1 *H*-isoquinolin-2-yl)-(5-furan-2-yl-7-trifluoromethyl-pyrazolo[1,5-a]pyrimidin-2-yl)-methanone;
   (4-Benzyl-piperazin-1-yl)-(3-chloro-5-furan-2-yl-7-trifluoromethyl-pyrazolo[1,5-a]pyrimidin-2-yl)-methanone;
   [3-Chloro-5-(4-methoxy-phenyl)-7-trifluoromethyl-pyrazolo[1,5-a]pyrimidin-2-yl]-piperidin-1-yl-methanone;
   (3-Chloro-5-*p*-tolyl-7-trifluoromethyl-pyrazolo[1,5-a]pyrimidin-2-yl)-(4-methyl-piperazin-1-yl)-methanone;
   (3-Chloro-5-*p*-tolyl-7-trifluoromethyl-pyrazolo[1,5-a]pyrimidin-2-yl)-(4-phenyl-piperazin-1-yl)-methanone;
   [3-Chloro-5-(3,4-dimethoxy-phenyl)-7-trifluoromethyl-pyrazolo[1,5-a]pyrimidin-2-yl]-piperidin-1-yl-methanone;
   [4-(4-Fluoro-phenyl)-piperazin-1-yl]-(5-p-tolyl-7-trifluoromethyl-pyrazolo[1,5-a]pyrimidin-2-yl)-methanone;
   (4-Phenyl-piperazin-1-yl)-(5-p-tolyl-7-trifluoromethyl-pyrazolo[1,5-a]pyrimidin-2-yl)-methanone;
   Morpholin-4-yl-(5-*p*-tolyl-7-trifluoromethyl-pyrazolo[1,5-a]pyrimidin-2-yl)-methanone;
   (4-Benzyl-piperazin-1-yl)-(5-p-tolyl-7-trifluoromethyl-pyrazolo[1,5-a]pyrimidin-2-yl)-methanone;
   [5-(4-Chloro-phenyl)-7-trifluoromethyl-pyrazolo[1,5-a]pyrimidin-2-yl]-(4-methyl-piperazin-1-yl)-methanone;
   [5-(4-Chloro-phenyl)-7-trifluoromethyl-pyrazolo[1,5-a]pyrimidin-2-yl]-morpholin-4-yl-methanone;
   (4-Benzyl-piperazin-1-yl)-[5-(4-chloro-phenyl)-7-trifluoromethyl-pyrazolo[1,5-a]pyrimidin-2-yl]-methanone;
   [5-(4-Fluoro-phenyl)-7-trifluoromethyl-pyrazolo[1,5-a]pyrimidin-2-yl]-morpholin-4-yl-methanone;
   [5-(4-Fluoro-phenyl)-7-trifluoromethyl-pyrazolo[1,5-a]pyrimidin-2-yl]-piperidin-1-yl-methanone;
   (3,4-Dihydro-2H-quinolin-1-yl)-[5-(4-fluoro-phenyl)-7-trifluoromethyl-pyrazolo[1,5-a]pyrimidin-2-yl]-methanone;
   Azepan-1-yl-[5-(3,4-dimethoxy-phenyl)-7-trifluoromethyl-pyrazolo[1,5-a]pyrimidin-2-yl]-methanone;
   (4-Benzyl-piperazin-1-yl)-[5-(4-methoxy-phenyl)-7-trifluoromethyl-pyrazolo[1,5-a]pyrimidin-2-yl]-methanone;
   [5-(4-Methoxy-phenyl)-7-trifluoromethyl-pyrazolo[1,5-a]pyrimidin-2-yl]-pyrrolidin-1-yl-methanone;
   (5-Benzo[1,3]dioxol-5-yl-7-trifluoromethyl-pyrazolo[1,5-a]pyrimidin-2-yl)-pyrrolidin-1 -yl-methanone;
   (3,4-Dihydro-2H-quinolin-1-yl)-[5-(4-nitro-phenyl)-7-trifluoromethyl-pyrazolo[1,5-a]pyrimidin-2-yl]-methanone;
   [3-Chloro-5-(4-methoxy-phenyl)-7-trifluoromethyl-pyrazolo[1,5-a]pyrimidin-2-yl]-(4-methylpiperidin-1-yl)-methanone;
   [3-Chloro-5-(4-methoxy-phenyl)-7-trifluoromethyl-pyrazolo[1,5-a]pyrimidin-2-yl]-(3,4-dihydro-2H-quinolin-1-yl)-methanone;
   3-Chloro-5-(4-methoxy-phenyl)-7-trifluoromethyl-pyrazolo[1,5-a]pyrimidine-2-carboxylic acid cyclohexyl-methyl-amide;
   [3-Chloro-5-(4-fluoro-phenyl)-7-trifluoromethyl-pyrazolo[1,5-a]pyrimidin-2-yl]-(4-methylpiperazin-1-yl)-methanone;
   (3-Chloro-5-p-tolyl-7-trifluoromethyl-pyrazolo[1,5-a]pyrimidin-2-yl)-morpholin-4-yl-methanone;
   Azepan-1-yl-(3-chloro-5-p-tolyl-7-trifluoromethyl-pyrazolo[1,5-a]pyrimidin-2-yl)-methanone;
   Azepan-1-yl-[3-chloro-5-(3,4-dimethoxy-phenyl)-7-trifluoromethyl-pyrazolo[1,5-a]pyrimidin-2-yl]-methanone;
   [5-(3,4-Dichloro-phenyl)-7-trifluoromethyl-pyrazolo[1,5-a]pyrimidin-2-yl]-(4-methyl-piperazin-1-yl)-methanone;
   5,7-Diphenyl-pyrazolo[1,5-a]pyrimidine-2-carboxylic acid benzyl-methyl-amide;
   (5,7-Diphenyl-pyrazolo[1,5-a]pyrimidin-2-yl)-(4-phenyl-piperazin-1-yl)-methanone;
   Morpholin-4-yl-(5-phenyl-7-trifluoromethyl-pyrazolo[1,5-a]pyrimidin-2-yl)-methanone;
   Morpholin-4-yl-(5-thiophen-2-yl-7-trifluoromethyl-pyrazolo[1,5-a]pyrimidin-2-yl)-methanone;
   Piperidin-1-yl-(5-thiophen-2-yl-7-trifluoromethyl-pyrazolo[1,5-a]pyrimidin-2-yl)-methanone;
   [3-Bromo-5-(4-bromo-thiophen-2-yl)-7-trifluoromethyl-pyrazolo[1,5-a]pyrimidin-2-yl]-piperidin-1-yl-methanone;
   (3-Bromo-5-thiophen-2-yl-7-trifluoromethyl-pyrazolo[1,5-a]pyrimidin-2-yl)-(3,4-dihydro-2*H-*quinolin-1-yl)-methanone;
   (3-Chloro-5-thiophen-2-yl-7-trifluoromethyl-pyrazolo[1,5-a]pyrimidin-2-yl)-piperidin-1-yl-methanone;
   (7-Methyl-5-phenyl-pyrazolo[1,5-a]pyrimidin-2-yl)-morpholin-4-yl-methanone;
   (7-Methyl-5-phenyl-pyrazolo[1,5-a]pyrimidin-2-yl)-pyrrolidin-1-yl-methanone;
   7-Methyl-5-phenyl-pyrazolo[1,5-a]pyrimidine-2-carboxylic acid benzyl-methyl-amide;
   (2,6-Dimethyl-piperidin-1-yl)-[5-(4-fluoro-phenyl)-7-trifluoromethyl-pyrazolo[1,5-a]pyrimidin-2-yl]-methanone;
   Azepan-1-yl-[5-(4-fluoro-phenyl)-7-trifluoromethyl-pyrazolo[1,5-a]pyrimidin-2-yl]-methanone;
   [5-(4-Fluoro-phenyl)-7-trifluoromethyl-pyrazolo[1,5-a]pyrimidin-2-yl]-(4-methyl-piperazin-1-yl)-methanone;
   [5-(4-Fluoro-phenyl)-7-trifluoromethyl-pyrazolo[1,5-a]pyrimidin-2-yl]-(4-phenyl-piperazin-1-yl)-methanone;
   (2-Ethyl-piperidin-1-yl)-[5-(4-fluoro-phenyl)-7-trifluoromethyl-pyrazolo[1,5-a]pyrimidin-2-yl]-methanone;
   (5-Phenyl-7-trifluoromethyl-pyrazolo[1,5-a]pyrimidin-3-yl)-piperidin-1-yl-methanone;
   (5-Methyl-7-phenyl-pyrazolo[1,5-a]pyrimidin-3-yl)-(1,3,3-trimethyl-6-aza-bicyclo[3.2.1]oct-6-yl)-methanone;
   (5-Thiophen-2-yl-7-trifluoromethyl-pyrazolo[1,5-a]pyrimidin-3-yl)-(1,3,3-trimethyl-6-azabicyclo[3.2.1]oct-6-yl)-methanone;
   (4-Phenyl-piperazin-1-yl)-pyrazolo[1,5-a]pyrimidin-3-yl-methanone;
   Pyrazolo[1,5-a]pyrimidine-3-carboxylic acid benzyl-(2-hydroxy-ethyl)-amide;
   [5-(4-Bromo-phenyl)-7-trifluoromethyl-pyrazolo[1,5-a]pyrimidin-3-yl]-(3,4-dihydro-1 H-isoquinolin-2-yl)-methanone;
   (5,7-Dimethyl-pyrazolo[1,5-a]pyrimidin-3-yl)-[4-(4-methoxy-phenyl)-piperazin-1-yl]-methanone;
   (3,4-Dihydro-2H-quinolin-1-yl)-[5-(4-fluoro-phenyl)-7-trifluoromethyl-pyrazolo[1,5-a]pyrimidin-3-yl]-methanone;
   (5-Furan-2-yl-7-trifluoromethyl-pyrazolo[1,5-a]pyrimidin-3-yl)-piperidin-1-yl-methanone;
   Azepan-1-yl-[7-trifluoromethyl-5-(3,4,5-trimethoxy-phenyl)-pyrazolo[1,5-a]pyrimidin-3-yl]-methanone;
   (5-Benzo[1,3]dioxol-5-yl-7-trifluoromethyl-pyrazolo[1 ,5-a]pyrimidin-3-yl)-pyrrolidin-1-yl-methanone;
   Azepan-1-yl-(5,7-diphenyl-pyrazolo[1,5-a]pyrimidin-3-yl)-methanone;
   4-(5,7-Dimethyl-pyrazolo[1,5-a]pyrimidine-3-carbonyl)-piperazine-1-carboxylic acid ethyl ester;
   (3,4-Dihydro-1 H-isoquinolin-2-yl)-(5,7-dimethyl-pyrazolo[1,5-a]pyrimidin-3-yl)-methanone;
   [4-(3-Chloro-phenyl)-piperazin-1-yl]-(5-p-tolyl-7-trifluoromethyl-pyrazolo[1,5-a]pyrimidin-3-yl)-methanone;
   (4-Methyl-piperidin-1-yl)-pyrazolo[1,5-a]pyrimidin-3-yl-methanone;
   [4-(2-Methoxy-phenyl)-piperazin-1-yl]-pyrazolo[1,5-a]pyrimidin-3-yl-methanone;
   (3,4-Dihydro-2H-quinolin-1-yl)-[5-(4-methoxy-phenyl)-7-trifluoromethyl-pyrazolo[1,5-a]pyrimidin-3-yl]-methanone;
   [5-(4-Methoxy-phenyl)-7-trifluoromethyl-pyrazolo[1,5-a]pyrimidin-3-yl]-thiomorpholin-4-yl-methanone;
   (5-Furan-2-yl-7-trifluoromethyl-pyrazolo[1,5-a]pyrimidin-3-yl)-(2-methyl-piperidin-1-yl)-methanone;
   (4-Benzyl-piperazin-1-yl)-(5,7-dimethyl-pyrazolo[1,5-a]pyrimidin-3-yl)-methanone;
   [5-(4-Bromo-phenyl)-7-trifluoromethyl-pyrazolo[1,5-a]pyrimidin-3-yl]-morpholin-4-yl-methanone;
   (5,7-Dimethyl-pyrazolo[1,5-a]pyrimidin-3-yl)-piperidin-1-yl-methanone;
   (5-Furan-2-yl-7-trifluoromethyl-pyrazolo[1,5-a]pyrimidin-3-yl)-[4-(2-methoxy-phenyl)-piperazin-1-yl]-methanone;
   (5-Phenyl-7-trifluoromethyl-pyrazolo[1,5-a]pyrimidin-3-yl)-piperidin-1-yl-methanone;
   Azepan-1-yl-(5-thiophen-2-yl-7-trifluoromethyl-pyrazolo[1,5-a]pyrimidin-3-yl)-methanone;
   (2,6-Dimethyl-piperidin-1-yl)-(5-thiophen-2-yl-7-trifluoromethyl-pyrazolo[1,5-a]pyrimidin-3-yl)-methanone;
   [5-(4-Methoxy-phenyl)-7-trifluoromethyl-pyrazolo[1,5-a]pyrimidin-3-yl]-(4-methyl-piperazin-1-yl)-methanone;
   (4-Methyl-piperazin-1-yl)-(5-thiophen-2-yl-7-trifluoromethyl-pyrazolo[1,5-a]pyrimidin-3-yl)-methanone;
   [5-(4-Bromo-phenyl)-7-trifluoromethyl-pyrazolo[1,5-a]pyrimidin-3-yl]-(4-methyl-piperazin-1-yl)-methanone;
   (Hexahydro-pyrrolo[1,2-a]pyrazin-2-yl)-(5-p-tolyl-7-trifluoromethyl-pyrazolo[1,5-a]pyrimidin-3-yl)-methanone;
   [5-(4-Bromo-phenyl)-7-trifluoromethyl-pyrazolo[1,5-a]pyrimidin-3-yl]-(hexahydro-pyrrolo[1,2-a]pyrazin-2-yl)-methanone;
   [5-(3,4-Dimethoxy-phenyl)-7-trifluoromethyl-pyrazolo[1,5-a]pyrimidin-3-yl]-(hexahydropyrrolo[1,2-a]pyrazin-2-yl)-methanone;
   1-[5-(3-Methoxy-phenyl)-7-trifluoromethyl-pyrazolo[1,5-a]pyrimidine-3-carbonyl]-piperidine-4-carboxylic acid ethyl ester;
   4-[5-(3-Methoxy-phenyl)-7-trifluoromethyl-pyrazolo[1,5-a]pyrimidine-3-carbonyl]-piperazine-1-carboxylic acid ethyl ester;
   (3,4-Dihydro-2H-quinolin-1-yl)-[5-(3-methoxy-phenyl)-7-trifluoromethyl-pyrazolo[1,5-a]pyrimidin-3-yl]-methanone;
   [4-(4-Fluoro-phenyl)-piperazin-1-yl]-[5-(3-methoxy-phenyl)-7-trifluoromethyl-pyrazolo[1,5-a]pyrimidin-3-yl]-methanone;
   5-p-Tolyl-7-trifluoromethyl-pyrazolo[1,5-a]pyrimidine-3-carboxylic acid (1,3-dimethyl-1H-pyrazol-4-ylmethyl)-methyl-amide;
   5-p-Tolyl-7-trifluoromethyl-pyrazolo[1,5-a]pyrimidine-3-carboxylic acid methyl-(1 ,3,5-trimethyl-1 H-pyrazol-4-ylmethyl)-amide;
   [7-Difluoromethyl-5-(4-methoxy-phenyl)-pyrazolo[1,5-a]pyrimidin-3-yl]-(2-ethyl-imidazol-1-yl)-methanone;
   [7-Difluoromethyl-5-(4-methoxy-phenyl)-pyrazolo[1,5-a]pyrimidin-3-yl]-morpholin-4-yl-methanone;
   1-[7-Difluoromethyl-5-(4-methoxy-phenyl)-pyrazolo[1,5-a]pyrimidine-3-carbonyl]-1*H*-indole-3-carboxylic acid methyl ester;
   [4-(4-Chloro-phenyl)-piperazin-1-yl]-[7-difluoromethyl-5-(4-methoxy-phenyl)-pyrazolo[1,5-a]pyrimidin-3-yl]-methanone;
   [7-Difluoromethyl-5-(4-methoxy-phenyl)-pyrazolo[1,5-a]pyrimidin-3-yl]-(2-ethyl-piperidin-1-yl)-methanone;
   7-Difluoromethyl-5-p-tolyl-pyrazolo[1,5-a]pyrimidine-3-carboxylic acid (1-ethyl-5-methyl-1*H-*pyrazol-4-ylmethyl)-methyl-amide;
   1-(7-Difluoromethyl-5-methyl-pyrazolo[1,5-a]pyrimidine-3-carbonyl)-piperidine-4-carboxylic acid amide;
   (7-Difluoromethyl-5-phenyl-pyrazolo[1,5-a]pyrimidin-3-yl)-(2-ethyl-imidazol-1-yl)-methanone;
   (7-Difluoromethyl-5-phenyl-pyrazolo[1,5-a]pyrimidin-3-yl)-thiomorpholin-4-yl-methanone;
   (7-Difluoromethyl-5-*p*-tolyl-pyrazolo[1,5-a]pyrimidin-3-yl)-(4-pyridin-2-yl-piperazin-1-yl)-methanone;
   [7-Difluoromethyl-5-(4-methoxy-phenyl)-pyrazolo[1,5-a]pyrimidin-3-yl]-[4-(furan-2-carbonyl)-piperazin-1-yl]-methanone;
   7-Difluoromethyl-5-phenyl-pyrazolo[1,5-a]pyrimidine-3-carboxylic acid (1-ethyl-3-methyl-1*H-*pyrazol-4-ylmethyl)-methyl-amide;
   [7-Difluoromethyl-5-(4-methoxy-phenyl)-pyrazolo[1,5-a]pyrimidin-3-yl]-thiomorpholin-4-yl-methanone;
   (7-Difluoromethyl-5-phenyl-pyrazolo[1,5-a]pyrimidin-3-yl)-(3,4-dihydro-1 H-isoquinolin-2-yl)-methanone;
   (7-Difluoromethyl-5-methyl-pyrazolo[1,5-a]pyrimidin-3-yl)-piperidin-1-yl-methanone;
   Azepan-1-yl-(7-difluoromethyl-5-phenyl-pyrazolo[1,5-a]pyrimidin-3-yl)-methanone;
   7-Difluoromethyl-5-p-tolyl-pyrazolo[1,5-a]pyrimidine-3-carboxylic acid methyl-(1-methyl-1H-pyrazol-4-ylmethyl)-amide;
   (7-Difluoromethyl-5-*p*-tolyl-pyrazolo[1,5-a]pyrimidin-3-yl)-(3-methyl-piperidin-1-yl)-methanone;
   (7-Difluoromethyl-5-*p*-tolyl-pyrazolo[1,5-a]pyrimidin-3-yl)-(4-ethyl-piperazin-1-yl)-methanone;
   (7-Difluoromethyl-5-*p*-tolyl-pyrazolo[1,5-a]pyrimidin-3-yl)-morpholin-4-yl-methanone;
   7-Difluoromethyl-5-phenyl-pyrazolo[1,5-a]pyrimidine-3-carboxylic acid methyl-(1,3,5-trimethyl-1 H-pyrazol-4-ylmethyl)-amide;
   (7-Difluoromethyl-5-phenyl-pyrazolo[1,5-a]pyrimidin-3-yl)-[4-(2-methyl-2H-pyrazole-3-carbonyl)-piperazin-1-yl]-methanone;
   7-Difluoromethyl-5-p-tolyl-pyrazolo[1,5-a]pyrimidine-3-carboxylic acid benzyl-methyl-amide;
   7-Difluoromethyl-5-(4-methoxy-phenyl)-pyrazolo[1,5-a]pyrimidine-3-carboxylic acid (1-ethyl-3-methyl-1 H-pyrazol-4-ylmethyl)-methyl-amide;
   7-Difluoromethyl-5-phenyl-pyrazolo[1,5-a]pyrimidine-3-carboxylic acid methyl-(4-pyrazol-1-yl-benzyl)-amide;
   Azepan-1-yl-(5-cyclopropyl-7-difluoromethyl-pyrazolo[1,5-a]pyrimidin-3-yl)-methanone;
   (4-Benzyl-piperazin-1-yl)-[5-(4-methoxy-phenyl)-7-trifluoromethyl-pyrazolo[1,5-a]pyrimidin-3-yl]-methanone;
   (3,4-Dihydro-2*H*-quinolin-1-yl)-[5-(4-methoxy-phenyl)-7-trifluoromethyl-pyrazolo[1,5-a]pyrimidin-3-yl]-methanone;
   5-(4-Methoxy-phenyl)-7-trifluoromethyl-pyrazolo[1,5-a]pyrimidine-3-carboxylic acid benzyl-methyl-amide;
   (5-Furan-2-yl-7-trifluoromethyl-pyrazolo[1,5-a]pyrimidin-3-yl)-(4-methyl-piperazin-1-yl)-methanone;
   (5-Furan-2-yl-7-trifluoromethyl-pyrazolo[1,5-a]pyrimidin-3-yl)-thiomorpholin-4-yl-methanone;
   [5-(4-Methoxy-phenyl)-7-trifluoromethyl-pyrazolo[1,5-a]pyrimidin-3-yl]-morpholin-4-yl-methanone;
   (4-Benzyl-piperazin-1-yl)-(5-thiophen-2-yl-7-trifluoromethyl-pyrazolo[1,5-a]pyrimidin-3-yl)-methanone;
   [5-(4-Bromo-phenyl)-7-trifluoromethyl-pyrazolo[1,5-a]pyrimidin-3-yl]-morpholin-4-yl-methanone;
   Azepan-1-yl-(5-benzo[1,3]dioxol-5-yl-7-trifluoromethyl-pyrazolo[1,5-a]pyrimidin-3-yl)-methanone;
   (5-Benzo[1,3]dioxol-5-yl-7-trifluoromethyl-pyrazolo[1,5-a]pyrimidin-3-yl)-(3,4-dihydro-2H-quinolin-1-yl)-methanone;
   5-Benzo[1,3]dioxol-5-yl-7-trifluoromethyl-pyrazolo[1,5-a]pyrimidine-3-carboxylic acid cyclohexyl-methyl-amide;
   [5-(4-Bromo-phenyl)-7-trifluoromethyl-pyrazolo[1,5-a]pyrimidin-3-yl]-(3,4-dihydro-2H-quinolin-1-yl)-methanone;
   [5-(4-Bromo-phenyl)-7-trifluoromethyl-pyrazolo[1,5-a]pyrimidin-3-yl]-piperidin-1-yl-methanone;
   Azepan-1-yl-(5-furan-2-yl-7-trifluoromethyl-pyrazolo[1,5-a]pyrimidin-3-yl)-methanone;
   (5-Benzo[1,3]dioxol-5-yl-7-trifluoromethyl-pyrazolo[1,5-a]pyrimidin-3-yl)-pyrrolidin-1 -yl-methanone;
   (5-Benzo[1,3]dioxol-5-yl-7-trifluoromethyl-pyrazolo[1,5-a]pyrimidin-3-yl)-(4-methyl-piperazin-1-yl)-methanone;
   (3,4-Dihydro-1*H*-isoquinolin-2-yl)-[5-(4-methoxy-phenyl)-7-trifluoromethyl-pyrazolo[1,5-a]pyrimidin-3-yl]-methanone;
   Azepan-1-yl-[5-(4-bromo-phenyl)-7-trifluoromethyl-pyrazolo[1,5-a]pyrimidin-3-yl]-methanone;
   (3,4-Dihydro-2H-quinolin-1-yl)-(5,7-diphenyl-pyrazolo[1,5-a]pyrimidin-3-yl)-methanone;
   Azepan-1-yl-(5,7-diphenyl-pyrazolo[1,5-a]pyrimidin-3-yl)-methanone;
   (5-Benzo[1,3]dioxol-5-yl-7-trifluoromethyl-pyrazolo[1,5-a]pyrimidin-3-yl)-(4-benzyl-piperazin-1-yl)-methanone;
   (4-Benzyl-piperazin-1-yl)-(5,7-diphenyl-pyrazolo[1,5-a]pyrimidin-3-yl)-methanone;
   (5,7-Diphenyl-pyrazolo[1,5-a]pyrimidin-3-yl)-(4-methyl-piperazin-1-yl)-methanone;
   [5-(4-Bromo-phenyl)-7-trifluoromethyl-pyrazolo[1,5-a]pyrimidin-3-yl]-(2-methyl-piperidin-1-yl)-methanone;
   (3,4-Dihydro-2H-quinolin-1-yl)-(5-furan-2-yl-7-trifluoromethyl-pyrazolo[1,5-a]pyrimidin-3-yl)-methanone;
   (5-Furan-2-yl-7-trifluoromethyl-pyrazolo[1,5-a]pyrimidin-3-yl)-(2-methyl-piperidin-1-yl)-methanone;
   [5-(4-Methoxy-phenyl)-7-trifluoromethyl-pyrazolo[1,5-a]pyrimidin-3-yl]-piperidin-1-yl-methanone;
   (5-Benzo[1,3]dioxol-5-yl-7-trifluoromethyl-pyrazolo[1,5-a]pyrimidin-3-yl)-morpholin-4-yl-methanone;
   (5-Benzo[1,3]dioxol-5-yl-7-trifluoromethyl-pyrazolo[1,5-a]pyrimidin-3-yl)-piperidin-1-yl-methanone;
   Morpholin-4-yl-[7-trifluoromethyl-5-(3,4,5-trimethoxy-phenyl)-pyrazolo[1,5-a]pyrimidin-3-yl]-methanone;
   (4-Benzyl-piperazin-1-yl)-[7-trifluoromethyl-5-(3,4,5-trimethoxy-phenyl)-pyrazolo[1,5-a]pyrimidin-3-yl]-methanone;
   (3,4-Dihydro-2H-quinolin-1-yl)-[7-trifluoromethyl-5-(3,4,5-trimethoxy-phenyl)-pyrazolo[1,5-a]pyrimidin-3-yl]-methanone;
   (4-Methyl-piperazin-1-yl)-[7-trifluoromethyl-5-(3,4,5-trimethoxy-phenyl)-pyrazolo[1,5-a]pyrimidin-3-yl]-methanone;
   Azepan-1-yl-[7-trifluoromethyl-5-(3,4,5-trimethoxy-phenyl)-pyrazolo[1,5-a]pyrimidin-3-yl]-methanone;
   [5-(4-Methoxy-phenyl)-7-trifluoromethyl-pyrazolo[1,5-a]pyrimidin-3-yl]-piperidin-1-yl-methanone;
   [5-(4-Methoxy-phenyl)-7-trifluoromethyl-pyrazolo[1,5-a]pyrimidin-3-yl]-pyrrolidin-1-yl-methanone;
   Piperidin-1-yl-(5-thiophen-2-yl-7-trifluoromethyl-pyrazolo[1,5-a]pyrimidin-3-yl)-methanone;
   (4-Benzyl-piperazin-1-yl)-[5-(4-methoxy-phenyl)-7-trifluoromethyl-pyrazolo[1,5-a]pyrimidin-3-yl]-methanone;
   (3,4-Dihydro-2H-quinolin-1-yl)-[5-(4-methoxy-phenyl)-7-trifluoromethyl-pyrazolo[1,5-a]pyrimidin-3-yl]-methanone;
   (5-Furan-2-yl-7-trifluoromethyl-pyrazolo[1,5-a]pyrimidin-3-yl)-(2-methyl-piperidin-1-yl)-methanone;
   (5-Furan-2-yl-7-trifluoromethyl-pyrazolo[1,5-a]pyrimidin-3-yl)-morpholin-4-yl-methanone;
   (5-Furan-2-yl-7-trifluoromethyl-pyrazolo[1,5-a]pyrimidin-3-yl)-thiomorpholin-4-yl-methanone;
   [4-(4-Fluoro-phenyl)-piperazin-1-yl]-(5-thiophen-2-yl-7-trifluoromethyl-pyrazolo[1,5-a]pyrimidin-3-yl)-methanone;
   (4-Methyl-piperidin-1-yl)-(5-thiophen-2-yl-7-trifluoromethyl-pyrazolo[1,5-a]pyrimidin-3-yl)-methanone;
   (5-Furan-2-yl-7-trifluoromethyl-pyrazolo[1,5-a]pyrimidin-3-yl)-(4-methyl-piperazin-1-yl)-methanone;
   (4-Benzyl-piperazin-1-yl)-(5-thiophen-2-yl-7-trifluoromethyl-pyrazolo[1,5-a]pyrimidin-3-yl)-methanone;
   [5-(4-Methoxy-phenyl)-7-trifluoromethyl-pyrazolo[1,5-a]pyrimidin-3-yl]-morpholin-4-yl-methanone;
   Piperidin-1-yl-[7-trifluoromethyl-5-(3,4,5-trimethoxy-phenyl)-pyrazolo[1,5-a]pyrimidin-3-yl]-methanone;
   (2-Methyl-piperidin-1-yl)-(5-thiophen-2-yl-7-trifluoromethyl-pyrazolo[1,5-a]pyrimidin-3-yl)-methanone;
   Azepan-1-yl-[5-(4-methoxy-phenyl)-7-trifluoromethyl-pyrazolo[1,5-a]pyrimidin-3-yl]-methanone;
   5-Thiophen-2-yl-7-trifluoromethyl-pyrazolo[1,5-a]pyrimidine-3-carboxylic acid benzyl-methyl-amide;
   [5-(4-Methoxy-phenyl)-7-trifluoromethyl-pyrazolo[1,5-a]pyrimidin-3-yl]-pyrrolidin-1-yl-methanone;
   [5-(4-Chloro-phenyl)-2-methyl-7-trifluoromethyl-pyrazolo[1,5-a]pyrimidin-3-yl]-piperidin-1-yl-methanone;
   Azepan-1-yl-[5-(4-chloro-phenyl)-2-methyl-7-trifluoromethyl-pyrazolo[1,5-a]pyrimidin-3-yl]-methanone;
   [5-(4-Chloro-phenyl)-2-methyl-7-trifluoromethyl-pyrazolo[1,5-a]pyrimidin-3-yl]-(4-methylpiperazin-1-yl)-methanone;
   Morpholin-4-yl-(5-p-tolyl-7-trifluoromethyl-pyrazolo[1,5-a]pyrimidin-3-yl)-methanone;
   (4-Benzyl-piperazin-1-yl)-(5-p-tolyl-7-trifluoromethyl-pyrazolo[1,5-a]pyrimidin-3-yl)-methanone;
   5-(4-Methoxy-phenyl)-7-trifluoromethyl-pyrazolo[1,5-a]pyrimidine-3-carboxylic acid benzyl-methyl-amide;
   (4-Benzyl-piperazin-1-yl)-(5-phenyl-7-trifluoromethyl-pyrazolo[1,5-a]pyrimidin-3-yl)-methanone;
   Morpholin-4-yl-(5-phenyl-7-trifluoromethyl-pyrazolo[1,5-a]pyrimidin-3-yl)-methanone; or
   a salt thereof with a pharmaceutically acceptable acid or base, or any optical isomer or mixture of optical isomers, including a racemic mixture, or any tautomeric forms.
38. A compound according to clause 36 or 37, which is an agent useful for the treatment, prevention and/or prophylaxis of any conditions, disorders and diseases wherein a modulation or an inhibition of the activity of 11 βHSD1 is beneficial.
39. A compound according to clause 36 or 37, which is an agent useful for the treatment, prevention and/or prophylaxis of any conditions, disorders and diseases that are influenced by intracellular glucocorticoid levels.
40. A compound according to clause 36 or 37, which is an agent useful for the treatment, prevention and/or prophylaxis of conditions, disorders or diseases selected from the group consisting of the metabolic syndrome, insulin resistance, dyslipidemia, hypertension and obesity.
41. A compound according to clause 36 or 37, which is an agent useful for the treatment, prevention and/or prophylaxis of type 2 diabetes, impaired glucose tolerance (IGT), impaired fasting glucose (IFG).
42. A compound according to clause 36 or 37, which is an agent useful for the delaying or prevention of the progression from IGT into type 2 diabetes.
43. A compound according to clause 36 or 37, which is an agent useful for delaying or prevention of the progression of the metabolic syndrome into type 2 diabetes.
44. A compound according to clause 36 or 37, which is an agent useful for the treatment, prevention and/or prophylaxis of adverse effects of glucocorticoid receptor agonist treatment or therapy.
45. A pharmaceutical composition comprising, as an active ingredient, at least one compound according to clause 36 or 37, together with one ore more pharmaceutically acceptable carriers or excipients.
46. The pharmaceutical composition according to clause 45 which is for oral, nasal, buccal, transdermal, pulmonal or parenteral administration.
47. The pharmaceutical composition according to clause 45 or 46 in unit dosage form, comprising from 0.05 mg to 2000 mg/day, from 0.1 mg to 1000 mg or from 0.5 mg to 500 mg per day of the compound according to clause 36 or 37.
48. A use of a compound according to clause 36 or 37, for the preparation of a pharmaceutical composition for the treatment, prevention and/or prophylaxis of any conditions, disorders and diseases wherein a modulation or an inhibition of the activity of 11βHSD1 is beneficial.
49. A use of a compound according to clause 36 or 37, for the preparation of a pharmaceutical composition for the treatment, prevention and/or prophylaxis of any conditions, disorders and diseases that are influenced by intracellular glucocorticoid levels.
50. A use of a compound according to clause 36 or 37, for the preparation of a pharmaceutical composition for the treatment, prevention and/or prophylaxis of conditions, disorders or diseases selected from the group consisting of the metabolic syndrome, insulin resistance, dyslipidemia, hypertension and obesity.
51. A use of a compound according to clause 36 or 37, for the preparation of a pharmaceutical composition for the treatment, prevention and/or prophylaxis of type 2 diabetes, impaired glucose tolerance (IGT), impaired fasting glucose (IFG).
52. A use of a compound according to clause 36 or 37, for the preparation of a pharmaceutical composition for the delaying or prevention of the progression from IGT to type 2 diabetes.
53. A use of a compound according to clause 36 or 37, for the preparation of a pharmaceutical composition for the delaying or prevention of the progression of the metabolic syndrome into type 2 diabetes.
54. A use of a compound according to clause 36 or 37, for the preparation of a pharmaceutical composition for the treatment, prevention and/or prophylaxis of adverse effects of glucocorticoid receptor agonist treatment or therapy.
55. A method for the treatment, prevention and/or prophylaxis of any conditions, disorders or diseases wherein a modulation or an inhibition of the activity of 11 βHSD1 is beneficial, the method comprising administering to a subject in need thereof an effective amount of a compound according to the invention.
56. The method according to clause 55 wherein the conditions, disorders or diseases are selected from the group consisting of the metabolic syndrome, insulin resistance, dyslipidemia, hypertension and obesity.

## Claims

1. The use of a substituted pyrazolo[1 ,5-a]pyrimidine or a prodrug thereof is of the general formula (I) 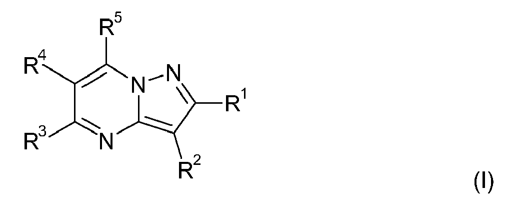 wherein
R¹ and R² independently are hydrogen, halo, C(=O)NR⁶R⁷, CO₂R¹⁵, NR⁶C(=O)R¹¹, OR¹² or SR¹²;
R³ and R⁵ independently are hydrogen, NR¹³R¹⁴, trihalomethyl, trihalomethoxy, C₁-C₆alkyl, C₂-C₆alkenyl, C₂-C₆alkynyl, C₁-C₆alkyloxy, C₁-C₆alkylthio, aryl, arylC₁-C₆alkyl, hetaryl or hetarylC₁-C₆alkyl, wherein alkyl, alkynyl, alkenyl, aryl, hetaryl, arylalkyl or hetarylalkyl groups independently are optionally substituted with one or more of R⁸;
R⁴ is hydrogen, cyano, halo, CO₂R¹⁵, C(=O)NR⁶R⁷, C₁-C₆alkyl, C₂-C₆alkenyl, C₂-C₆alkynyl, aryl, hetaryl, arylC₁-C₆alkyl, hetarylC₁-C₆alkyl, hydroxy, C₁-C₆alkyloxy, arylC₁-C₆alkyloxy, hetarylC₁-C₆alkyloxy, or C₁-C₆alkyloxyC₁-C₆alkyl, wherein the alkyl, alkynyl, alkenyl, cycloalkyl, aryl, hetaryl, arylalkyl or hetarylalkyl groups independently are optionally substituted with one or more of R⁹;
R⁶ and R⁷ independently are C₁-C₆alkyl, C₃-C₁₀cycloalkyl, hetC₃-C₁₀cycloalkyl, arylC₁-C₆alkyl or hetarylC₁-C₆alkyl, wherein the alkyl, cycloalkyl, hetcycloalkyl, arylalkyl, and hetarylalkyl groups independently are optionally substituted with one or more of R¹⁰; or
R⁶ and R⁷ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆-alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R⁸ and R⁹ independently are hydrogen, halo, hydroxy, oxo, cyano, nitro, C₃-C₁₀cycloalkyl, C₃-C₁₀hetocycloalkyl, C₁-C₆alkyl, C₁-C₆alkyloxy, trihalomethyl, trihalomethoxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆-alkylcarboxy, hetarylC₁-C₆alkylcarboxy, C₁-C₆alkylcarbonylamino or arylC₁-C₆alkylcarbonylamino;
R¹⁰ is hydrogen, halo, cyano, nitro, hydroxy, oxo, C₃-C₁₀cycloalkyl, C₃-C₁₀hetcycloalkyl, C₁-C₆alkyl, C₁-C₆alkyloxy, trihalomethyl, trihalometh dialkylamino oxy, aryl₁-C₆alkyloxy, hetaryl-C₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;
R¹¹ is C₁-C₆alkyl, C₂-C₆alkenyl, C₂-C₆alkynyl, arylC₁-C₆alkyl, hetarylC₁-C₆alkyl, C₁-C₆alkylcarbonylC₁-C₆alkyl, C₁-C₆alkyloxy, aryloxy, C₁-C₆alkyloxy, arylcarbonyl, arylC₁-C₆alkylcarbonyl, hetarylcarbonylC₁-C₆alkyl, wherein the alkyl, alkenyl, alkynyl, aryl and hetaryl groups independently are optionally substituted with one or more of R⁹;
R¹² is C₁-C₆alkylcarbonylaminoC₁-C₆alkyl, arylcarbonylaminoC₁-C₆alkyl or arylC₁-C₆alkylcarbonylaminoC₁-C₆alkyl;
R¹³ and R¹⁴ independently are hydrogen, oxo, C₃-C₁₀cycloalkyl, C₁-C₆alkyl, C₁-C₆alkylcarbonyl, arylcarbonyl, hetarylcarbonyl, arylC₁-C₆alkylcarbonyl, hetarylC₁-C₆alkylcarbonyl;
R¹⁵ is hydrogen, C₃-C₁₀cydoalkyl, C₁-C₆alkyl, aryl, hetaryl, arylC₁-C₆alkyl, hetarylC₁-C₆alkyl, C₁-C₆alkyloxyalkyl or arylC₁-C₆alkyloxyalkyl; or
a salt thereof with a pharmaceutically acceptable acid or base, or any optical isomer or mixture of optical isomers, including a racemic mixture, or any tautomeric forms.

2. The use according to claim 1, wherein the substituted pyrazolo[1,5-*a*]pyrimidine or a prodrug thereof is of the general formula (I) 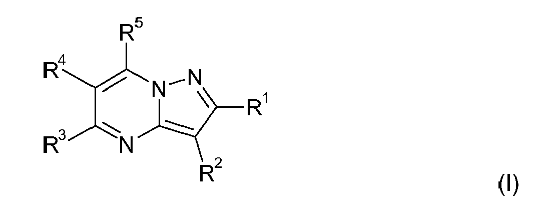 wherein
R¹ and R² independently are hydrogen, halo, C(=O)NR⁶R⁷, NC(=O)R¹¹, OR¹² or SR¹²;
R³ and R⁵ independently are hydrogen, NR¹³R¹⁴, trihalomethyl, trihalomethoxy, C₁-C₆alkyl, C₂-C₆alkenyl, C₂-C₆alkynyl, C₁-C₆alkyloxy, C₁-C₆alkylthio, aryl, arylC₁-C₆alkyl, hetaryl or hetaryl-C₁-C₆alkyl, wherein alkyl, alkynyl, alkenyl, aryl, hetaryl, arylalkyl or hetarylalkyl groups independently are optionally substituted with one or more of R⁸;
R⁴ is hydrogen, cyano, halo, CO₂R¹⁵, C(=O)NR⁶R⁷, C₁-C₆alkyl, C₂-C₆alkenyl, C₂-C₆alkynyl, aryl, hetaryl, arylC₁-C₆alkyl, hetarylC₁-C₆alkyl, hydroxy, C₁-C₆alkyloxy, arylC₁-C₆alkyloxy, hetarylC₁-C₆alkyloxy, or C₁-C₆alkyloxyC₁-C₆alkyl, wherein the alkyl, alkynyl, alkenyl, cycloalkyl, aryl, hetaryl, arylalkyl or hetarylalkyl groups independently are optionally substituted with one or more of R⁹;
R⁶ and R⁷ independently are C₁-C₆alkyl, C₃-C₁₀cycloalkyl, hetC₃-C₁₀cycloalkyl, arylC₁-C₆alkyl or hetarylC₁-C₆alkyl, wherein the alkyl, cycloalkyl, hetcycloalkyl, arylalkyl, and hetarylalkyl groups independently are optionally substituted with one or more of R¹⁰; or
R⁶ and R⁷ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, halo, hydroxy, oxo, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy or hetarylC₁-C₆alkylcarboxy;
R⁸ and R⁹ independently are hydrogen, halo, hydroxy, oxo, cyano, nitro, C₃-C₁₀cycloalkyl, C₃-C₁₀hetocycloalkyl, C₁-C₆alkyl, C₁-C₆alkyloxy, trihalomethyl, trihalomethoxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆-alkylcarboxy, hetarylC₁-C₆alkylcarboxy,C₁-C₆alkylcarbonylamino or arylC₁-C₆alkylcarbonylamino;
R¹⁰ is hydrogen, halo, cyano, nitro, hydroxy, oxo, C₃-C₁₀cycloalkyl, C₃-C₁₀hetcycloalkyl, C₁-C₆alkyl, C₁-C₆alkyloxy, trihalomethyl, trihalometh dialkylamino oxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;
R11 is C₁-C₆alkyl, C₂-C₆alkenyl, C₂-C₆alkynyl, arylC₁-C₆alkyl, hetarylC₁-C₆alkyl, C₁-C₆alkylcarbonylC₁-C₆alkyl, C₁-C₆alkyl, arylcarbonyl, arylC₁-C₆alkylcarbonyl, hetarylcarbonylC₁-C₆-alkyl, wherein the alkyl, alkenyl, alkynyl, aryl and hetaryl groups independently are optionally substituted with one or more of R⁹;
R¹² is C₁-C₆alkylcarbonylaminoC₁-C₆alkyl, arylcarbonylaminoC₁-C₆alkyl or arylC₁-C₆alkylcarbonylaminoC₁-C₆alkyl;
R¹³and R¹⁴ independently are hydrogen, oxo, C₃-C₁₀cycloalkyl, C₁-C₆alkyl, C₁-C₆alkyloxy, trihalomethyl, trihalomethoxy, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy, hetarylcarboxy, arylC₁-C₆alkylcarboxy;
R¹⁵ is hydrogen, C₃-C₁₀cycloalkyl, C₁-C₆alkyl, aryl, hetaryl, arylC₁-C₆alkyl, hetarylC₁-C₆alkyl, C₁-C₆alkyloxyalkyl or arylC₁-C₆alkyloxyalkyl; or
a salt thereof with a pharmaceutically acceptable acid or base, or any optical isomer or mixture of optical isomers, including a racemic mixture, or any tautomeric forms.

3. The use according to claim 1 or 2, wherein the substituted pyrazolo[1,5-a]pyrimidine or a prodrug thereof is selected from the group consisting of:
(3-Bromo-5-thiophen-2-yl-7-trifluoromethyl-pyrazolo[1,5-a]pyrimidin-2-yl)-(2,6-dimethylpiperidin-1-yl-)methanone;
(3-Bromo-5-thiophen-2-yl-7-trifluoromethyl-pyrazolo[1,5-*a*]pyrimidin-2-yl)-(2-ethyl-piperidin-1-yl-)methanone;
(5-Thiophen-2-yl-7-trifluoromethyl-pyrazolo[1,5-a]pyrimidin-2-yl)-(1,3,3-trimethyl-6-azabicyclo[3.2.1]oct-6-yl)methanone;
(3-Chloro-5-thiophen-2-yl-7-trifluoromethyl-pyrazolo[1,5-a]pyrimidin-2-yl)-(2-methyl-piperidin-1-yl-)methanone;
5-Methyl-7-phenyl-pyrazolo[1,5-a]pyrimidine-2-carboxylic acid cyclohexyl amide;
Azepan-1-yl-(3-chloro-5-thiophen-2-yl-7-trifluoromethyl-pyrazolo[1,5-a]pyrimidin-2-yl)-methanone;
Azepan-1-yl-(3-chloro-5-thiophen-2-yl-7-trifluoromethyl-pyrazolo[1,5-a]pyrimidin-3-yl)-methanone;
(2,6-Dimethyl-piperidin-1-yl-)-(3-chloro-5-thiophen-2-yl-7-trifluoromethyl-pyrazolo[1,5-a]pyrimidin-3-yl)methanone;
3-Bromo-5-furan-2-yl-7-trifluoromethyl-pyrazolo[1,5-*a*]pyrimidin-2-carboxylic acid cyclohexylamide;
(7-Methyl-5-phenyl-pyrazolo[1,5-*a*]pyrimidin-3-yl)-(4-methyl-piperidin-1-yl)methanone;
5-(-4-Methoxy-phenyl-7-trifluoromethyl-pyrazolo[1,5-*a*]pyrimidine-3-carboxylic acid benzyl-methyl amide;
[5-(4-Ethoxy-phenyl)-7-methyl-pyrazolo[1,5-*a*]pyrimidine-2-yl]-piperidin-1-yl-methanone;
Azepan-1-yl-(3-bromo-5-furan-2-yl-7-trifluoromethyl-pyrazolo[1,5-*a*]pyrimidin-2-yl)methanone;
5-Benzo[1,3]dioxol-5-yl-7-trifluoromethyl-pyrazolo[1,5-*a*]pyrimidine-3-carboxylic acid cyclohexyl-methyl amide;
(3,4-Dihydro-1 H-isoquinolin-2-yl)-[5-(-4-Methoxy-phenyl-7-trifluoromethyl-pyrazolo[1,5-a]pyrimidin-3-yl]methanone;
Azepan-1-yl-[5-(4-bromo-phenyl)-7-trifluoromethyl-pyrazolo[1,5-a]pyrimidin-3-yl)methanone;
Azepan-1-yl-(5,7-diphenyl)-pyrazolo[1,5-a]pyrimidin-3-yl)methanone;
[5-(4-Bromo-phenyl)-7-trifluoromethyl-pyrazolo[1,5-a]pyrimidin-3-yl]-(2-methyl-piperidin-1-yl)-methanone;
(5-Furan-2-yl-7-trifluoromethyl-pyrazolo[1,5-a]pyrimidin-3-yl)-(2-methyl-piperidin-1-yl)-methanone;
(2,6-Dimethyl-piperidin-1-yl-)-[7-(4-ethoxy-phenyl)-5-methyl-pyrazolo[1,5-a]pyrimidin-2-yl] methanone;
5-(4-Ethoxy-phenyl)-7-methyl-pyrazolo[1,5-a]pyrimidine-2-carboxylic acid cyclohexylamide;
Piperidin-1-yl-(5-thiophen-2-yl-7-trifluoromethyl-pyrazolo[1,5-a]pyrimidin-3-yl)-methanone;
(5-Furan-2-yl-7-trifluoromethyl-pyrazolo[1,5-a]pyrimidin-3-yl)-(2-methyl-piperidin-1-yl)-methanone;
(2-Methyl-piperidin-1-yl)-(5-thiophen-2-yl-7-trifluoromethyl-pyrazolo[1,5-a]pyrimidin-3-yl)-methanone;
Azepan-1-yl-[5-(4-methoxy-phenyl)-7-trifluoromethyl-pyrazolo[1,5-a]pyrimidin-3-yl]-methanone;
5-Thiophen-2-yl -7-trifluoromethyl-pyrazolo[1,5-a]pyrimidine-3-carboxylic acid benzyl-methyl-amide;
5-p-Tolyl-7-trifluoromethyl-pyrazolo[1,5-a]pyrimidine-3-carboxylic acid (1,3-dimethyl-1 H-pyrazol-4-yl-methyl)methyl amide;
5-Methyl-7-phenyl-pyrazolo[1,5-a]pyrimidine-2-carboxylic acid cyclohexyl-methyl amide;
7-Methyl-5-phenyl-pyrazolo[1,5-a]pyrimidine-2-carboxylic acid cyclohexyl-methyl amide;
(5-Phenyl-7-trifluoromethyl-pyrazolo[1,5-a]pyrimidin-2-yl)-piperidin-1-yl-methanone;
5-Naphthalen-1-yl-7-trifluoromethyl-pyrazolo[1,5-*a*]pyrimidine-2-carboxylic acid cyclohexyl-methyl amide;
5-Naphthalen-1-yl-7-trifluoromethyl-pyrazolo[1,5-a]pyrimidine-2-carboxylic acid cyclohexyl amide;
(5-Naphthalen-1-yl-7-trifluoromethyl-pyrazolo[1,5-*a*]pyrimidin-2-yl)-piperidin-1-yl-methanone
(2-Methyl-piperidin-1-yl)-(5-naphthalen-1-yl-7-trifluoromethyl-pyrazolo[1,5-a]pyrimidin-2-yl)-methanone;
5,7-Diphenyl-pyrazolo[1,5-a]pyrimidine-2-carboxylic acid cyclohexyl amide;
5,7-Diphenyl-pyrazolo[1,5-a]pyrimidine-2-carboxylic acid cyclohexyl-methyl amide;
(5,7-Diphenyl-pyrazolo[1,5-a]pyrimidin-2-yl)-(2-methyl-piperidin-1-yl)methanone;
(5,7-Diphenyl-pyrazolo[1,5-a]pyrimidin-2-yl)-piperidin-1-yl-methanone;
5-Methyl-7-trifluoromethyl-pyrazolo[1,5-a]pyrimidine-2-carboxylic acid cyclohexyl amide;
(5-Methyl-7-trifluoromethyl-pyrazolo[1,5-*a*]pyrimidin-2-yl)-piperidin-1-yl-methanone;
5-Methyl-7-trifluoromethyl-pyrazolo[1,5-*a*]pyrimidine-2-carboxylic acid cyclohexyl-methyl-amide;
(2-Methyl-piperidin-1-yl)-(5-methyl-7-trifluoromethyl-pyrazolo[1,5-a]pyrimidin-2-yl)-methanone;
5-Phenyl-7-trifluoromethyl-pyrazolo[1,5-a!]pyrimidine-2-carboxylic acid cyclohexyl amide;
(5-Phenyl-7-trifluoromethyl-pyrazolo[1,5-a]pyrimidin-2-yl)-piperidin-1-yl-methanone;
7-Phenyl-5-trifluoromethyl-pyrazolo[1,5-a]pyrimidine-2-carboxylic acid cyclohexyl-methyl-amide;
5-Phenyl-7-trifluoromethyl-pyrazolo[1,5-a]pyrimidine-3-carboxylic acid cyclohexyl amide;
(5-Phenyl-7-trifluoromethyl-pyrazolo[1,5-a]pyrimidin-3-yl)-piperidin-1-yl-methanone;or
a salt thereof with a pharmaceutically acceptable acid or base, or any optical isomer or mixture of optical isomers, including a racemic mixture, or any tautomeric forms.

4. The use according to any of the claims 1-3, for the preparation of a pharmaceutical composition for the treatment, prevention and/or prophylaxis any conditions, disorders and diseases that are influenced by intracellular glucocorticoid levels.

5. The use according to any of the claims 1-3, for the preparation of a pharmaceutical composition for the treatment, prevention and/or prophylaxis of adverse effects of glucocorticoid receptor agonist treatment or therapy.

6. A substituted pyrazolo[1,5-a]pyrimidine or a prodrug thereof the general formula (I) which is:
5-Methyl-7-trifluoromethyl-pyrazolo[1,5-a]pyrimidine-2-carboxylic acid methyl ester;
5-Methyl-7-phenyl-pyrazolo[1,5-a]pyrimidine-2-carboxylic acid methyl ester;
7-Methyl-5-phenyl-pyrazolo[1,5-a]pyrimidine-2-carboxylic acid methyl ester;
(5-methyl-7-trifluoromethyl-pyrazolo[1,5-a]pyrimidin-2-yl)- (piperidin-1-yl)methanone;
(5-methyl-7-phenyl-pyrazolo[1,5-a]pyrimidine-2-carboxylic acid cyclohexyl-methyl amide; or
a salt thereof with a pharmaceutically acceptable acid or base, or any optical isomer or mixture of optical isomers, including a racemic mixture, or any tautomeric forms.

7. A substituted pyrazolo[1,5-a]pyrimidine or a prodrug thereof the general formula (I) which is:
7-Methyl-5-phenyl-pyrazolo[1,5-a]pyrimidine-2-carboxylic acid cyclohexyl-methyl-amide;
7-Phenyl-5-trifluoromethyl-pyrazolo[1,5-a]pyrimidine-2-carboxylic acid cyclohexyl-methyl-amide;
(5-Phenyl-7-trifluoromethyl-pyrazolo[1,5-a]pyrimidin-2-yl)-piperidin-1-yl-methanone;
(2-Methyl-piperidin-1-yl)-(5-methyl-7-trifluoromethyl-pyrazolo[1,5-a]pyrimidin-2-yl)-methanone;
5-Methyl-7-trifluoromethyl-pyrazolo[1,5-a]pyrimidine-2-carboxylic acid cyclohexyl-methyl-amide;
(5-Methyl-7-trifluoromethyl-pyrazolo[1,5-a]pyrimidin-2-yl)-piperidin-1-yl-methanone;
(5,7-Diphenyl-pyrazolo[1,5-a]pyrimidin-2-yl)-piperidin-1-yl-methanone;
(5,7-Diphenyl-pyrazolo[1,5-a]pyrimidin-2-yl)-(2-methyl-piperidin-1-yl)-methanone;
5,7-Diphenyl-pyrazolo[1,5-a]pyrimidine-2-carboxylic acid cyclohexyl-methyl-amide;
(2-Methyl-piperidin-1-yl)-(5-naphthalen-1-yl-7-trifluoromethyl-pyrazolo[1,5-a]pyrimidin-2-yl)-methanone;
(5-Naphthalen-1-yl-7-trifluoromethyl-pyrazolo[1,5-a]pyrimidin-2-yl)-piperidin-1-yl-methanone;
5-Naphthalen-1-yl-7-trifluoromethyl-pyrazolo[1,5-a]pyrimidine-2-carboxylic acid cyclohexyl-methyl-amide;
[7-(4-Ethoxy-phenyl)-5-methyl-pyrazolo[1,5-a]pyrimidin-2-yl]-(1,3,3-trimethyl-6-azabicyclo[3.2.1]oct-6-yl)-methanone;
(2-Methyl-piperidin-1-yl)-(7-phenyl-5-trifluoromethyl-pyrazolo[1,5-a]pyrimidin-2-yl)-methanone;
(5-Methyl-7-trifluoromethyl-pyrazolo[1,5-a]pyrimidin-2-yl)-(1,3,3-trimethyl-6-azabicyclo[3.2.1]oct-6-yl)-methanone;
(5-Phenyl-7-trifluoromethyl-pyrazolo[1,5-a]pyrimidin-2-yl)-(1,3,3-trimethyl-6-azabicyclo[3.2.1]oct-6-yl)-methanone;
(6-Bromo-3-chloro-pyrazolo[1,5-a]pyrimidin-2-yl)-morpholin-4-yl-methanone;
(5-Phenyl-7-trifluoromethyl-pyrazolo[1,5-a]pyrimidin-2-yl)-piperidin-1-yl-methanone;
Morpholin-4-yl-(5-phenyl-7-trifluoromethyl-pyrazolo[1,5-a]pyrimidin-2-yl)-methanone;
(6-Bromo-pyrazolo[1,5-a]pyrimidin-2-yl)-piperidin-1-yl-methanone;
(6-Bromo-pyrazolo[1,5-a]pyrimidin-2-yl)-morpholin-4-yl-methanone;
(3,6-Dibromo-pyrazolo[1,5-a]pyrimidin-2-yl)-morpholin-4-yl-methanone;
(3-Bromo-5-phenyl-7-trifluoromethyl-pyrazolo[1,5-a]pyrimidin-2-yl)-piperidin-1-yl-methanone;
(3-Bromo-5-phenyl-7-trifluoromethyl-pyrazolo[1,5-a]pyrimidin-2-yl)-morpholin-4-yl-methanone;
(3-Chloro-5-phenyl-7-trifluoromethyl-pyrazolo[1,5-a]pyrimidin-2-yl)-piperidin-1-yl-methanone;
(3-Chloro-5-phenyl-7-trifluoromethyl-pyrazolo[1,5-a]pyrimidin-2-yl)-morpholin-4-yl-methanone;
(3,6-Dibromo-pyrazolo[1,5-a]pyrimidin-2-yl)-piperidin-1-yl-methanone;
[4-(4-Fluoro-phenyl)-piperazin-1-yl]-(5-thiophen-2-yl-7-trifluoromethyl-pyrazolo[1,5-a]pyrimidin-2-yl)-methanone;
5-Thiophen-2-yl-7-trifluoromethyl-pyrazolo[1,5-a]pyrimidine-2-carboxylic acid cyclohexyl-methyl-amide;
(5-Benzo[1,3]dioxol-5-yl-7-trifluoromethyl-pyrazolo[1,5-a]pyrimidin-2-yl)-(4-methyl-piperazin-1-yl)-methanone;
[5-(3,4-Dichloro-phenyl)-7-trifluoromethyl-pyrazolo[1,5-a]pyrimidin-2-yl]-(2-methyl-piperidin-1-yl)-methanone;
3-Chloro-5-(4-methoxy-phenyl)-7-trifluoromethyl-pyrazolo[1,5-a]pyrimidine-2-carboxylic acid methyl-(2-pyridin-2-yl-ethyl)-amide;
[3-Chloro-5-(4-chloro-phenyl)-7-trifluoromethyl-pyrazolo[1,5-a]pyrimidin-2-yl]-morpholin-4-yl-methanone;
(3-Chloro-5-thiophen-2-yl-7-trifluoromethyl-pyrazolo[1,5-a]pyrimidin-2-yl)-(3,4-dihydro-2H-quinolin-1-yl)-methanone;
[5-(3,4-Dichloro-phenyl)-7-trifluoromethyl-pyrazolo[1,5-a]pyrimidin-2-yl]-[4-(2-methoxyphenyl)-piperazin-1-yl]-methanone;
(4-Benzoyl-piperazin-1-yl)-[3-chloro-5-(4-chloro-phenyl)-7-trifluoromethyl-pyrazolo[1,5-a]pyrimidin-2-yl]-methanone;
4-(5-Benzo[1,3]dioxol-5-yl-7-trifluoromethyl-pyrazolo[1,5-a]pyrimidine-2-carbonyl)-piperazine-1-carboxylic acid ethyl ester;
(5-Benzo[1,3]dioxol-5-yl-7-trifluoromethyl-pyrazolo[1,5-a]pyrimidin-2-yl)-(6,7-dimethoxy-1 - methyl-3,4-dihydro-1 H-isoquinolin-2-yl)-methanone;
[4-(Furan-2-carbonyl)-piperazin-1-yl]-[5-(4-methoxy-phenyl)-7-trifluoromethyl-pyrazolo[1,5-a]pyrimidin-2-yl]-methanone;
(3-Chloro-5-thiophen-2-yl-7-trifluoromethyl-pyrazolo[1,5-a]pyrimidin-2-yl)-[4-(2-methoxyphenyl)-piperazin-1-yl]-methanone;
[5-(4-Methoxy-phenyl)-7-trifluoromethyl-pyrazolo[1,5-a]pyrimidin-2-yl]-piperidin-1-yl-methanone;
(3,4-Dihydro-2H-quinolin-1-yl)-(5-phenyl-7-trifluoromethyl-pyrazolo[1,5-a]pyrimidin-2-yl)-methanone;
(3-Bromo-5-thiophen-2-yl-7-trifluoromethyl-pyrazolo[1,5-a]pyrimidin-2-yl)-(2,6-dimethylpiperidin-1-yl)-methanone;
(3-Bromo-5-thiophen-2-yl-7-trifluoromethyl-pyrazolo[1,5-a]pyrimidin-2-yl)-(2-ethyl-piperidin-1-yl)-methanone;
3-Bromo-5-thiophen-2-yl-7-trifluoromethyl-pyrazolo[1,5-a]pyrimidine-2-carboxylic acid cyclohexyl-methyl-amide;
[5-(4-Bromo-phenyl)-3-chloro-7-trifluoromethyl-pyrazolo[1,5-a]pyrimidin-2-yl]-pyrrolidin-1-yl-methanone;
(3-Chloro-5-furan-2-yl-7-trifluoromethyl-pyrazolo[1,5-a]pyrimidin-2-yl)-[4-(4-fluoro-phenyl)-piperazin-1-yl]-methanone;
(3-Chloro-5-furan-2-yl-7-trifluoromethyl-pyrazolo[1,5-a]pyrimidin-2-yl)-(4-phenyl-piperazin-1-yl)-methanone;
[3-Chloro-5-(4-chloro-phenyl)-7-trifluoromethyl-pyrazolo[1,5-a]pyrimidin-2-yl]-(4-methylpiperazin-1-yl)-methanone;
Azepan-1-yl-[3-chloro-5-(4-chloro-phenyl)-7-trifluoromethyl-pyrazolo[1,5-a]pyrimidin-2-yl]-methanone;
[3-Chloro-5-(3,4-dimethoxy-phenyl)-7-trifluoromethyl-pyrazolo[1,5-a]pyrimidin-2-yl]-pyrrolidin-1-yl-methanone;
[5-(4-Chloro-phenyl)-7-trifluoromethyl-pyrazolo[1,5-a]pyrimidin-2-yl]-(2-methyl-piperidin-1-yl)-methanone;
(5-Thiophen-2-yl-7-trifluoromethyl-pyrazolo[1,5-a]pyrimidin-2-yl)-(1,3,3-trimethyl-6-azabicyclo[3.2.1]oct-6-yl)-methanone;
[4-(2-Chloro-phenyl)-piperazin-1-yl]-(5-thiophen-2-yl-7-trifluoromethyl-pyrazolo[1,5-a]pyrimidin-2-yl)-methanone;
(3,4-Dihydro-1 H-isoquinolin-2-yl)-(5-thiophen-2-yl-7-trifluoromethyl-pyrazolo[1,5-a]pyrimidin-2-yl)-methanone;
(4-Methyl-piperazin-1-yl)-[5-(4-nitro-phenyl)-7-trifluoromethyl-pyrazolo[1,5-a]pyrimidin-2-yl]-methanone;
[5-(3,4-Dichloro-phenyl)-7-trifluoromethyl-pyrazolo[1,5-a]pyrimidin-2-yl]-thiomorpholin-4-yl-methanone;
(3-Bromo-5-furan-2-yl-7-trifluoromethyl-pyrazolo[1,5-a]pyrimidin-2-yl)-(3,4-dihydro-2H-quinolin-1-yl)-methanone;
(5-Furan-2-yl-7-trifluoromethyl-pyrazolo[1,5-a]pyrimidin-2-yl)-pyrrolidin-1-yl-methanone;
[4-(Furan-2-carbonyl)-piperazin-1-yl]-[5-(4-methoxy-phenyl)-7-trifluoromethyl-pyrazolo[1,5-a]pyrimidin-2-yl]-methanone;
[5-(4-Nitro-phenyl)-7-trifluoromethyl-pyrazolo[1,5-a]pyrimidin-2-yl]-piperidin-1-yl-methanone;
(5-Furan-2-yl-7-trifluoromethyl-pyrazolo[1,5-a]pyrimidin-2-yl)-pyrrolidin-1-yl-methanone;
(4-Methyl-piperidin-1-yl)-(5-phenyl-7-trifluoromethyl-pyrazolo[1,5-a]pyrimidin-2-yl)-methanone;
(3-Bromo-5-thiophen-2-yl-7-trifluoromethyl-pyrazolo[1,5-a]pyrimidin-2-yl)-[4-(4-fluoro-phenyl)-piperazin-1-yl]-methanone;
[4-(4-Fluoro-phenyl)-piperazin-1-yl]-(5-phenyl-7-trifluoromethyl-pyrazolo[1,5-a]pyrimidin-2-yl)-methanone;
[4-(4-Fluoro-phenyl)-piperazin-1-yl]-(5-thiophen-2-yl-7-trifluoromethyl-pyrazolo[1,5-a]pyrimidin-2-yl)-methanone;
[3-Bromo-5-(4-methoxy-phenyl)-7-trifluoromethyl-pyrazolo[1,5-a]pyrimidin-2-yl]-morpholin-4-yl-methanone;
[4-(4-Fluoro-phenyl)-piperazin-1-yl]-(7-methyl-5-phenyl-pyrazolo[1,5-a]pyrimidin-2-yl)-methanone;
(4-Benzoyl-piperazin-1-yl)-(5-phenyl-7-trifluoromethyl-pyrazolo[1,5-a]pyrimidin-2-yl)-methanone;
(3-Chloro-5-thiophen-2-yl-7-trifluoromethyl-pyrazolo[1,5-a]pyrimidin-2-yl)-(4-phenyl-piperazin-1-yl)-methanone;
(2-Ethyl-piperidin-1-yl)-(5-phenyl-7-trifluoromethyl-pyrazolo[1,5-a]pyrimidin-2-yl)-methanone;
(5,7-Diphenyl-pyrazolo[1,5-a]pyrimidin-2-yl)-(4-methyl-piperazin-1-yl)-methanone;
(4-Benzyl-piperazin-1-yl)-(3-chloro-5-phenyl-7-trifluoromethyl-pyrazolo[1,5-a]pyrimidin-2-yl)-methanone;
(3-Chloro-5-thiophen-2-yl-7-trifluoromethyl-pyrazolo[1,5-a]pyrimidin-2-yl)-(4-methyl-piperazin-1-yl)-methanone;
(4-Phenyl-piperazin-1-yl)-(5-thiophen-2-yl-7-trifluoromethyl-pyrazolo[1,5-a]pyrimidin-2-yl)-methanone;
[5-(4-Bromo-phenyl)-7-trifluoromethyl-pyrazolo[1,5-a]pyrimidin-2-yl]-pyrrolidin-1-yl-methanone;
(5,7-Diphenyl-pyrazolo[1,5-a]pyrimidin-2-yl)-pyrrolidin-1-yl-methanone;
(3-Chloro-5-thiophen-2-yl-7-trifluoromethyl-pyrazolo[1,5-a]pyrimidin-2-yl)-(2-ethyl-piperidin-1-yl)-methanone;
(3-Chloro-5-thiophen-2-yl-7-trifluoromethyl-pyrazolo[1,5-a]pyrimidin-2-yl)-(2-methyl-piperidin-1-yl)-methanone;
5-Methyl-7-phenyl-pyrazolo[1,5-a]pyrimidine-2-carboxylic acid cyclohexyl-methyl-amide;
(3-Chloro-5-thiophen-2-yl-7-trifluoromethyl-pyrazolo[1,5-a]pyrimidin-2-yl)-morpholin-4-yl-methanone;
(3-Chloro-5-thiophen-2-yl-7-trifluoromethyl-pyrazolo[1,5-a]pyrimidin-2-yl)-[4-(4-fluoro-phenyl)-piperazin-1-yl]-methanone;
(3-Bromo-5-thiophen-2-yl-7-trifluoromethyl-pyrazolo[1,5-a]pyrimidin-2-yl)-(4-methyl-piperazin-1-yl)-methanone;
(7-Methyl-5-phenyl-pyrazolo[1,5-a]pyrimidin-2-yl)-piperidin-1-yl-methanone;
Azepan-1-yl-(3-chloro-5-thiophen-2-yl-7-trifluoromethyl-pyrazolo[1,5-a]pyrimidin-2-yl)-methanone;
Azepan-1-yl-[3-bromo-5-(4-bromo-phenyl)-7-trifluoromethyl-pyrazolo[1,5-a]pyrimidin-2-yl]-methanone;
3-Bromo-5-(4-bromo-phenyl)-7-trifluoromethyl-pyrazolo[1,5-a]pyrimidine-2-carboxylic acid cyclohexyl-methyl-amide;
(2,6-Dimethyl-piperidin-1-yl)-[5-(4-methoxy-phenyl)-7-trifluoromethyl-pyrazolo[1,5-a]pyrimidin-2-yl]-methanone;
[5-(4-Bromo-phenyl)-7-trifluoromethyl-pyrazolo[1,5-a]pyrimidin-2-yl]-(4-methyl-piperazin-1-yl)-methanone;
5-Phenyl-7-trifluoromethyl-pyrazolo[1,5-a]pyrimidine-2-carboxylic acid benzyl-methyl-amide;
[5-(4-Ethoxy-phenyl)-7-methyl-pyrazolo[1,5-a]pyrimidin-2-yl]-(4-phenyl-piperazin-1-yl)-methanone;
(3,4-Dihydro-1 H-isoquinolin-2-yl)-[5-(4-methoxy-phenyl)-7-trifluoromethyl-pyrazolo[1,5-a]pyrimidin-2-yl]-methanone;
Morpholin-4-yl-[5-(4-nitro-phenyl)-7-trifluoromethyl-pyrazolo[1,5-a]pyrimidin-2-yl]-methanone;
(Hexahydro-pyrrolo[1,2-a]pyrazin-2-yl)-(5-phenyl-7-trifluoromethyl-pyrazolo[1,5-a]pyrimidin-2-yl)-methanone;
(Hexahydro-pyrrolo[1,2-a]pyrazin-2-yl)-(5-p-tolyl-7-trifluoromethyl-pyrazolo[1,5-a]pyrimidin-2-yl)-methanone;
(Hexahydro-pyrrolo[1,2-a]pyrazin-2-yl)-(5-thiophen-2-yl-7-trifluoromethyl-pyrazolo[1,5-a]pyrimidin-2-yl)-methanone;
(3-Chloro-5-phenyl-7-trifluoromethyl-pyrazolo[1,5-a]pyrimidin-2-yl)-(hexahydro-pyrrolo[1,2-a]pyrazin-2-yl)-methanone;
[5-(4-Fluoro-phenyl)-7-methyl-pyrazolo[1,5-a]pyrimidin-2-yl]-morpholin-4-yl-methanone;
(5,7-Diphenyl-pyrazolo[1,5-a]pyrimidin-2-yl)-(2-methyl-piperidin-1-yl)-methanone;
5-Phenyl-7-trifluoromethyl-pyrazolo[1,5-a]pyrimidine-2-carboxylic acid (1,3-dimethyl-1H-pyrazol-4-ylmethyl)-methyl-amide;
5-Thiophen-2-yl-7-trifluoromethyl-pyrazolo[1,5-a]pyrimidine-2-carboxylic acid (1,5-dimethyl-1 H-pyrazol-4-ylmethyl)-methyl-amide;
5-Thiophen-2-yl-7-trifluoromethyl-pyrazolo[1,5-a]pyrimidine-2-carboxylic acid methyl-(1,3,5-trimethyl-1 *H*-pyrazol-4-ylmethyl)-amide;
3-Chloro-5-(4-methoxy-phenyl)-7-trifluoromethyl-pyrazolo[1,5-a]pyrimidine-2-carboxylic acid (1,3-dimethyl-1 H-pyrazol-4-ylmethyl)-methyl-amide;
5-(4-Chloro-phenyl)-7-trifluoromethyl-pyrazolo[1,5-a]pyrimidine-2-carboxylic acid (1,3-dimethyl-1 H-pyrazol-4-ylmethyl)-methyl-amide;
5-(3,4-Dimethoxy-phenyl)-7-trifluoromethyl-pyrazolo[1,5-a]pyrimidine-2-carboxylic acid (1,3-dimethyl-1 H-pyrazol-4-ylmethyl)-methyl-amide;
(2,5-Dimethyl-piperidin-1-yl)-(5-phenyl-7-trifluoromethyl-pyrazolo[1,5-a]pyrimidin-2-yl)-methanone;
5-Furan-2-yl-7-trifluoromethyl-pyrazolo[1,5-a]pyrimidine-2-carboxylic acid methyl-(1,3,5-trimethyl-1 H-pyrazol-4-ylmethyl)-amide;
5-(4-Chloro-phenyl)-7-trifluoromethyl-pyrazolo[1,5-a]pyrimidine-2-carboxylic acid (1,5-dimethyl-1 H-pyrazol-4-ylmethyl)-methyl-amide;
(2,4-Dimethyl-piperidin-1-yl)-(5-phenyl-7-trifluoromethyl-pyrazolo[1,5-a]pyrimidin-2-yl)-methanone;
(3-Bromo-5-phenyl-7-trifluoromethyl-pyrazolo[1,5-a]pyrimidin-2-yl)-(hexahydro-pyrrolo[1,2-a]pyrazin-2-yl)-methanone;
[5-(3,4-Dimethoxy-phenyl)-7-trifluoromethyl-pyrazolo[1,5-a]pyrimidin-2-yl]-(hexahydropyrrolo[1,2-a]pyrazin-2-yl)-methanone;
(3-Chloro-5-p-tolyl-7-trifluoromethyl-pyrazolo[1,5-a]pyrimidin-2-yl)-(hexahydro-pyrrolo[1,2-a]pyrazin-2-yl)-methanone;
(3-Bromo-5-furan-2-yl-7-trifluoromethyl-pyrazolo[1,5-a]pyrimidin-2-yl)-piperidin-1-yl-methanone;
(3-Chloro-5-phenyl-7-trifluoromethyl-pyrazolo[1,5-a]pyrimidin-2-yl)-pyrrolidin-1-yl-methanone;
(7-Methyl-5-phenyl-pyrazolo[1,5-a]pyrimidin-2-yl)-(4-methyl-piperidin-1-yl)-methanone;
(4-Methyl-piperazin-1-yl)-(5-thiophen-2-yl-7-trifluoromethyl-pyrazolo[1,5-a]pyrimidin-2-yl)-methanone;
[3-Bromo-5-(4-methoxy-phenyl)-7-trifluoromethyl-pyrazolo[1,5-a]pyrimidin-2-yl]-piperidin-1-yl-methanone;
(4-Methyl-piperazin-1-yl)-(5-phenyl-7-trifluoromethyl-pyrazolo[1,5-a]pyrimidin-2-yl)-methanone;
[4-(4-Fluoro-phenyl)-piperazin-1-yl]-(5-furan-2-yl-7-trifluoromethyl-pyrazolo[1,5-a]pyrimidin-2-yl)-methanone;
[5-(4-Methoxy-phenyl)-7-trifluoromethyl-pyrazolo[1,5-a]pyrimidin-2-yl]-morpholin-4-yl-methanone;
(3,4-Dihydro-2*H*-quinolin-1-yl)-(5-thiophen-2-yl-7-trifluoromethyl-pyrazolo[1,5-a]pyrimidin-2-yl)-methanone;
(3-Bromo-5-thiophen-2-yl-7-trifluoromethyl-pyrazolo[1,5-a]pyrimidin-2-yl)-morpholin-4-yl-methanone;
Azepan-1-yl-(3-chloro-5-phenyl-7-trifluoromethyl-pyrazolo[1,5-a]pyrimidin-2-yl)-methanone;
(4-Benzoyl-piperazin-1-yl)-[5-(4-bromo-phenyl)-7-trifluoromethyl-pyrazolo[1,5-a]pyrimidin-2-yl]-methanone;
(4-Benzyl-piperazin-1-yl)-[5-(4-bromo-phenyl)-3-chloro-7-trifluoromethyl-pyrazolo[1,5-a]pyrimidin-2-yl]-methanone;
[5-(4-Bromo-phenyl)-3-chloro-7-trifluoromethyl-pyrazolo[1,5-a]pyrimidin-2-yl]-(4-methylpiperazin-1-yl)-methanone;
Azepan-1-yl-[5-(4-bromo-phenyl)-7-trifluoromethyl-pyrazolo[1,5-a]pyrimidin-2-yl]-methanone;
[3-Bromo-5-(4-bromo-phenyl)-7-trifluoromethyl-pyrazolo[1,5-a]pyrimidin-2-yl]-(3,4-dihydro-2H-quinolin-1-yl)-methanone;
[5-(3,4-Dimethoxy-phenyl)-7-trifluoromethyl-pyrazolo[1,5-a]pyrimidin-2-yl]-morpholin-4-yl-methanone;
(3-Bromo-5-furan-2-yl-7-trifluoromethyl-pyrazolo[1,5-a]pyrimidin-2-yl)-(4-methyl-piperazin-1-yl)-methanone;
(4-Benzoyl-piperazin-1-yl)-(3-bromo-5-furan-2-yl-7-trifluoromethyl-pyrazolo[1,5-a]pyrimidin-2-yl)-methanone;
(3-Bromo-5-furan-2-yl-7-trifluoromethyl-pyrazolo[1,5-a]pyrimidin-2-yl)-(3,4-dihydro-2H-quinolin-1-yl)-methanone;
[5-(4-Bromo-phenyl)-7-trifluoromethyl-pyrazolo[1,5-a]pyrimidin-2-yl]-piperidin-1-yl-methanone;
[5-(4-Ethoxy-phenyl)-7-methyl-pyrazolo[1,5-a]pyrimidin-2-yl]-piperidin-1-yl-methanone;
[5-(4-Ethoxy-phenyl)-7-methyl-pyrazolo[1,5-a]pyrimidin-2-yl]-morpholin-4-yl-methanone;
Azepan-1-yl-(3-bromo-5-furan-2-yl-7-trifluoromethyl-pyrazolo[1,5-a]pyrimidin-2-yl)-methanone;
(3,4-Dihydro-1 H-isoquinolin-2-yl)-(5-thiophen-2-yl-7-trifluoromethyl-pyrazolo[1,5-a]pyrimidin-2-yl)-methanone;
(4-Benzyl-piperazin-1-yl)-(3-bromo-5-furan-2-yl-7-trifluoromethyl-pyrazolo[1,5-a]pyrimidin-2-yl)-methanone;
Azepan-1-yl-[5-(4-bromo-phenyl)-3-chloro-7-trifluoromethyl-pyrazolo[1,5-a]pyrimidin-2-yl]-methanone;
(3-Chloro-5-thiophen-2-yl-7-trifluoromethyl-pyrazolo[1,5-a]pyrimidin-2-yl)-(3,4-dihydro-1*H-*isoquinolin-2-yl)-methanone;
(5-Benzo[1,3]dioxol-5-yl-7-trifluoromethyl-pyrazolo[1,5-a]pyrimidin-2-yl)-piperidin-1-yl-methanone;
Azepan-1-yl-(5,7-diphenyl-pyrazolo[1,5-a]pyrimidin-2-yl)-methanone;
Azepan-1-yl-(5-benzo[1,3]dioxol-5-yl-7-trifluoromethyl-pyrazolo[1,5-a]pyrimidin-2-yl)-methanone;
[5-(4-Bromo-phenyl)-7-trifluoromethyl-pyrazolo[1,5-a]pyrimidin-2-yl]-morpholin-4-yl-methanone;
[3-Bromo-5-(4-bromo-phenyl)-7-trifluoromethyl-pyrazolo[1,5-a]pyrimidin-2-yl]-morpholin-4-yl-methanone;
[5-(4-Methoxy-phenyl)-7-trifluoromethyl-pyrazolo[1,5-a]pyrimidin-2-yl]-thiomorpholin-4-yl-methanone;
[5-(4-Bromo-phenyl)-3-chloro-7-trifluoromethyl-pyrazolo[1,5-a]pyrimidin-2-yl]-piperidin-1-yl-methanone;
(5-Benzo[1,3]dioxol-5-yl-7-trifluoromethyl-pyrazolo[1,5-a]pyrimidin-2-yl)-(3,4-dihydro-2H-quinolin-1-yl)-methanone;
(5-Benzo[1,3]dioxol-5-yl-7-trifluoromethyl-pyrazolo[1,5-a]pyrimidin-2-yl)-(4-benzyl-piperazin-1-yl)-methanone;
(5-Benzo[1,3]dioxol-5-yl-7-trifluoromethyl-pyrazolo[1,5-a]pyrimidin-2-yl)-morpholin-4-yl-methanone;
[5-(4-Bromo-phenyl)-7-trifluoromethyl-pyrazolo[1,5-a]pyrimidin-2-yl]-[4-(2-methoxy-phenyl)-piperazin-1-yl]-methanone;
[3-Bromo-5-(4-bromo-phenyl)-7-trifluoromethyl-pyrazolo[1,5-a]pyrimidin-2-yl]-(4-methylpiperazin-1-yl)-methanone;
Azepan-1-yl-[5-(4-methoxy-phenyl)-7-trifluoromethyl-pyrazolo[1,5-a]pyrimidin-2-yl]-methanone;
(2,6-Dimethyl-piperidin-1-yl)-[7-(4-ethoxy-phenyl)-5-methyl-pyrazolo[1,5-a]pyrimidin-2-yl]-methanone;
(3,4-Dihydro-2*H*-quinolin-1-yl)-(5,7-diphenyl-pyrazolo[1,5-a]pyrimidin-2-yl)-methanone;
(3,6-Dibromo-pyrazolo[1,5-a]pyrimidin-2-yl)-piperidin-1-yl-methanone;
(3-Bromo-5-furan-2-yl-7-trifluoromethyl-pyrazolo[1,5-a]pyrimidin-2-yl)-piperidin-1-yl-methanone;
[5-(4-Methoxy-phenyl)-7-trifluoromethyl-pyrazolo[1,5-a]pyrimidin-2-yl]-thiomorpholin-4-yl-methanone;
[5-(3,4-Dichloro-phenyl)-7-trifluoromethyl-pyrazolo[1,5-a]pyrimidin-2-yl]-thiomorpholin-4-yl-methanone;
(3,4-Dihydro-1 H-isoquinolin-2-yl)-[5-(4-nitro-phenyl)-7-trifluoromethyl-pyrazolo[1,5-a]pyrimidin-2-yl]-methanone;
Azepan-1-yl-[5-(4-methoxy-phenyl)-7-trifluoromethyl-pyrazolo[1,5-a]pyrimidin-2-yl]-methanone;
Azepan-1-yl-[5-(4-nitro-phenyl)-7-trifluoromethyl-pyrazolo[1,5-a]pyrimidin-2-yl]-methanone;
[5-(3,4-Dichloro-phenyl)-7-trifluoromethyl-pyrazolo[1,5-a]pyrimidin-2-yl]-morpholin-4-yl-methanone;
Azepan-1-yl-[5-(3,4-dichloro-phenyl)-7-trifluoromethyl-pyrazolo[1,5-a]pyrimidin-2-yl]-methanone;
(3,4-Dihydro-1 H-isoquinolin-2-yl)-(5-phenyl-7-trifluoromethyl-pyrazolo[1,5-a]pyrimidin-2-yl)-methanone;
[5-(4-Bromo-phenyl)-3-chloro-7-trifluoromethyl-pyrazolo[1,5-a]pyrimidin-2-yl]-pyrrolidin-1-yl-methanone;
5,7-Diphenyl-pyrazolo[1,5-a]pyrimidine-2-carboxylic acid cyclohexyl-methyl-amide;
(3,4-Dihydro-1 *H*-isoquinolin-2-yl)-(5-furan-2-yl-7-trifluoromethyl-pyrazolo[1,5-a]pyrimidin-2-yl)-methanone;
(4-Benzyl-piperazin-1-yl)-(3-chloro-5-furan-2-yl-7-trifluoromethyl-pyrazolo[1,5-a]pyrimidin-2-yl)-methanone;
[3-Chloro-5-(4-methoxy-phenyl)-7-trifluoromethyl-pyrazolo[1,5-a]pyrimidin-2-yl]-piperidin-1-yl-methanone;
(3-Chloro-5-*p*-tolyl-7-trifluoromethyl-pyrazolo[1,5-a]pyrimidin-2-yl)-(4-methyl-piperazin-1-yl)-methanone;
(3-Chloro-5-*p*-tolyl-7-trifluoromethyl-pyrazolo[1,5-a]pyrimidin-2-yl)-(4-phenyl-piperazin-1-yl)-methanone;
[3-Chloro-5-(3,4-dimethoxy-phenyl)-7-trifluoromethyl-pyrazolo[1,5-a]pyrimidin-2-yl]-piperidin-1-yl-methanone;
[4-(4-Fluoro-phenyl)-piperazin-1-yl]-(5-p-tolyl-7-trifluoromethyl-pyrazolo[1,5-a]pyrimidin-2-yl)-methanone;
(4-Phenyl-piperazin-1-yl)-(5-p-tolyl-7-trifluoromethyl-pyrazolo[1,5-a]pyrimidin-2-yl)-methanone;
Morpholin-4-yl-(5-*p*-tolyl-7-trifluoromethyl-pyrazolo[1,5-a]pyrimidin-2-yl)-methanone;
(4-Benzyl-piperazin-1-yl)-(5-*p*-tolyl-7-trifluoromethyl-pyrazolo[1,5-a]pyrimidin-2-yl)-methanone;
[5-(4-Chloro-phenyl)-7-trifluoromethyl-pyrazolo[1,5-a]pyrimidin-2-yl]-(4-methyl-piperazin-1-yl)-methanone;
[5-(4-Chloro-phenyl)-7-trifluoromethyl-pyrazolo[1,5-a]pyrimidin-2-yl]-morpholin-4-yl-methanone;
(4-Benzyl-piperazin-1-yl)-[5-(4-chloro-phenyl)-7-trifluoromethyl-pyrazolo[1,5-a]pyrimidin-2-yl]-methanone;
[5-(4-Fluoro-phenyl)-7-trifluoromethyl-pyrazolo[1,5-a]pyrimidin-2-yl]-morpholin-4-yl-methanone;
[5-(4-Fluoro-phenyl)-7-trifluoromethyl-pyrazolo[1,5-a]pyrimidin-2-yl]-piperidin-1-yl-methanone; (3,4-Dihydro-2H-quinolin-1-yl)-[5-(4-fluoro-phenyl)-7-trifluoromethyl-pyrazolo[1,5-a]pyrimidin-2-yl]-methanone;
Azepan-1-yl-[5-(3,4-dimethoxy-phenyl)-7-trifluoromethyl-pyrazolo[1,5-a]pyrimidin-2-yl]-methanone;
(4-Benzyl-piperazin-1-yl)-[5-(4-methoxy-phenyl)-7-trifluoromethyl-pyrazolo[1,5-a]pyrimidin-2-yl]-methanone;
[5-(4-Methoxy-phenyl)-7-trifluoromethyl-pyrazolo[1,5-a]pyrimidin-2-yl]-pyrrolidin-1-yl-methanone;
(5-Benzo[1,3]dioxol-5-yl-7-trifluoromethyl-pyrazolo[1,5-a]pyrimidin-2-yl)-pyrrolidin-1 -yl-methanone;
(3,4-Dihydro-2H-quinolin-1-yl)-[5-(4-nitro-phenyl)-7-trifluoromethyl-pyrazolo[1,5-a]pyrimidin-2-yl]-methanone;
[3-Chloro-5-(4-methoxy-phenyl)-7-trifluoromethyl-pyrazolo[1,5-a]pyrimidin-2-yl]-(4-methylpiperidin-1-yl)-methanone;
[3-Chloro-5-(4-methoxy-phenyl)-7-trifluoromethyl-pyrazolo[1,5-a]pyrimidin-2-yl]-(3,4-dihydro-2H-quinolin-1-yl)-methanone;
3-Chloro-5-(4-methoxy-phenyl)-7-trifluoromethyl-pyrazolo[1,5-a]pyrimidine-2-carboxylic acid cyclohexyl-methyl-amide;
[3-Chloro-5-(4-fluoro-phenyl)-7-trifluoromethyl-pyrazolo[1,5-a]pyrimidin-2-yl]-(4-methylpiperazin-1-yl)-methanone;
(3-Chloro-5-p-tolyl-7-trifluoromethyl-pyrazolo[1,5-a]pyrimidin-2-yl)-morpholin-4-yl-methanone;
Azepan-1-yl-(3-chloro-5-p-tolyl-7-trifluoromethyl-pyrazolo[1,5-a]pyrimidin-2-yl)-methanone;
Azepan-1-yl-[3-chloro-5-(3,4-dimethoxy-phenyl)-7-trifluoromethyl-pyrazolo[1,5-a]pyrimidin-2-yl]-methanone;
[5-(3,4-Dichloro-phenyl)-7-trifluoromethyl-pyrazolo[1,5-a]pyrimidin-2-yl]-(4-methyl-piperazin-1-yl)-methanone;
5,7-Diphenyl-pyrazolo[1,5-a]pyrimidine-2-carboxylic acid benzyl-methyl-amide;
(5,7-Diphenyl-pyrazolo[1,5-a]pyrimidin-2-yl)-(4-phenyl-piperazin-1-yl)-methanone;
Morpholin-4-yl-(5-phenyl-7-trifluoromethyl-pyrazolo[1,5-a]pyrimidin-2-yl)-methanone;
Morpholin-4-yl-(5-thiophen-2-yl-7-trifluoromethyl-pyrazolo[1,5-a]pyrimidin-2-yl)-methanone;
Piperidin-1-yl-(5-thiophen-2-yl-7-trifluoromethyl-pyrazolo[1,5-a]pyrimidin-2-yl)-methanone;
[3-Bromo-5-(4-bromo-thiophen-2-yl)-7-trifluoromethyl-pyrazolo[1,5-a]pyrimidin-2-yl]-piperidin-1-yl-methanone;
(3-Bromo-5-thiophen-2-yl-7-trifluoromethyl-pyrazolo[1,5-a]pyrimidin-2-yl)-(3,4-dihydro-2*H-*quinolin-1-yl)-methanone;
(3-Chloro-5-thiophen-2-yl-7-trifluoromethyl-pyrazolo[1,5-a]pyrimidin-2-yl)-piperidin-1-yl-methanone;
(7-Methyl-5-phenyl-pyrazolo[1,5-a]pyrimidin-2-yl)-morpholin-4-yl-methanone;
(7-Methyl-5-phenyl-pyrazolo[1,5-a]pyrimidin-2-yl)-pyrrolidin-1-yl-methanone;
7-Methyl-5-phenyl-pyrazolo[1,5-a]pyrimidine-2-carboxylic acid benzyl-methyl-amide;
(2,6-Dimethyl-piperidin-1-yl)-[5-(4-fluoro-phenyl)-7-trifluoromethyl-pyrazolo[1,5-a]pyrimidin-2-yl]-methanone;
Azepan-1-yl-[5-(4-fluoro-phenyl)-7-trifluoromethyl-pyrazolo[1,5-a]pyrimidin-2-yl]-methanone;
[5-(4-Fluoro-phenyl)-7-trifluoromethyl-pyrazolo[1,5-a]pyrimidin-2-yl]-(4-methyl-piperazin-1-yl)-methanone;
[5-(4-Fluoro-phenyl)-7-trifluoromethyl-pyrazolo[1,5-a]pyrimidin-2-yl]-(4-phenyl-piperazin-1-yl)-methanone;
(2-Ethyl-piperidin-1-yl)-[5-(4-fluoro-phenyl)-7-trifluoromethyl-pyrazolo[1,5-a]pyrimidin-2-yl]-methanone;
(5-Phenyl-7-trifluoromethyl-pyrazolo[1,5-a]pyrimidin-3-yl)-piperidin-1-yl-methanone;
(5-Methyl-7-phenyl-pyrazolo[1,5-a]pyrimidin-3-yl)-(1,3,3-trimethyl-6-aza-bicyclo[3.2.1]oct-6-yl)-methanone;
(5-Thiophen-2-yl-7-trifluoromethyl-pyrazolo[1,5-a]pyrimidin-3-yl)-(1,3,3-trimethyl-6-azabicyclo[3.2.1]oct-6-yl)-methanone;
(4-Phenyl-piperazin-1-yl)-pyrazolo[1,5-a]pyrimidin-3-yl-methanone;
Pyrazolo[1,5-a]pyrimidine-3-carboxylic acid benzyl-(2-hydroxy-ethyl)-amide;
[5-(4-Bromo-phenyl)-7-trifluoromethyl-pyrazolo[1,5-a]pyrimidin-3-yl]-(3,4-dihydro-1H-isoquinolin-2-yl)-methanone;
(5,7-Dimethyl-pyrazolo[1,5-a]pyrimidin-3-yl)-[4-(4-methoxy-phenyl)-piperazin-1-yl]-methanone;
(3,4-Dihydro-2H-quinolin-1-yl)-[5-(4-fluoro-phenyl)-7-trifluoromethyl-pyrazolo[1,5-a]pyrimidin-3-yl]-methanone;
(5-Furan-2-yl-7-trifluoromethyl-pyrazolo[1,5-a]pyrimidin-3-yl)-piperidin-1-yl-methanone;
Azepan-1-yl-[7-trifluoromethyl-5-(3,4,5-trimethoxy-phenyl)-pyrazolo[1,5-a]pyrimidin-3-yl]-methanone;
(5-Benzo[1,3]dioxol-5-yl-7-trifluoromethyl-pyrazolo[1,5-a]pyrimidin-3-yl)-pyrrolidin-1-yl-methanone;
Azepan-1-yl-(5,7-diphenyl-pyrazolo[1,5-a]pyrimidin-3-yl)-methanone;
4-(5,7-Dimethyl-pyrazolo[1,5-a]pyrimidine-3-carbonyl)-piperazine-1-carboxylic acid ethyl ester;
(3,4-Dihydro-1 H-isoquinolin-2-yl)-(5,7-dimethyl-pyrazolo[1,5-a]pyrimidin-3-yl)-methanone;
[4-(3-Chloro-phenyl)-piperazin-1-yl]-(5-p-tolyl-7-trifluoromethyl-pyrazolo[1,5-a]pyrimidin-3-yl)-methanone;
(4-Methyl-piperidin-1-yl)-pyrazolo[1,5-a]pyrimidin-3-yl-methanone;
[4-(2-Methoxy-phenyl)-piperazin-1-yl]-pyrazolo[1,5-a]pyrimidin-3-yl-methanone;
(3,4-Dihydro-2H-quinolin-1-yl)-[5-(4-methoxy-phenyl)-7-trifluoromethyl-pyrazolo[1,5-a]pyrimidin-3-yl]-methanone;
[5-(4-Methoxy-phenyl)-7-trifluoromethyl-pyrazolo[1,5-a]pyrimidin-3-yl]-thiomorpholin-4-yl-methanone;
(5-Furan-2-yl-7-trifluoromethyl-pyrazolo[1,5-a]pyrimidin-3-yl)-(2-methyl-piperidin-1-yl)-methanone;
(4-Benzyl-piperazin-1-yl)-(5,7-dimethyl-pyrazolo[1,5-a]pyrimidin-3-yl)-methanone;
[5-(4-Bromo-phenyl)-7-trifluoromethyl-pyrazolo[1,5-a]pyrimidin-3-yl]-morpholin-4-yl-methanone;
(5,7-Dimethyl-pyrazolo[1,5-a]pyrimidin-3-yl)-piperidin-1-yl-methanone;
(5-Furan-2-yl-7-trifluoromethyl-pyrazolo[1,5-a]pyrimidin-3-yl)-[4-(2-methoxy-phenyl)-piperazin-1-yl]-methanone;
(5-Phenyl-7-trifluoromethyl-pyrazolo[1,5-a]pyrimidin-3-yl)-piperidin-1-yl-methanone;
Azepan-1-yl-(5-thiophen-2-yl-7-trifluoromethyl-pyrazolo[1,5-a]pyrimidin-3-yl)-methanone;
(2,6-Dimethyl-piperidin-1-yl)-(5-thiophen-2-yl-7-trifluoromethyl-pyrazolo[1,5-a]pyrimidin-3-yl)-methanone;
[5-(4-Methoxy-phenyl)-7-trifluoromethyl-pyrazolo[1,5-a]pyrimidin-3-yl]-(4-methyl-piperazin-1-yl)-methanone;
(4-Methyl-piperazin-1-yl)-(5-thiophen-2-yl-7-trifluoromethyl-pyrazolo[1,5-a]pyrimidin-3-yl)-methanone;
[5-(4-Bromo-phenyl)-7-trifluoromethyl-pyrazolo[1,5-a]pyrimidin-3-yl]-(4-methyl-piperazin-1-yl)-methanone;
(Hexahydro-pyrrolo[1,2-a]pyrazin-2-yl)-(5-p-tolyl-7-trifluoromethyl-pyrazolo[1,5-a]pyrimidin-3-yl)-methanone;
[5-(4-Bromo-phenyl)-7-trifluoromethyl-pyrazolo[1,5-a]pyrimidin-3-yl]-(hexahydro-pyrrolo[1,2-a]pyrazin-2-yl)-methanone;
[5-(3,4-Dimethoxy-phenyl)-7-trifluoromethyl-pyrazolo[1,5-a]pyrimidin-3-yl]-(hexahydropyrrolo[1,2-a]pyrazin-2-yl)-methanone;
1-[5-(3-Methoxy-phenyl)-7-trifluoromethyl-pyrazolo[1,5-a]pyrimidine-3-carbonyl]-piperidine-4-carboxylic acid ethyl ester;
4-[5-(3-Methoxy-phenyl)-7-trifluoromethyl-pyrazolo[1,5-a]pyrimidine-3-carbonyl]-piperazine-1-carboxylic acid ethyl ester;
(3,4-Dihydro-2H-quinolin-1-yl)-[5-(3-methoxy-phenyl)-7-trifluoromethyl-pyrazolo[1,5-a]pyrimidin-3-yl]-methanone;
[4-(4-Fluoro-phenyl)-piperazin-1-yl]-[5-(3-methoxy-phenyl)-7-trifluoromethyl-pyrazolo[1,5-a]pyrimidin-3-yl]-methanone;
5-p-Tolyl-7-trifluoromethyl-pyrazolo[1,5-a]pyrimidine-3-carboxylic acid (1,3-dimethyl-1H-pyrazol-4-ylmethyl)-methyl-amide;
5-p-Tolyl-7-trifluoromethyl-pyrazolo[1,5-a]pyrimidine-3-carboxylic acid methyl-(1 ,3,5-trimethyl-1 H-pyrazol-4-ylmethyl)-amide;
[7-Difluoromethyl-5-(4-methoxy-phenyl)-pyrazolo[1,5-a]pyrimidin-3-yl]-(2-ethyl-imidazol-1-yl)-methanone;
[7-Difluoromethyl-5-(4-methoxy-phenyl)-pyrazolo[1,5-a]pyrimidin-3-yl]-morpholin-4-yl-methanone;
1-[7-Difluoromethyl-5-(4-methoxy-phenyl)-pyrazolo[1,5-a]pyrimidine-3-carbonyl]-1 H-indole-3-carboxylic acid methyl ester;
[4-(4-Chloro-phenyl)-piperazin-1-yl]-[7-difluoromethyl-5-(4-methoxy-phenyl)-pyrazolo[1,5-a]pyrimidin-3-yl]-methanone;
[7-Difluoromethyl-5-(4-methoxy-phenyl)-pyrazolo[1,5-a]pyrimidin-3-yl]-(2-ethyl-piperidin-1-yl)-methanone;
7-Difluoromethyl-5-p-tolyl-pyrazolo[1,5-a]pyrimidine-3-carboxylic acid (1-ethyl-5-methyl-1*H-*pyrazol-4-ylmethyl)-methyl-amide;
1-(7-Difluoromethyl-5-methyl-pyrazolo[1,5-a]pyrimidine-3-carbonyl)-piperidine-4-carboxylic acid amide;
(7-Difluoromethyl-5-phenyl-pyrazolo[1,5-a]pyrimidin-3-yl)-(2-ethyl-imidazol-1-yl)-methanone;
(7-Difluoromethyl-5-phenyl-pyrazolo[1,5-a]pyrimidin-3-yl)-thiomorpholin-4-yl-methanone;
(7-Difluoromethyl-5-*p*-tolyl-pyrazolo[1,5-a]pyrimidin-3-yl)-(4-pyridin-2-yl-piperazin-1-yl)-methanone;
[7-Difluoromethyl-5-(4-methoxy-phenyl)-pyrazolo[1,5-a]pyrimidin-3-yl]-[4-(furan-2-carbonyl)-piperazin-1-yl]-methanone;
7-Difluoromethyl-5-phenyl-pyrazolo[1,5-a]pyrimidine-3-carboxylic acid (1-ethyl-3-methyl-1*H-*pyrazol-4-ylmethyl)-methyl-amide;
[7-Difluoromethyl-5-(4-methoxy-phenyl)-pyrazolo[1,5-a]pyrimidin-3-yl]-thiomorpholin-4-yl-methanone;
(7-Difluoromethyl-5-phenyl-pyrazolo[1,5-a]pyrimidin-3-yl)-(3,4-dihydro-1H-isoquinolin-2-yl)-methanone;
(7-Difluoromethyl-5-methyl-pyrazolo[1,5-a]pyrimidin-3-yl)-piperidin-1-yl-methanone;
Azepan-1-yl-(7-difluoromethyl-5-phenyl-pyrazolo[1,5-a]pyrimidin-3-yl)-methanone;
7-Difluoromethyl-5-p-tolyl-pyrazolo[1,5-a]pyrimidine-3-carboxylic acid methyl-(1-methyl-1H-pyrazol-4-ylmethyl)-amide;
(7-Difluoromethyl-5-*p*-tolyl-pyrazolo[1,5-a]pyrimidin-3-yl)-(3-methyl-piperidin-1-yl)-methanone;
(7-Difluoromethyl-5-*p*-tolyl-pyrazolo[1,5-a]pyrimidin-3-yl)-(4-ethyl-piperazin-1-yl)-methanone;
(7-Difluoromethyl-5-*p*-tolyl-pyrazolo[1,5-a]pyrimidin-3-yl)-morpholin-4-yl-methanone;
7-Difluoromethyl-5-phenyl-pyrazolo[1,5-a]pyrimidine-3-carboxylic acid methyl-(1,3,5-trimethyl-1 H-pyrazol-4-ylmethyl)-amide;
(7-Difluoromethyl-5-phenyl-pyrazolo[1,5-a]pyrimidin-3-yl)-[4-(2-methyl-2H-pyrazole-3-carbonyl)-piperazin-1-yl]-methanone;
7-Difluoromethyl-5-p-tolyl-pyrazolo[1,5-a]pyrimidine-3-carboxylic acid benzyl-methyl-amide;
7-Difluoromethyl-5-(4-methoxy-phenyl)-pyrazolo[1,5-a]pyrimidine-3-carboxylic acid (1-ethyl-3-methyl-1 H-pyrazol-4-ylmethyl)-methyl-amide;
7-Difluoromethyl-5-phenyl-pyrazolo[1,5-a]pyrimidine-3-carboxylic acid methyl-(4-pyrazol-1-yl-benzyl)-amide;
Azepan-1-yl-(5-cyclopropyl-7-difluoromethyl-pyrazolo[1,5-a]pyrimidin-3-yl)-methanone;
(4-Benzyl-piperazin-1-yl)-[5-(4-methoxy-phenyl)-7-trifluoromethyl-pyrazolo[1,5-a]pyrimidin-3-yl]-methanone;
(3,4-Dihydro-2H-quinolin-1-yl)-[5-(4-methoxy-phenyl)-7-trifluoromethyl-pyrazolo[1,5-a]pyrimidin-3-yl]-methanone;
5-(4-Methoxy-phenyl)-7-trifluoromethyl-pyrazolo[1,5-a]pyrimidine-3-carboxylic acid benzyl-methyl-amide;
(5-Furan-2-yl-7-trifluoromethyl-pyrazolo[1,5-a]pyrimidin-3-yl)-(4-methyl-piperazin-1-yl)-methanone;
(5-Furan-2-yl-7-trifluoromethyl-pyrazolo[1,5-a]pyrimidin-3-yl)-thiomorpholin-4-yl-methanone;
[5-(4-Methoxy-phenyl)-7-trifluoromethyl-pyrazolo[1,5-a]pyrimidin-3-yl]-morpholin-4-yl-methanone;
(4-Benzyl-piperazin-1-yl)-(5-thiophen-2-yl-7-trifluoromethyl-pyrazolo[1,5-a]pyrimidin-3-yl)-methanone;
[5-(4-Bromo-phenyl)-7-trifluoromethyl-pyrazolo[1,5-a]pyrimidin-3-yl]-morpholin-4-yl-methanone;
Azepan-1-yl-(5-benzo[1,3]dioxol-5-yl-7-trifluoromethyl-pyrazolo[1,5-a]pyrimidin-3-yl)-methanone;
(5-Benzo[1,3]dioxol-5-yl-7-trifluoromethyl-pyrazolo[1,5-a]pyrimidin-3-yl)-(3,4-dihydro-2H-quinolin-1-yl)-methanone;
5-Benzo[1,3]dioxol-5-yl-7-trifluoromethyl-pyrazolo[1,5-a]pyrimidine-3-carboxylic acid cyclohexyl-methyl-amide;
[5-(4-Bromo-phenyl)-7-trifluoromethyl-pyrazolo[1,5-a]pyrimidin-3-yl]-(3,4-dihydro-2*H*-quinolin-1-yl)-methanone;
[5-(4-Bromo-phenyl)-7-trifluoromethyl-pyrazolo[1,5-a]pyrimidin-3-yl]-piperidin-1-yl-methanone;
Azepan-1-yl-(5-furan-2-yl-7-trifluoromethyl-pyrazolo[1,5-a]pyrimidin-3-yl)-methanone;
(5-Benzo[1,3]dioxol-5-yl-7-trifluoromethyl-pyrazolo[1,5-a]pyrimidin-3-yl)-pyrrolidin-1-yl-methanone;
(5-Benzo[1,3]dioxol-5-yl-7-trifluoromethyl-pyrazolo[1,5-a]pyrimidin-3-yl)-(4-methyl-piperazin-1-yl)-methanone;
(3,4-Dihydro-1H-isoquinolin-2-yl)-[5-(4-methoxy-phenyl)-7-trifluoromethyl-pyrazolo[1,5-a]pyrimidin-3-yl]-methanone;
Azepan-1-yl-[5-(4-bromo-phenyl)-7-trifluoromethyl-pyrazolo[1,5-a]pyrimidin-3-yl]-methanone;
(3,4-Dihydro-2*H*-quinolin-1-yl)-(5,7-diphenyl-pyrazolo[1,5-a]pyrimidin-3-yl)-methanone;
Azepan-1-yl-(5,7-diphenyl-pyrazolo[1,5-a]pyrimidin-3-yl)-methanone;
(5-Benzo[1,3]dioxol-5-yl-7-trifluoromethyl-pyrazolo[1,5-a]pyrimidin-3-yl)-(4-benzyl-piperazin-1-yl)-methanone;
(4-Benzyl-piperazin-1-yl)-(5,7-diphenyl-pyrazolo[1,5-a]pyrimidin-3-yl)-methanone;
(5,7-Diphenyl-pyrazolo[1,5-a]pyrimidin-3-yl)-(4-methyl-piperazin-1-yl)-methanone;
[5-(4-Bromo-phenyl)-7-trifluoromethyl-pyrazolo[1,5-a]pyrimidin-3-yl]-(2-methyl-piperidin-1-yl)-methanone;
(3,4-Dihydro-2*H*-quinolin-1-yl)-(5-furan-2-yl-7-trifluoromethyl-pyrazolo[1,5-a]pyrimidin-3-yl)-methanone;
(5-Furan-2-yl-7-trifluoromethyl-pyrazolo[1,5-a]pyrimidin-3-yl)-(2-methyl-piperidin-1-yl)-methanone;
[5-(4-Methoxy-phenyl)-7-trifluoromethyl-pyrazolo[1,5-a]pyrimidin-3-yl]-piperidin-1-yl-methanone;
(5-Benzo[1,3]dioxol-5-yl-7-trifluoromethyl-pyrazolo[1,5-a]pyrimidin-3-yl)-morpholin-4-yl-methanone;
(5-Benzo[1,3]dioxol-5-yl-7-trifluoromethyl-pyrazolo[1,5-a]pyrimidin-3-yl)-piperidin-1-yl-methanone;
Morpholin-4-yl-[7-trifluoromethyl-5-(3,4,5-trimethoxy-phenyl)-pyrazolo[1,5-a]pyrimidin-3-yl]-methanone;
(4-Benzyl-piperazin-1-yl)-[7-trifluoromethyl-5-(3,4,5-trimethoxy-phenyl)-pyrazolo[1,5-a]pyrimidin-3-yl]-methanone;
(3,4-Dihydro-2*H*-quinolin-1-yl)-[7-trifluoromethyl-5-(3,4,5-trimethoxy-phenyl)-pyrazolo[1,5-a]pyrimidin-3-yl]-methanone;
(4-Methyl-piperazin-1-yl)-[7-trifluoromethyl-5-(3,4,5-trimethoxy-phenyl)-pyrazolo[1,5-a]pyrimidin-3-yl]-methanone;
Azepan-1-yl-[7-trifluoromethyl-5-(3,4,5-trimethoxy-phenyl)-pyrazolo[1,5-a]pyrimidin-3-yl]-methanone;
[5-(4-Methoxy-phenyl)-7-trifluoromethyl-pyrazolo[1,5-a]pyrimidin-3-yl]-piperidin-1-yl-methanone;
[5-(4-Methoxy-phenyl)-7-trifluoromethyl-pyrazolo[1,5-a]pyrimidin-3-yl]-pyrrolidin-1-yl-methanone;
Piperidin-1-yl-(5-thiophen-2-yl-7-trifluoromethyl-pyrazolo[1,5-a]pyrimidin-3-yl)-methanone;
(4-Benzyl-piperazin-1-yl)-[5-(4-methoxy-phenyl)-7-trifluoromethyl-pyrazolo[1,5-a]pyrimidin-3-yl]-methanone;
(3,4-Dihydro-2H-quinolin-1-yl)-[5-(4-methoxy-phenyl)-7-trifluoromethyl-pyrazolo[1,5-a]pyrimidin-3-yl]-methanone;
(5-Furan-2-yl-7-trifluoromethyl-pyrazolo[1,5-a]pyrimidin-3-yl)-(2-methyl-piperidin-1-yl)-methanone;
(5-Furan-2-yl-7-trifluoromethyl-pyrazolo[1,5-a]pyrimidin-3-yl)-morpholin-4-yl-methanone;
(5-Furan-2-yl-7-trifluoromethyl-pyrazolo[1,5-a]pyrimidin-3-yl)-thiomorpholin-4-yl-methanone;
[4-(4-Fluoro-phenyl)-piperazin-1-yl]-(5-thiophen-2-yl-7-trifluoromethyl-pyrazolo[1,5-a]pyrimidin-3-yl)-methanone;
(4-Methyl-piperidin-1-yl)-(5-thiophen-2-yl-7-trifluoromethyl-pyrazolo[1,5-a]pyrimidin-3-yl)-methanone;
(5-Furan-2-yl-7-trifluoromethyl-pyrazolo[1,5-a]pyrimidin-3-yl)-(4-methyl-piperazin-1-yl)-methanone;
(4-Benzyl-piperazin-1-yl)-(5-thiophen-2-yl-7-trifluoromethyl-pyrazolo[1,5-a]pyrimidin-3-yl)-methanone;
[5-(4-Methoxy-phenyl)-7-trifluoromethyl-pyrazolo[1,5-a]pyrimidin-3-yl]-morpholin-4-yl-methanone;
Piperidin-1-yl-[7-trifluoromethyl-5-(3,4,5-trimethoxy-phenyl)-pyrazolo[1,5-a]pyrimidin-3-yl]-methanone;
(2-Methyl-piperidin-1-yl)-(5-thiophen-2-yl-7-trifluoromethyl-pyrazolo[1,5-a]pyrimidin-3-yl)-methanone;
Azepan-1-yl-[5-(4-methoxy-phenyl)-7-trifluoromethyl-pyrazolo[1,5-a]pyrimidin-3-yl]-methanone;
5-Thiophen-2-yl-7-trifluoromethyl-pyrazolo[1,5-a]pyrimidine-3-carboxylic acid benzyl-methyl-amide;
[5-(4-Methoxy-phenyl)-7-trifluoromethyl-pyrazolo[1,5-a]pyrimidin-3-yl]-pyrrolidin-1-yl-methanone;
[5-(4-Chloro-phenyl)-2-methyl-7-trifluoromethyl-pyrazolo[1,5-a]pyrimidin-3-yl]-piperidin-1-yl-methanone;
Azepan-1-yl-[5-(4-chloro-phenyl)-2-methyl-7-trifluoromethyl-pyrazolo[1,5-a]pyrimidin-3-yl]-methanone;
[5-(4-Chloro-phenyl)-2-methyl-7-trifluoromethyl-pyrazolo[1,5-a]pyrimidin-3-yl]-(4-methylpiperazin-1-yl)-methanone;
Morpholin-4-yl-(5-p-tolyl-7-trifluoromethyl-pyrazolo[1,5-a]pyrimidin-3-yl)-methanone;
(4-Benzyl-piperazin-1-yl)-(5-p-tolyl-7-trifluoromethyl-pyrazolo[1,5-a]pyrimidin-3-yl)-methanone;
5-(4-Methoxy-phenyl)-7-trifluoromethyl-pyrazolo[1,5-a]pyrimidine-3-carboxylic acid benzyl-methyl-amide;
(4-Benzyl-piperazin-1-yl)-(5-phenyl-7-trifluoromethyl-pyrazolo[1,5-a]pyrimidin-3-yl)-methanone;
Morpholin-4-yl-(5-phenyl-7-trifluoromethyl-pyrazolo[1,5-a]pyrimidin-3-yl)-methanone; or
a salt thereof with a pharmaceutically acceptable acid or base, or any optical isomer or mixture of optical isomers, including a racemic mixture, or any tautomeric forms.

8. A compound according to claim 6 or 7, which is an agent useful for the treatment, prevention and/or prophylaxis of any conditions, disorders and diseases wherein a modulation or an inhibition of the activity of 11βHSD1 is beneficial.

9. A pharmaceutical composition comprising, as an active ingredient, at least one compound according to claim 6 or 7, together with one ore more pharmaceutically acceptable carriers or excipients.

10. A use of a compound according to claim 6 or 7, for the preparation of a pharmaceutical composition for the treatment, prevention and/or prophylaxis of any conditions, disorders and diseases wherein a modulation or an inhibition of the activity of 11 βHSD1 is beneficial.
